# EUROPÄISCHE PATENTSCHRIFT

(11) **EP 2 579 887 B1**
(45) Veröffentlichungstag und Bekanntmachung des Hinweises auf die Patenterteilung: **12.11.2014**
(21) Anmeldenummer: 11725412.8
(22) Anmeldetag: 06.06.2011
(51) Int. Cl.: A61K 38/08, C07K 7/02

(54) **NEUE AURISTATIN-DERIVATE UND IHRE VERWENDUNG**
NOVEL AURISTATIN DERIVATIVES AND USE THEREOF
NOUVEAUX DÉRIVÉS DE L'AURISTATINE ET LEUR UTILISATION

(30) Priorität: 10.06.2010 EP 10165550; 16.03.2011 EP 11158464
(43) Veröffentlichungstag der Anmeldung: 17.04.2013
(73) Patentinhaber: Seattle Genetics, Inc., Bothell, WA 98021 (US)
(72) Erfinder: LERCHEN, Hans-Georg, 51375 Leverkusen (DE); STELTE-LUDWIG, Beatrix, 42489 Wülfrath (DE); GOLFIER, Sven, 12247 Berlin (DE); SCHUHMACHER, Joachim, 42113 Wuppertal (DE); KRENZ, Ursula, 42799 Leichlingen (DE)
(74) Vertreter: Hoffmann Eitle
(86) Internationale Anmeldenummer: PCT/EP2011/059300
(87) Internationale Veröffentlichungsnummer: WO 2011/154359

(56) Entgegenhaltungen:
- WO-A1-96/18408
- WO-A1-99/35164

## Beschreibung

Die vorliegende Anmeldung betrifft neue Derivate von Monomethylauristatin F, Verfahren zur Herstellung dieser Derivate, die Verwendung dieser Derivate zur Behandlung und/oder Prävention von Krankheiten sowie die Verwendung dieser Derivate zur Herstellung von Arzneimitteln zur Behandlung und/oder Prävention von Krankheiten, insbesondere von hyperproliferativen und/oder angiogenen Erkrankungen wie beispielsweise Krebserkrankungen. Solche Behandlungen können als Monotherapie oder auch in Kombination mit anderen Arzneimitteln oder weiteren therapeutischen Maßnahmen erfolgen.

Krebserkrankungen sind die Folge unkontrollierten Zellwachstums verschiedenster Gewebe. In vielen Fällen dringen die neuen Zellen in bestehende Gewebe ein (invasives Wachstum), oder sie metastasieren in entfernte Organe. Krebserkrankungen treten in verschiedensten Organen auf und haben oft gewebespezifische Krankheitsverläufe. Daher beschreibt die Bezeichnung Krebserkrankung als Oberbegriff eine große Gruppe definierter Erkrankungen verschiedener Organe, Gewebe und Zelltypen.

Tumore früher Stadien lassen sich gegebenenfalls durch chirurgische und radiotherapeutische Maßnahmen entfernen. Metastasierte Tumore können im Regelfall durch Chemotherapeutika nur palliativ therapiert werden. Ziel hierbei ist, die optimale Kombination aus einer Verbesserung der Lebensqualität und der Verlängerung der Lebenszeit zu erreichen.

Die meisten der heutzutage parenteral applizierten Chemotherapeutika sind oft nicht zielgerichtet auf das Tumorgewebe oder die Tumorzellen, sondern durch die systemische Gabe unspezifisch im Körper verteilt, d.h. auch an Orten, an denen eine Wirkstoffexposition unerwünscht ist, wie beispielsweise in gesunden Zellen, Geweben und Organen. Dies kann zu unerwünschten Nebenwirkungen bis hin zu gravierenden allgemein-toxischen Effekten führen, die dann den therapeutisch nutzbaren Dosisbereich des Wirkstoffs oftmals stark limitieren oder ein völliges Absetzen der Medikation erfordern.

Die verbesserte und selektive Verfügbarkeit dieser Chemotherapeutika in der Tumorzelle oder dem direkt umgebenden Gewebe und die damit verbundene Wirksteigerung einerseits und Minimierung toxischer Nebeneffekte andererseits steht daher seit mehreren Jahren im Fokus bei der Entwicklung neuer Chemotherapeutika. Viele Versuche wurden bislang unternommen, effiziente Methoden für die Wirkstoffeinbringung in die Zielzelle zu entwickeln. Die Optimierung der Assoziation zwischen Wirkstoff und intrazellulärem Ziel und die Minimierung der interzellulären Wirkstoffverteilung, beispielsweise zu Nachbarzellen, stellen jedoch nach wie vor eine schwierige Aufgabe dar.

Für die zielgerichtete Adressierung von Tumorgewebe und Tumorzellen sind beispielsweise monoklonale Antikörper geeignet. Die Bedeutung solcher Antikörper für die klinische Behandlung von Krebserkrankungen hat allgemein in den letzten Jahren erheblich zugenommen, basierend auf der Wirksamkeit solcher Agentien wie Trastuzumab (Herceptin), Rituximab (Rituxan), Cetuximab (Erbitux) und Bevacizumab (Avastin), welche inzwischen für die Therapie einzelner, spezifischer Tumorerkrankungen zugelassen sind [siehe z.B. G. P. Adams und L. M. Weiner, Nat. Biotechnol. 23, 1147-1157 (2005)]. In Folge hiervon ist auch das Interesse an so genannten Immunokonjugaten deutlich gestiegen, in welchen ein internalisierender, gegen ein Tumor-assoziiertes Antigen gerichteter Antikörper auf kovalente Weise über eine Verknüpfungseinheit ("linker") mit einem cytotoxisch wirkenden Agens verbunden ist, das nach Einschleusung und anschließender Spaltung des Konjugats innerhalb der Tumorzelle freigesetzt wird und dort seine Wirkung direkt und selektiv entfalten kann. Auf diesem Wege könnte die Schädigung von normalem Gewebe im Vergleich zu einer konventionellen Chemotherapie der Krebserkrankung in signifikant engeren Grenzen gehalten werden [siehe z.B. J. M. Lambert, Curr. Opin. Pharmacol. 5, 543-549 (2005); A. M. Wu und P. D. Senter, Nat. Biotechnol. 23, 1137-1146 (2005); P. D. Senter, Curr. Opin. Chem. Biol. 13, 235-244 (2009); L. Ducry und B. Stump, Bioconjugate Chem. 21, 5-13 (2010)].

Statt Antikörpern können auch Liganden aus dem Wirkstoffbereich kleiner Moleküle verwendet werden, die selektiv an einen speziellen Zielort ("target"), wie beispielsweise an einen Rezeptor, binden [siehe z.B. E. Ruoslahti et al., Science 279, 377-380 (1998); D. Karkan et al., PLoS ONE 3 (6), e2469 (June 25, 2008)]. Bekannt sind auch Konjugate aus cytotoxischem Wirkstoff und adressierendem Ligand, die zwischen Ligand und Wirkstoff eine definierte Spaltstelle zur Freisetzung des Wirkstoffs aufweisen. Eine solche "Soll-Bruchstelle" kann beispielsweise in einer Peptidkette bestehen, die an einer bestimmten Stelle selektiv durch ein spezielles Enzym am Wirkort gespalten werden kann [siehe z.B. R. A. Firestone und L. A. Telan, US Patent Application US 2002/0147138].

Auristatin E (AE) und Monomethylauristatin E (MMAE) sind synthetische Analoga der Dolastatine, einer speziellen Gruppe von linearen Pseudopeptiden, welche ursprünglich aus marinen Quellen isoliert wurden und die zum Teil sehr potente cytotoxische Aktivität gegenüber Tumorzellen aufweisen [für eine Übersicht siehe z.B. G. R. Pettit, Prog. Chem. Org. Nat. Prod. 70, 1-79 (1997); G. R. Pettit et al., Anti-Cancer Drug Design 10, 529-544 (1995); G. R. Pettit et al., Anti-Cancer Drug Design 13, 243-277 (1998)].

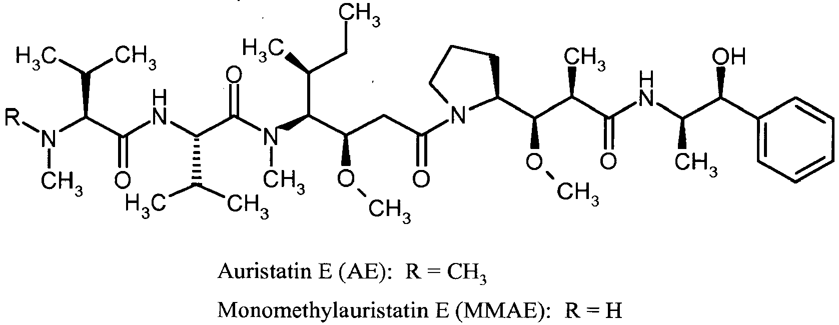

Allerdings besitzt MMAE den Nachteil einer vergleichsweise hohen systemischen Toxizität. Zudem ist diese Verbindung bei einer Anwendung in Form von Antikörper-Wirkstoff-Konjugaten (Immunokonjugaten) nicht kompatibel mit Verknüpfungseinheiten (Linkern) zwischen Antikörper und Wirkstoff, welche keine enzymatisch spaltbare Soll-Bruchstelle aufweisen [S. O. Doronina et al., Bioconjugate Chem. 17, 114-124 (2006)].

Monomethylauristatin F (MMAF) ist ein Auristatin-Derivat mit einer C-terminalen Phenylalanin-Einheit, das nur moderate anti-proliferative Wirkung im Vergleich zu MMAE aufweist. Dies ist sehr wahrscheinlich auf die freie Carboxylgruppe zurückzuführen, die aufgrund ihrer Polarität und Ladung die Zellgängigkeit dieser Verbindung negativ beeinflusst. In diesem Zusammenhang wurde der Methylester von MMAF (MMAF-OMe) als ein neutral geladenes, zellgängiges Prodrug-Derivat beschrieben, das im Vergleich zu MMAF eine um mehrere Größenordnungen erhöhte *in vitro-Cyto-*toxizität gegenüber verschiedenen Karzinoma-Zelllinien zeigt [S. O. Doronina et al., Bioconjugate Chem. 17, 114-124 (2006)]. Es ist anzunehmen, dass diese Wirkung durch MMAF selbst hervorgerufen wird, das nach Aufnahme des Prodrugs in die Zellen schnell durch intrazelluläre Ester-Hydrolyse freigesetzt wird.

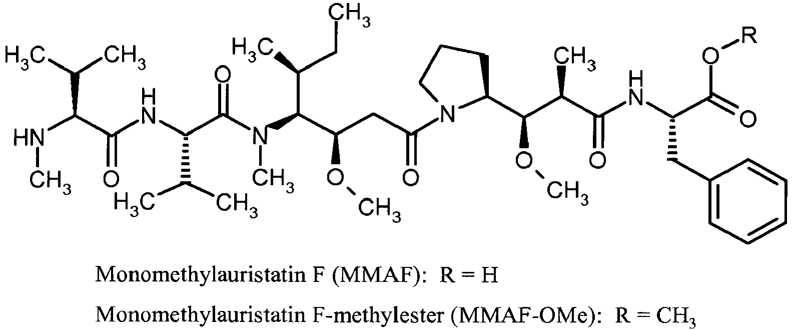

Wirkstoff-Verbindungen auf Basis von einfachen Ester-Derivaten unterliegen allgemein jedoch dem Risiko einer chemischen Instabilität infolge einer unspezifischen, vom vorgesehenen Wirkort unabhängigen Ester-Hydrolyse, beispielsweise durch im Blutplasma vorhandene Esterasen; dies kann die Anwendbarkeit solcher Verbindungen in der Therapie deutlich einschränken.

Aufgabe der vorliegenden Erfindung war es daher, ausgehend vom nur moderat wirksamen Monomethylauristatin F (MMAF) neue Verbindungen zu identifizieren und für die Behandlung insbesondere von Krebserkrankungen bereitzustellen, die einerseits eine deutlich stärkere cytotoxische Aktivität in Ganzzell-Assays aufweisen und andererseits eine erhöhte Plasmastabilität im Vergleich zu einfachen Ester-Derivaten wie MMAF-OMe besitzen. Solche Substanzen könnten sich in besonderem Maße als Toxophore für die Verknüpfung mit Proteinen, wie beispielsweise Antikörpern, oder auch mit niedermolekularen Liganden zu anti-proliferativ wirkenden (Immuno-)Konjugaten eignen.

Monomethylauristatin F (MMAF) sowie verschiedene Ester- und Amid-Derivate hiervon wurden in WO 2005/081711-A2 offenbart. Weitere Auristatin-Analoga mit einer C-terminalen, amidisch substituierten Phenylalanin-Einheit sind in WO 01/18032-A2 beschrieben. In WO 02/088172-A2 und WO 2007/008603-A1 werden MMAF-Analoga beansprucht, welche Seitenketten-Modifikationen des Phenylalanins betreffen, und in WO 2007/008848-A2 solche, in denen die Carboxylgruppe des Phenylalanins modifiziert ist. Über den C-Terminus verknüpfte Auristatin-Konjugate wurden vor kurzem in WO 2009/117531-A1 beschrieben [siehe auch S. O. Doronina et al., Biocon-jugate Chem. 19, 1960-1963 (2008)].

Gegenstand der vorliegenden Erfindung sind nunmehr Verbindungen der allgemeinen Formel (I) 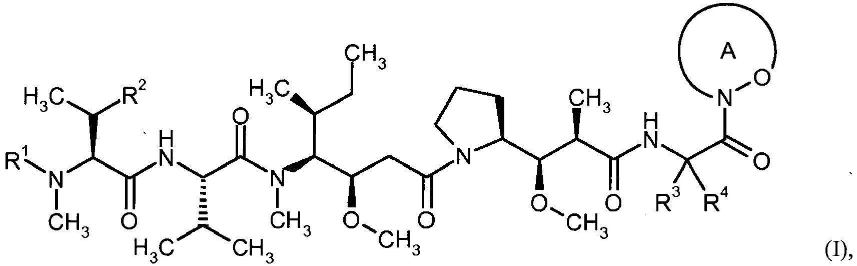 in welcher
- R¹: für Wasserstoff, (C₁-C₆)-Alkyl oder eine Gruppe der Formel Q¹-L¹-* oder Q²-L²-* steht, worin
* die Verknüpfungsstelle mit dem Stickstoffatom kennzeichnet,
Q¹ Hydroxycarbonyl, (C₁-C₄)-Alkoxycarbonyl oder Benzyloxycarbonyl bedeutet,
L¹ geradkettiges (C₁-C₁₂)-Alkandiyl bedeutet, das bis zu vierfach mit Methyl substituiert sein kann und in dem (a) zwei Kohlenstoffatome in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können oder (b) bis zu drei zueinander nicht benachbarte CH₂-Gruppen gegen -O- ausgetauscht sein können,
Q² Hydroxy, Amino oder Mono-(C₁-C₄)-alkylamino bedeutet, und
L² geradkettiges (C₂-C₁₂)-Alkandiyl bedeutet, das bis zu vierfach mit Methyl substituiert sein kann und in dem (a) zwei Kohlenstoffatome in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können oder (*b*) bis zu drei zueinander nicht benachbarte CH₂-Gruppen gegen -O- ausgetauscht sein können,
- R²: für Methyl oder Hydroxy steht,
- R³: für Wasserstoff oder Methyl steht,
- R⁴: für Isopropyl, Isobutyl, sec.-Butyl, *tert.-Butyl,* Phenyl, Benzyl, 4-Hydroxybenzyl, 4-Hydroxy-3-nitrobenzyl, 1-Phenylethyl, Diphenylmethyl, 1*H*-Imidazol-4-ylmethyl oder 1*H*-Indol-3-ylmethyl steht,
oder
- R³ und R⁴: zusammen mit dem Kohlenstoffatom, an das sie beide gebunden sind, eine 2-Phenyl-cyclopropan-1,1-diyl-Gruppe der Formel 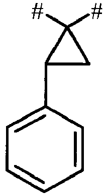 bilden, worin
# die Verknüpfungsstellen mit den übrigen Teilen des Moleküls kennzeichnet,
und
der Ring A mit der darin enthaltenen N-O-Gruppierung für einen mono- oder bicyclischen, gegebenenfalls substituierten Heterocyclus der Formel 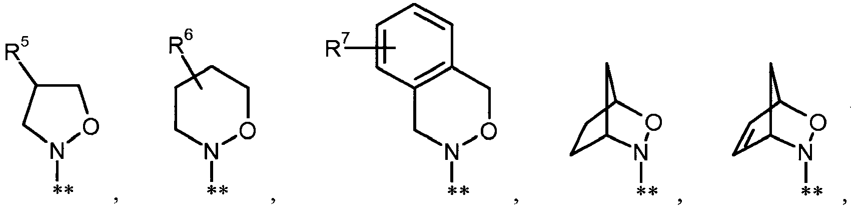 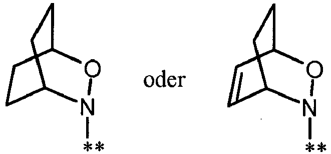 steht, worin
- **: die Verknüpfungsstelle mit der Carbonyl-Gruppe kennzeichnet,
R⁵ und R⁶ jeweils Wasserstoff, Hydroxy, (C₁-C₄)-Alkoxy oder Benzyloxy bedeuten, und
- R⁷: Wasserstoff, Fluor, Chlor, Cyano, Methyl oder Trifluormethyl bedeutet,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomeren und Diastereomeren und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylpiperidin, *N*-Methylmorpholin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-Butyl, Isobutyl, *sec.*-Butyl, *tert.-Butyl, n*-Pentyl, 2-Pentyl, 3-Pentyl, Neopentyl, *n*-Hexyl, 2-Hexyl und 3-Hexyl.
(C₁-C₁₂)-Alkandiyl, (C₁-C₆)-Alkandiyl, (C₁-C₁₂)-Alkandiyl und (C₂-C₆)-Alkandiyl stehen im Rahmen der Erfindung für einen geradkettigen, α,ω-divalenten Alkylrest mit 1 bis 12, 1 bis 6, 2 bis 12 bzw. 2 bis 6 Kohlenstoffatomen. Beispielhaft und vorzugsweise seien genannt: Methylen, Ethan-1,2-diyl (1,2-Ethylen), Propan-1,3-diyl (1,3-Propylen), Butan-1,4-diyl (1,4-Butylen), Pentan-1,5-diyl (1,5-Pentylen), Hexan-1,6-diyl (1,6-Hexylen), Heptan-1,7-diyl (1,7-Hexylen), Octan-1,8-diyl (1,8-Octylen), Nonan-1,9-diyl (1,9-Nonylen), Decan-1,10-diyl (1,10-Decylen), Undecan-1,11-diyl (1,11-Undecylen) und Dodecan-1,12-diyl (1,12-Dodecylen).
(C₃-C₆)-Cycloalkyl steht im Rahmen der Erfindung für eine monocyclische, gesättigte Cycloalkylgruppe mit 3 bis 6 Kohlenstoffatomen. Beispielhaft und vorzugsweise seien genannt: Cyclopropyl, Cyclobutyl, Cyclopentyl und Cyclohexyl.
(C₁-C₄)-Allcoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, *n-*Propoxy, Isopropoxy, *n*-Butoxy, Isobutoxy, *sec*.-Butoxy und *tert*.-Butoxy.
(C₁-C₄)-Alkoxycarbonyl steht im Rahmen der Erfindung für einen geradkettigen oder verzweigten Alkoxyrest mit 1 bis 4 Kohlenstoffatomen, der über eine Carbonyl-Gruppe [-C(=O)-] verknüpft ist. Beispielhaft und vorzugsweise seien genannt: Methoxycarbonyl, Ethoxycarbonyl, *n*-Propoxy-carbonyl, Isopropoxycarbonyl, *n*-Butoxycarbonyl und *tert*.-Butoxycarbonyl.
Mono-(C₁-C₄)-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, *n*-Propylamino, Isopropylamino, *n-*Butylamino und *tert*.-Butylamino.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt. Besonders bevorzugt ist die Substitution mit einem Substituenten.

Bevorzugt im Rahmen der vorliegenden Erfindung sind Verbindungen der Formel (I), in welcher
- R¹: für Wasserstoff, (C₁-C₄)-Alkyl oder eine Gruppe der Formel Q¹-L¹-* oder Q²-L²-* steht, worin
* die Verknüpfungsstelle mit dem Stickstoffatom kennzeichnet,
Q¹ Hydroxycarbonyl oder (C₁-C₄)-Alkoxycarbonyl bedeutet,
L¹ geradkettiges (C₁-C₁₂)-Alkandiyl bedeutet, in dem (*a*) zwei Kohlenstoffatome in 1,3-oder 1,4-Relation zueinander unter Einbezug des einen beziehungsweise der beiden zwischen ihnen liegenden Kohlenstoffatome zu einem Phenyl-Ring verbrückt sein können oder (*b*) bis zu drei zueinander nicht benachbarte CH₂-Gruppen gegen -O-ausgetauscht sein können,
Q² Hydroxy, Amino oder Methylamino bedeutet,
und
L² geradkettiges (C₂-C₁₂)-Alkandiyl bedeutet, das ein- oder zweifach mit Methyl substituiert sein kann und in dem bis zu drei zueinander nicht benachbarte CH₂-Gruppen gegen -O- ausgetauscht sein können,
- R²: für Methyl oder Hydroxy steht,
- R³: für Wasserstoff steht,
- R⁴: für Benzyl, 4-Hydroxybenzyl, 1-Phenylethyl oder 1*H*-Indol-3-ylmethyl steht,
oder
R³ und R⁴ zusammen mit dem Kohlenstoffatom, an das sie beide gebunden sind, eine 2-Phenyl-cyclopropan-1,1-diyl-Gruppe der Formel 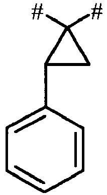 bilden, worin
# die Verknüpfungsstellen mit den übrigen Teilen des Moleküls kennzeichnet,
und
der Ring A mit der darin enthaltenen N-O-Gruppierung für einen mono- oder bicyclischen, gegebenenfalls substituierten Heterocyclus der Formel 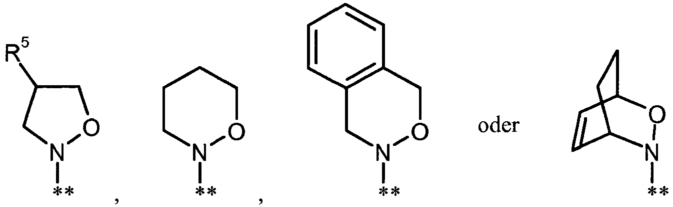 steht, worin
- **: die Verknüpfungsstelle mit der Carbonyl-Gruppe kennzeichnet,
und
- R⁵: Wasserstoff, Hydroxy oder Benzyloxy bedeutet,
sowie ihre Salze, Solvate und Solvate der Salze.

Besonders bevorzugt im Rahmen der vorliegenden Erfindung sind Verbindungen der Formel (I), in welcher
- R¹: für Wasserstoff, Methyl oder eine Gruppe der Formel Q¹-L¹-* oder Q²-L²-* steht, worin
* die Verknüpfungsstelle mit dem Stickstoffatom kennzeichnet,
Q¹ Hydroxycarbonyl, Methoxycarbonyl oder Ethoxycarbonyl bedeutet,
L¹ geradkettiges (C₁-C₆)-Alkandiyl bedeutet, in dem zwei Kohlenstoffatome in 1,4-Relation zueinander unter Einbezug der beiden zwischen ihnen liegenden Kohlenstoffatome zu einem Phenyl-Ring verbrückt sein können,
Q² Hydroxy oder Amino bedeutet,
und
L² geradkettiges (C₂-C₆)-Alkandiyl bedeutet,
- R²: für Methyl steht,
- R³: für Wasserstoff steht,
- R⁴: für Benzyl, 1-Phenylethyl oder 1*H*-Indol-3-ylmethyl steht,
oder
R³ und R⁴ zusammen mit dem Kohlenstoffatom, an das sie beide gebunden sind, eine (1*S*,2*R*)-2-Phenylcyclopropan-1,1-diyl-Gruppe der Formel 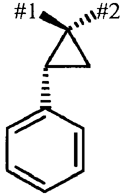 bilden, worin
- #1: die Verknüpfungsstelle mit dem angrenzenden Stickstoffatom
und
- #2: die Verknüpfungsstelle mit der Carbonyl-Gruppe kennzeichnen,
und
der Ring A mit der darin enthaltenen N-O-Gruppierung für einen mono- oder bicyclischen, gegebenenfalls substituierten Heterocyclus der Formel 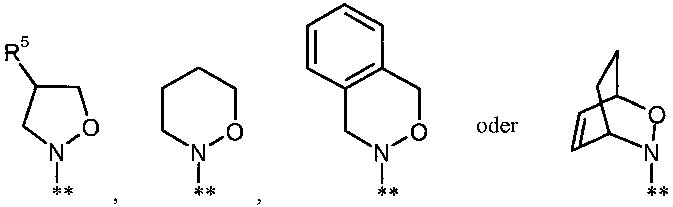 steht, worin
- **: die Verknüpfungsstelle mit der Carbonyl-Gruppe kennzeichnet,
und
- R⁵: Wasserstoff, Hydroxy oder Benzyloxy bedeutet,
sowie ihre Salze, Solvate und Solvate der Salze.

Von besonderer Bedeutung im Rahmen der vorliegenden Erfindung sind Verbindungen der Formel (I-A) 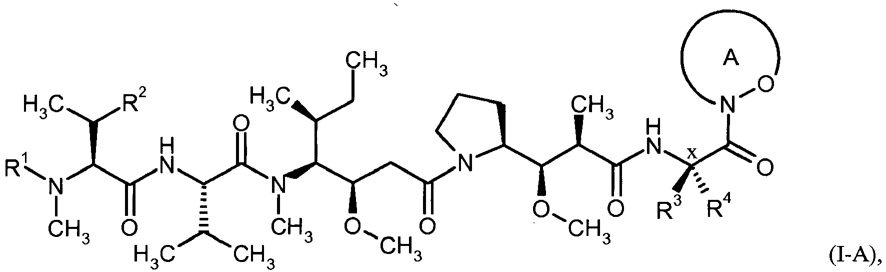 in welcher R¹, R², R³, R⁴ und der Ring A die oben definierten Bedeutungen haben und das die Reste R³ und R⁴ tragende C^{x}-Kohlenstoffatom die abgebildete S-Konfiguration aufweist,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man eine Verbindung der Formel (II) 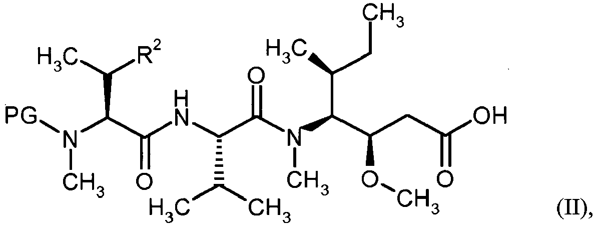 in welcher R² die oben angegebene Bedeutung hat
und
- PG: für eine Amino-Schutzgruppe wie beispielsweise (9*H*-Fluoren-9-ylmethoxy)carbonyl, *tert.-*Butoxycarbonyl oder Benzyloxycarbonyl steht,
in einem inerten Lösungsmittel unter Aktivierung der Carboxyl-Funktion in (II) entweder
[A] zunächst mit der Verbindung der Formel (III) 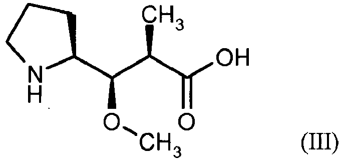 oder einem Salz hiervon zu einer Verbindung der Formel (IV) 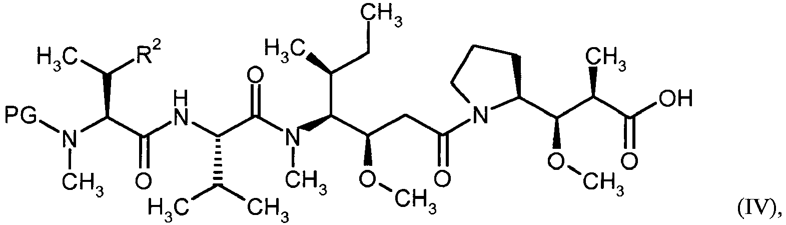 in welcher R² und PG die oben angegebenen Bedeutungen haben,
   kuppelt und diese anschließend in einem inerten Lösungsmittel unter Aktivierung der Carboxyl-Funktion mit einer Verbindung der Formel (V) 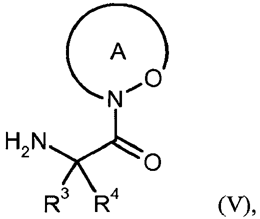 in welcher R³, R⁴ und der Ring A die oben angegebenen Bedeutungen haben,
   oder einem Salz dieser Verbindung zu einer Verbindung der Formel (VI) 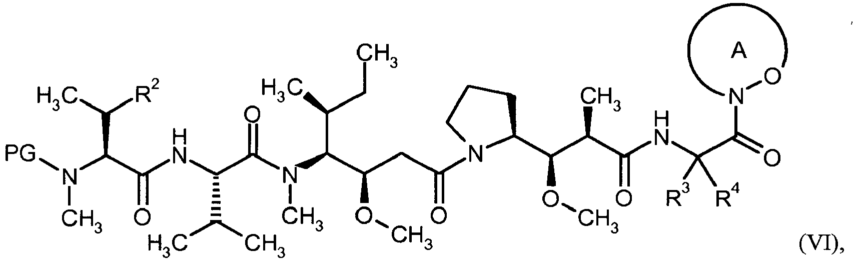 in welcher R², R³, R⁴, der Ring A und PG die oben angegebenen Bedeutungen haben,
   kuppelt
   oder
[B] mit einer Verbindung der Formel (VII) 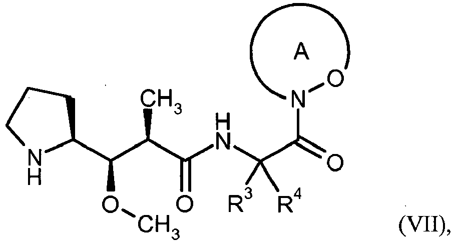 in welcher R³, R⁴ und der Ring A die oben angegebenen Bedeutungen haben,
   oder einem Salz dieser Verbindung gleichfalls zu der Verbindung der Formel (VI) 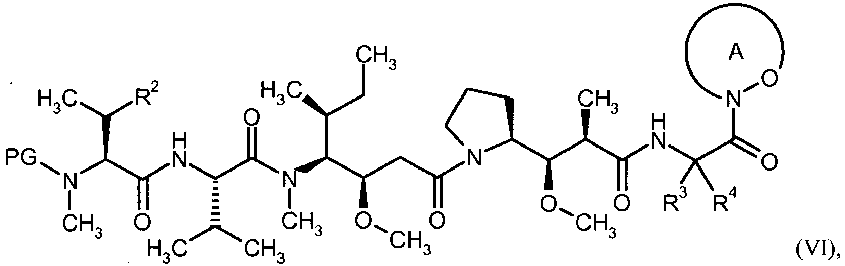 in welcher R², R³, R⁴, der Ring A und PG die oben angegebenen Bedeutungen haben,
   kuppelt
und die jeweils resultierende Verbindung der Formel (VI) dann nach üblichen Methoden der Peptidchemie zu einer erfindungsgemäßen Verbindung der Formel (I-B) 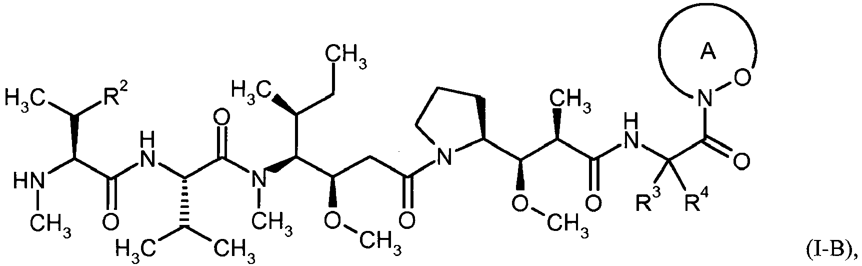 in welcher R², R³, R⁴ und der Ring A die oben angegebenen Bedeutungen haben,
entschützt und diese nachfolgend, falls gewünscht, entweder (i) durch baseninduzierte Alkylierung mit einer Verbindung der Formel (VIII)

R^{1A}-X (VIII),

in welcher
- R^{1A}: die oben angegebene Bedeutung von R¹ hat, jedoch nicht für Wasserstoff steht,
und
- X: für eine Fluchtgruppe wie beispielsweise Chlorid, Bromid, Iodid, Mesylat, Triflat oder Tosylat steht,
in eine erfindungsgemäße Verbindung der Formel (I-C) 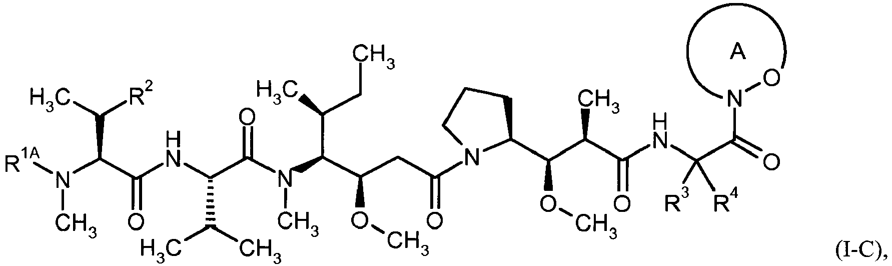 in welcher R^{1A}, R², R³, R⁴ und der Ring A die oben angegebenen Bedeutungen haben,
überführt oder *(ii)* durch Umsetzung mit einer Verbindung der Formel (IX) 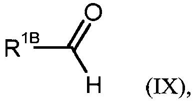 in welcher
- R^{1B}: die oben angegebene, jedoch in der Alkyl-Kettenlänge um eine CH₂-Einheit verkürzte Bedeutung von R^{1A} hat,
in Gegenwart eines geeigneten Reduktionsmittels in eine erfindungsgemäße Verbindung der Formel (I-D) 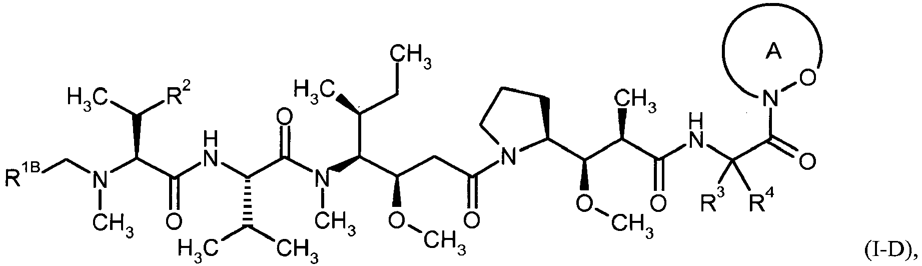 in welcher R^{1B}, R², R³, R⁴ und der Ring A die oben angegebenen Bedeutungen haben,
überführt
und die so erhaltenen Verbindungen der Formeln (I-B), (I-C) bzw. (I-D) gegebenenfalls in ihre Enantiomere und/oder Diastereomere trennt und/oder mit den entsprechenden (i) Lösungsmitteln und/oder *(ii)* Basen oder Säuren zu ihren Solvaten, Salzen und/oder Solvaten der Salze umsetzt.

Die oben beschriebenen Kupplungsreaktionen (Amid-Bildung aus jeweiliger Amin- und Carbonsäure-Komponente) werden nach allgemein üblichen Methoden der Peptidchemie durchgeführt [siehe z.B. M. Bodanszky, Principles of Peptide Synthesis, Springer-Verlag, Berlin, 1993; M. Bodanszky und A. Bodanszky, The Practice of Peptide Synthesis, Springer-Verlag, Berlin, 1984; H.-D. Jakubke und H. Jeschkeit, Aminosäuren, Peptide, Proteine, Verlag Chemie, Weinheim, 1982].

Inerte Lösungsmittel für die Kupplungsreaktionen (II) + (III) → (IV), (IV) + (V) → (VI) und (II) + (VII) → (VI) sind beispielsweise Ether wie Diethylether, Diisopropylether, *tert*.-Butylmethylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Heptan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder dipolar-aprotische Lösungsmittel wie Aceton, Methylethylketon, Acetonitril, Ethylacetat, Pyridin, Dimethylsulfoxid (DMSO), *N,N*-Dimethylformamid (DMF), *N,N*-Dimethylacetamid (DMA), *N,N*'-Dimethylpropylenharnstoff (DMPU) oder *N*-Methylpyrrolidinon (NMP). Ebenso ist es möglich, Gemische solcher Lösungsmittel zu verwenden. Bevorzugt wird *N,N*-Dimethylformamid eingesetzt.

Als Aktivierungs-/Kondensationsmittel für diese Kupplungen eignen sich beispielsweise Carbodiimide wie *N,N'*-Diethyl-, *N,N*'-Dipropyl-, *N*,*N'*-Diisopropyl-, *N,N*'-Dicyclohexylcarbodiimid (DCC) oder *N*-(3-Dimethylaminoisopropyl)-*N'*-ethylcarbodiimid-Hydrochlorid (EDC), Phosgen-Derivate wie *N*,*N'*-Carbonyldiimidazol (CDI) oder Isobutylchlorformiat, 1,2-Oxazolium-Verbindungen wie 2-Ethyl-5-phenyl-1,2-oxazolium-3-sulfat oder 2-*tert*.-Butyl-5-methylisoxazolium-perchlorat, Acylamino-Verbindungen wie 2-Ethoxy-1-ethoxycarbonyl-1,2-dihydrochinolin, Phosphor-Verbindungen wie Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat oder Benzotriazol-1-yloxy-tris(pyrrolidino)phosphonium-hexafluorophosphat (PyBOP), oder Uronium-Verbindungen wie *O*-(Benzotriazol-1-yl)-*N*,*N*,*N'*,*N'*-tetramethyluronium-tetrafluoroborat (TBTU), *O*-(Benzotriazol-1-yl)-*N*,*N*,*N'*,*N'*-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N*,*N*,*N'*,*N'*-tetramethyluronium-hexafluorophosphat (HATU) oder *O*-(1*H*-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder tertiäre Aminbasen wie Triethylamin, *N*-Methylmorpholin, *N*-Methylpiperidin, *N*,*N*-Diisopropylethylamin, Pyridin oder 4-*N*,*N*-Dimethylaminopyridin.

Im Rahmen der vorliegenden Erfindung wird als Aktivierungs-/Kondensationsmittel für solche Kupplungsreaktionen bevorzugt *N*-(3-Dimethylaminoisopropyl)-*N'*-ethylcarbodiimid-Hydrochlorid (EDC) in Kombination mit 1-Hydroxybenzotriazol (HOBt) und *N*,*N*-Diisopropylethylamin, oder *O-*(7-Azabenzotriazol-1-yl)-*N*,*N*,*N',N'*-tetramethyluronium-hexafluorophosphat (HATU) gleichfalls in Verbindung mit *N*,*N*-Disopropylethylamin verwendet.

Die Kupplungsreaktionen (II) + (III) → (IV), (IV) + (V) → (VI) und (II) + (VII) → (VI) werden in der Regel in einem Temperaturbereich von -20°C bis +60°C, bevorzugt bei 0°C bis +40°C durchgeführt. Die Umsetzungen können bei normalem, bei erhöhtem oder bei erniedrigtem Druck erfolgen (z.B. von 0.5 bis 5 bar); im Allgemeinen arbeitet man bei Normaldruck.

Als inerte Lösungsmittel für die Umsetzung (I-B) + (VIII) → (I-C) eignen sich beispielsweise Ether wie Diethylether, Diisopropylether, Methyl-*tert*.-butylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Heptan, Cyclohexan oder Erdölfraktionen, oder dipolar-aprotische Lösungsmittel wie Aceton, Methylethylketon, Acetonitril, Dimethylsulfoxid (DMSO), *N*,*N*-Dimethylformamid (DMF), *N*,*N*-Dimethylacetamid (DMA), *N*,*N'*-Dimethylpropylenharnstoff (DMPU), *N*-Methylpyrrolidinon (NMP) oder Pyridin. Ebenso ist es möglich, Gemische solcher Lösungsmittel einzusetzen. Bevorzugt wird *N*,*N*-Dimethylformamid verwendet.

Geeignete Basen für diese Alkylierungsreaktion sind insbesondere Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, oder übliche organische Amine wie Triethylamin, *N*-Methylmorpholin, *N*-Methylpiperidin, *N*,*N*-Diisopropylethylamin, Pyridin oder *4-N,N-*Dimethylaminopyridin. Bevorzugt wird Kalium- oder Cäsiumcarbonat verwendet. Gegebenenfalls ist der Zusatz eines Alkylierungskatalysators von Vorteil, wie beispielsweise Lithiumbromid oder -iodid, Natrium- oder Kaliumiodid, Tetra-*n*-butylammoniumbromid oder -iodid oder Benzyltriethylammoniumbromid.

Die Reaktion (I-B) + (VIII) → (I-C) wird im Allgemeinen in einem Temperaturbereich von -20°C bis +60°C, bevorzugt bei 0°C bis +40°C durchgeführt. Die Umsetzung kann bei normalem, bei erhöhtem oder bei erniedrigtem Druck erfolgen (z.B. von 0.5 bis 5 bar); in der Regel arbeitet man bei Normaldruck.

Die Umsetzung (I-B) + (IX) → (I-D) erfolgt in den für eine reduktive Aminierung üblichen, unter den Reaktionsbedingungen inerten Lösungsmitteln, gegebenenfalls in Gegenwart einer Säure und/ oder eines wasserentziehenden Mittels als Katalysator. Zu solchen Lösungsmitteln gehören beispielsweise Alkohole wie Methanol, Ethanol, *n*-Propanol, Isopropanol, *n*-Butanol oder *tert*.-Butanol, Ether wie Tetrahydrofuran, 1,4-Dioxan, 1,2-Dimethoxyethan oder Bis-(2-methoxyethyl)-ether, oder andere Solventien wie Dichlormethan, 1,2-Dichlorethan, *N*,*N*-Dimethylformamid oder auch Wasser. Ebenso ist es möglich, Gemische dieser Lösungsmittel einzusetzen. Bevorzugt wird als Lösungsmittel ein 1,4-Dioxan/Wasser-Gemisch verwendet unter Zusatz von Essigsäure oder verdünnter Salzsäure als Katalysator.

Als Reduktionsmittel eignen sich für diese Reaktion insbesondere komplexe Borhydride, wie beispielsweise Natriumborhydrid, Natriumcyanoborhydrid, Natriumtriacetoxyborhydrid oder Tetra-*n*-butylammoniumborhydrid. Bevorzugt wird Natriumcyanoborhydrid eingesetzt.

Die Umsetzung (I-B) + (IX) → (I-D) erfolgt im Allgemeinen in einem Temperaturbereich von 0°C bis +120°C, bevorzugt bei +50°C bis +100°C. Die Reaktion kann bei normalem, bei erhöhtem oder bei erniedrigtem Druck durchgeführt werden (z.B. von 0.5 bis 5 bar); in der Regel arbeitet man bei Normaldruck.

In den Resten R¹, R^{1A}, R^{1B}, R², R⁴, R⁵ und/oder R⁶ gegebenenfalls vorhandene funktionelle Gruppen - wie insbesondere Amino-, Hydroxy- und Carboxylgruppen - können bei den zuvor beschriebenen Verfahrensschritten, falls zweckmäßig oder erforderlich, auch in temporär geschützter Form vorliegen. Die Einführung und Entfernung solcher Schutzgruppen erfolgt hierbei nach üblichen, aus der Peptidchemie bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984]. Bei Vorhandensein mehrerer geschützter Gruppen kann deren Wiederfreisetzung gegebenenfalls simultan in einer Eintopf-Reaktion oder auch in separaten Reaktionsschritten vorgenommen werden.

Als Amino-Schutzgruppe wird bevorzugt *tert*.-Butoxycarbonyl (Boc), Benzyloxycarbonyl (Z) oder (9*H*-Fluoren-9-ylmethoxy)carbonyl (Fmoc) verwendet; für eine Hydroxy- oder Carboxyl-Funktion wird vorzugsweise *tert*.-Butyl oder Benzyl als Schutzgruppe eingesetzt. Die Abspaltung einer *tert.-*Butyl- oder *tert.*-Butoxycarbonyl-Gruppe geschieht üblicherweise durch Behandlung mit einer starken Säure, wie Chlorwasserstoff, Bromwasserstoff oder Trifluoressigsäure, in einem inerten Lösungsmittel wie Diethylether, 1,4-Dioxan, Dichlormethan oder Essigsäure; gegebenenfalls kann diese Reaktion auch ohne Zusatz eines inerten Lösungsmittels durchgeführt werden. Im Falle von Benzyl oder Benzyloxycarbonyl als Schutzgruppe werden diese bevorzugt durch Hydrogenolyse in Gegenwart eines geeigneten Palladium-Katalysators, wie beispielsweise Palladium auf Aktivkohle, entfernt. Die (9*H*-Fluoren-9-ylmethoxy)carbonyl-Gruppe wird im Allgemeinen mit Hilfe einer sekundären Aminbase wie Diethylamin oder Piperidin abgespalten.

Die Verbindungen der Formel (II) können nach üblichen Methoden der Peptidchemie beispielsweise dadurch hergestellt werden, dass man zunächst *N*-(Benzyloxycarbonyl)-L-Valin der Formel (X) 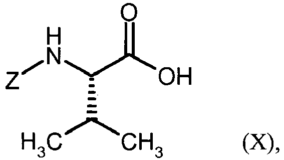 in welcher Z für die Benzyloxycarbonyl-Schutzgruppe steht,
mit Hilfe eines Kondensationsmittels mit einer Verbindung der Formel (XI) 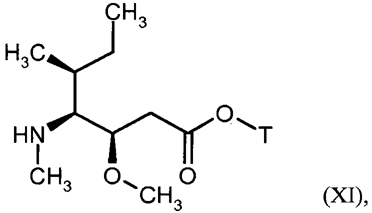 in welcher T für (C₁-C₄)-Alkyl steht,
oder einem Salz dieser Verbindung zu einer Verbindung der Formel (XII) 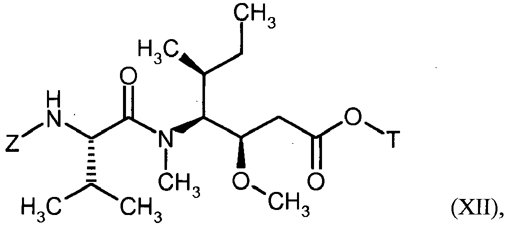 in welcher T und Z die oben angegebenen Bedeutungen haben,
kuppelt, diese nach hydrogenolytischer Entfernung der Z-Schutzgruppe dann in Gegenwart eines Kondensationsmittels mit *N*-geschütztem *N*-Methyl-L-valin oder *N*-Methyl-L-threonin der Formel (XIII) 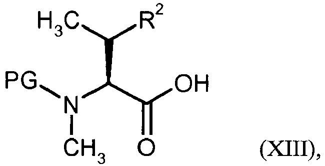 in welcher R² die oben angegebene Bedeutung hat
und
- PG: für eine Amino-Schutzgruppe wie beispielsweise (9*H*-Fluoren-9-ylmethoxy)carbonyl, *tert.-*Butoxycarbonyl oder Benzyloxycarbonyl steht,
zu einer Verbindung der Formel (XIV) 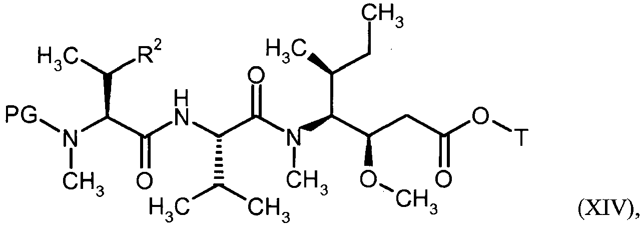 in welcher R², PG und T die oben angegebenen Bedeutungen haben,
kuppelt und abschließend die Ester-Gruppierung -C(O)OT in (XIV) nach üblichen Methoden in die freie Carbonsäure überführt.

Die Kupplungen (X) + (XI) → (XII) und Z-entschütztes (XII) + (XIII) → (XIV) werden unter analogen Reaktionsbedingungen durchgeführt wie zuvor bei den in Verfahren [A] und [B] dargestellten Kupplungsschritten beschrieben.

Die Hydrolyse der Ester-Gruppe -C(O)OT im Verfahrensschritt (XIV) → (II) wird nach üblichen Methoden durchgeführt, indem man den Ester in einem inerten Lösungsmittel mit einer Säure oder einer Base behandelt, wobei bei letzterer Variante das zunächst entstehende Carboxylat-Salz durch nachfolgende Zugabe einer Säure in die freie Carbonsäure überführt wird. Im Falle eines *tert*.-Butyl-esters erfolgt die Spaltung bevorzugt mittels einer Säure.

Der Alkyl-Rest T in Verbindung (XI) wird hierbei so gewählt, dass die Bedingungen seiner Abspaltung kompatibel mit der jeweils eingesetzten Schutzgruppe PG aus Verbindung (XIII) sind.

Als Base für die Ester-Hydrolyse sind die üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt sind Lithium-, Natrium- oder Kaliumhydroxid.

Als Säure eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle eines *tert*.-Butylesters und Salzsäure bei einem Methylester.

Als inerte Lösungsmittel eignen sich für diese Reaktionen Wasser oder die für eine Esterspaltung üblichen organischen Lösungsmittel. Hierzu gehören bevorzugt niedere Alkohole wie Methanol, Ethanol, *n*-Propanol oder Isopropanol, Ether wie Diethylether, Tetrahydrofuran, 1,4-Dioxan oder 1,2-Dimethoxyethan, oder andere Lösungsmittel wie Dichlormethan, Aceton, Methylethylketon, *N,N-*Dimethylformamid oder Dimethylsulfoxid. Ebenso ist es möglich, Gemische dieser Lösungsmittel einzusetzen. Im Falle einer basischen Ester-Hydrolyse werden bevorzugt Gemische von Wasser mit 1,4-Dioxan, Tetrahydrofuran, Methanol, Ethanol und/oder Dimethylformamid verwendet. Im Falle der Umsetzung mit Trifluoressigsäure wird bevorzugt Dichlormethan und im Falle der Umsetzung mit Chlorwasserstoff bevorzugt Tetrahydrofuran, Diethylether, 1,4-Dioxan oder Wasser eingesetzt.

Die Esterspaltung erfolgt im Allgemeinen in einem Temperaturbereich von -20°C bis +100°C, bevorzugt bei 0°C bis +50°C.

Die Verbindungen der Formel (V) können in Analogie zu bekannten Verfahren beispielsweise dadurch hergestellt werden, dass man eine geschützte Aminosäure der Formel (XV) 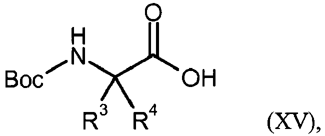 in welcher R³ und R⁴ die oben angegebenen Bedeutungen haben und Boc für die *tert*.-Butoxycarbonyl-Schutzgruppe steht,
unter Aktivierung der Carboxyl-Funktion entweder
[C] mit einer Verbindung der Formel (XVI) 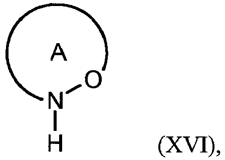 in welcher der Ring A die oben angegebene Bedeutung hat,
   oder einem Salz dieser Verbindung zu einer Verbindung der Formel (XVII) 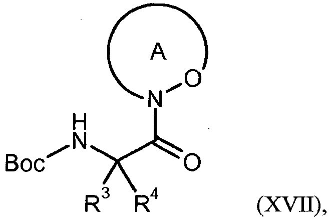 in welcher R³, R⁴, der Ring A und Boc die oben angegebenen Bedeutungen haben,
   kuppelt
   oder
[D] zunächst mit Hydroxylamin oder einem Salz hiervon zu einer Verbindung der Formel (XVIII) 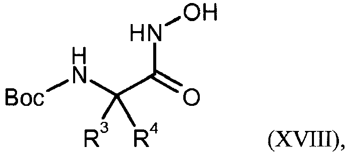 in welcher R³, R⁴ und Boc die oben angegebenen Bedeutungen haben,
   kuppelt und diese anschließend in Gegenwart einer Base mit einem Dibromid der Formel (XIX) 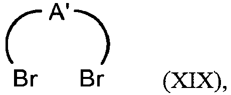 in welcher die verbindende Gruppe A' den übrigen, gegebenenfalls substituierten Elementen des oben definierten Ringes A - die N-O-Gruppierung ausgenommen - entspricht,
   unter Cyclisierung gleichfalls zu der Verbindung der Formel (XVII) 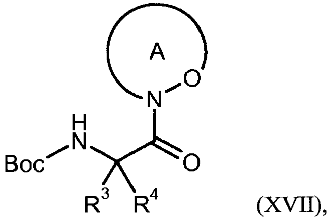 in welcher R³, R⁴, der Ring A und Boc die oben angegebenen Bedeutungen haben,
   alkyliert
und dann die Boc-Schutzgruppe in (XVII) auf üblichem Wege durch Behandlung mit einer Säure abspaltet.

Die Kupplungen (XV) + (XVI) → (XVII) und (XV) + Hydroxylamin → (XVIII) werden unter analogen Reaktionsbedingungen durchgeführt wie zuvor bei den in Verfahren [A] und [B] dargestellten Kupplungsschritten beschrieben.

Als Base für die Cyclo-Alkylierung im Verfahrensschritt (XVIII) + (XIX) → (XVII) werden vorzugsweise Alkalihydroxide, wie beispielsweise Lithium-, Natrium- oder Kaliumhydroxid, oder Alkali- oder Erdalkalicarbonate, wie Lithium-, Natrium-, Kalium-, Calcium- oder Cäsiumcarbonat, eingesetzt.

Als inerte Lösungsmittel eignen sich für diese Reaktion insbesondere Alkohole wie Methanol, Ethanol, *n*-Propanol, Isopropanol, *n*-Butanol oder *tert*.-Butanol, oder dipolar-aprotische Lösungsmittel wie Aceton, Methylethylketon, *N,N*-Dimethylformamid oder Dimethylsulfoxid. Bevorzugt wird Aceton verwendet.

Die Umsetzung (XVIII) + (XIX) → (XVII) erfolgt im Allgemeinen in einem Temperaturbereich von +20°C bis +120°C, bevorzugt bei +50°C bis +80°C. Die Reaktion kann bei normalem, bei erhöhtem oder bei erniedrigtem Druck durchgeführt werden (z.B. von 0.5 bis 5 bar); in der Regel arbeitet man bei Normaldruck.

Die Verbindungen der Formel (VII) ihrerseits sind durch Kupplung der oben beschriebenen Verbindung (V) mit der Verbindung (XX) 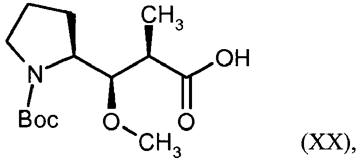 in welcher Boc für die *tert*.-Butoxycarbonyl-Schutzgruppe steht,
zu einer Verbindung der Formel (XXI) 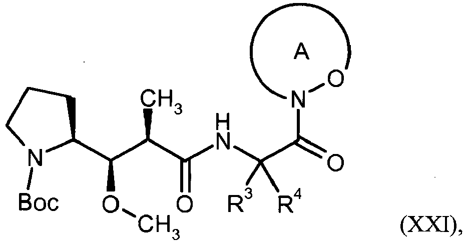 in welcher R³, R⁴, der Ring A und Boc die oben angegebenen Bedeutungen haben,
und nachfolgende Abspaltung der Boc-Schutzgruppe zugänglich. Die Verbindung (XX) selbst ist über eine entsprechende Schutzgruppen-Einführung aus der Verbindung (III) erhältlich.

Die Kupplungsreaktion (V) + (XX) → (XXI) wird wiederum unter analogen Bedingungen durchgeführt wie zuvor bei den in Verfahren [A] und [B] dargestellten Kupplungsschritten beschrieben.

Die Verbindungen der Formeln (III), (VIII), (IX), (X), (XI), (XIII), (XV), (XVI) und (XIX) einschließlich, wo zutreffend, chiraler oder diastereomerer Formen hiervon sind kommerziell erhältlich oder als solche in der Literatur beschrieben, oder sie können auf für den Fachmann offenkundigem Wege in Analogie zu in der Literatur publizierten Methoden hergestellt werden. Zahlreiche ausführliche Vorschriften sowie Literaturangaben zur Herstellung der Ausgangsmaterialien befinden sich auch im Experimentellen Teil im Abschnitt zur Herstellung der Ausgangsverbindungen und Intermediate.

Sollten entsprechende isomerenreine Ausgangsmaterialien nicht zur Verfügung stehen, so kann eine Trennung der erfindungsgemäßen Verbindungen in die korrespondierenden Enantiomere und/ oder Diastereomere zweckmäßigerweise auch bereits auf der Stufe der Verbindungen (V), (VI), (VII), (XV), (XVI), (XVII), (XVIII) oder (XXI) erfolgen, welche dann in separierter Form gemäß den zuvor beschriebenen Reaktionsschritten weiter umgesetzt werden. Eine solche Auftrennung der Stereoisomeren läßt sich nach üblichen, dem Fachmann bekannten Methoden durchführen. Bevorzugt werden chromatographische Verfahren an achiralen bzw. chiralen Trennphasen angewandt; im Falle von freien Carbonsäuren als Zwischenprodukten kann alternativ auch eine Trennung über diastereomere Salze mit Hilfe chiraler Basen erfolgen.

Die Herstellung der erfindungsgemäßen Verbindungen kann durch die folgenden Reaktionsschemata beispielhaft veranschaulicht werden:

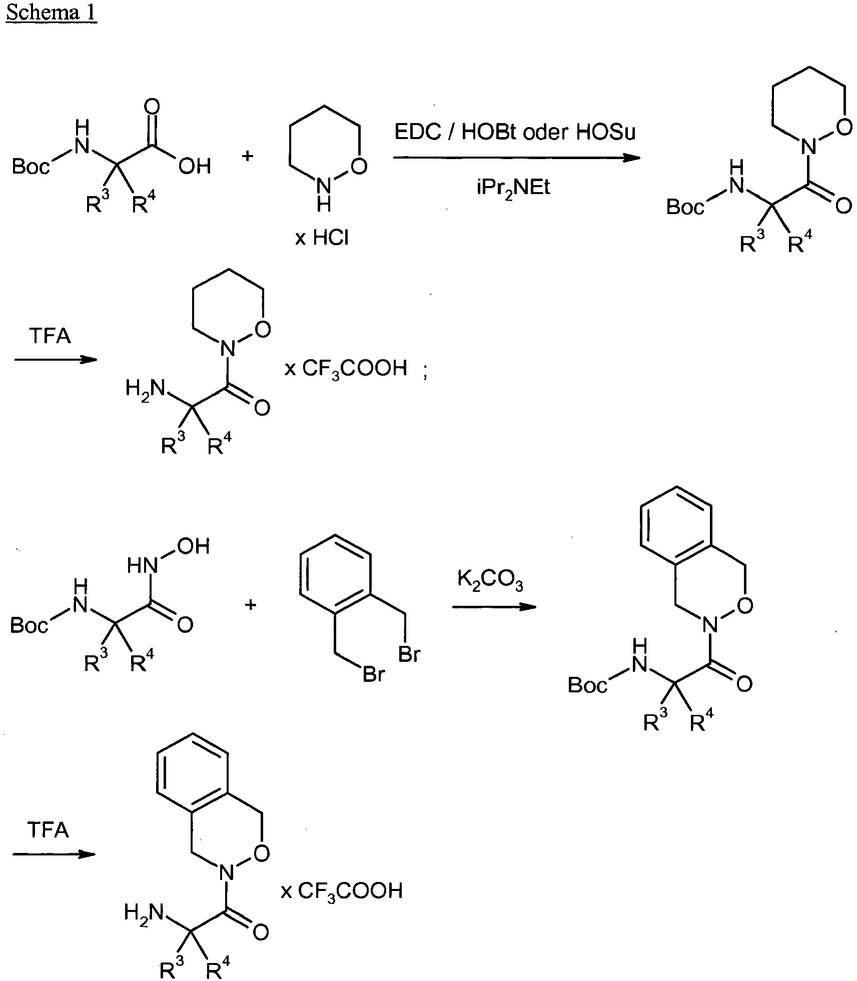

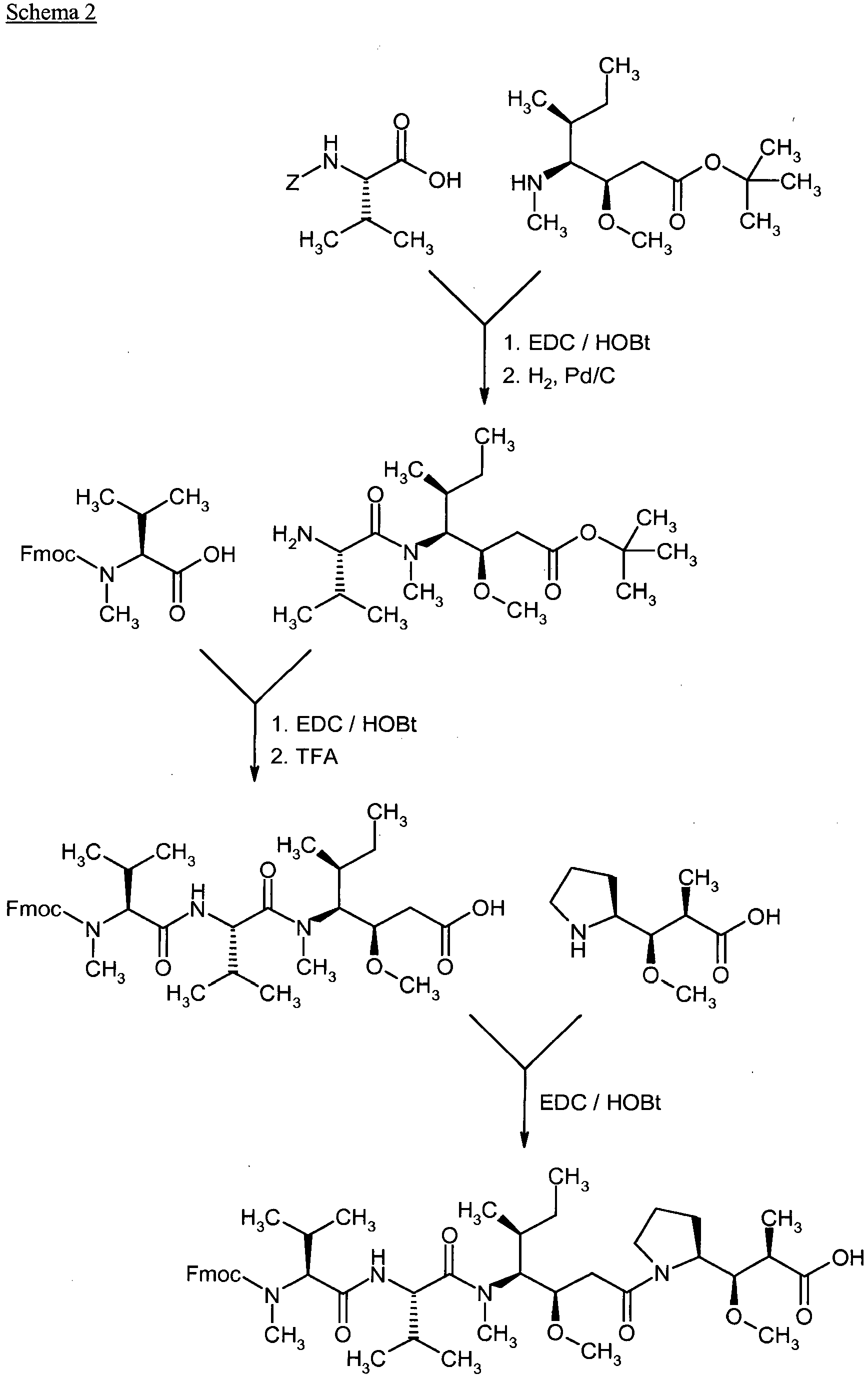

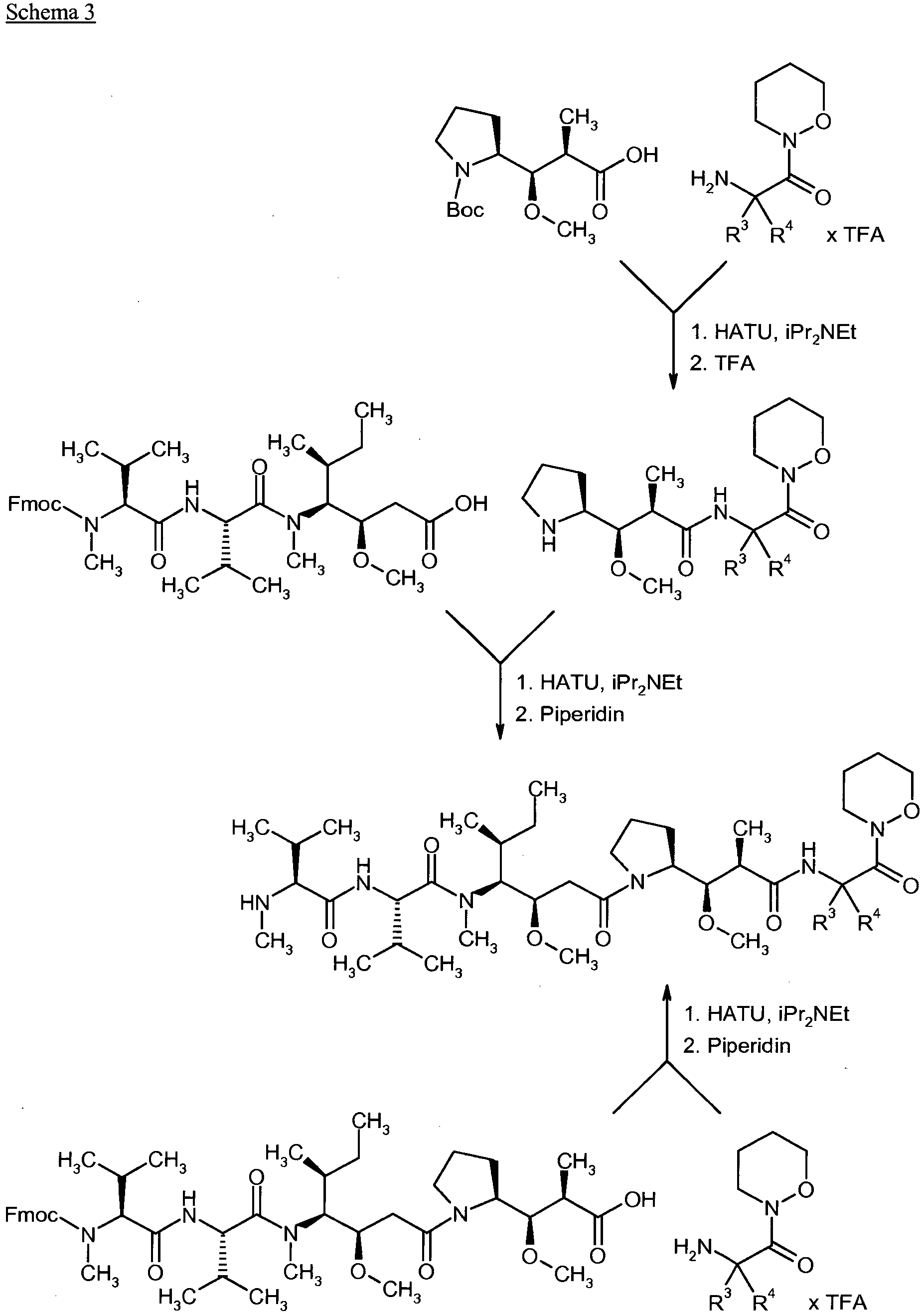

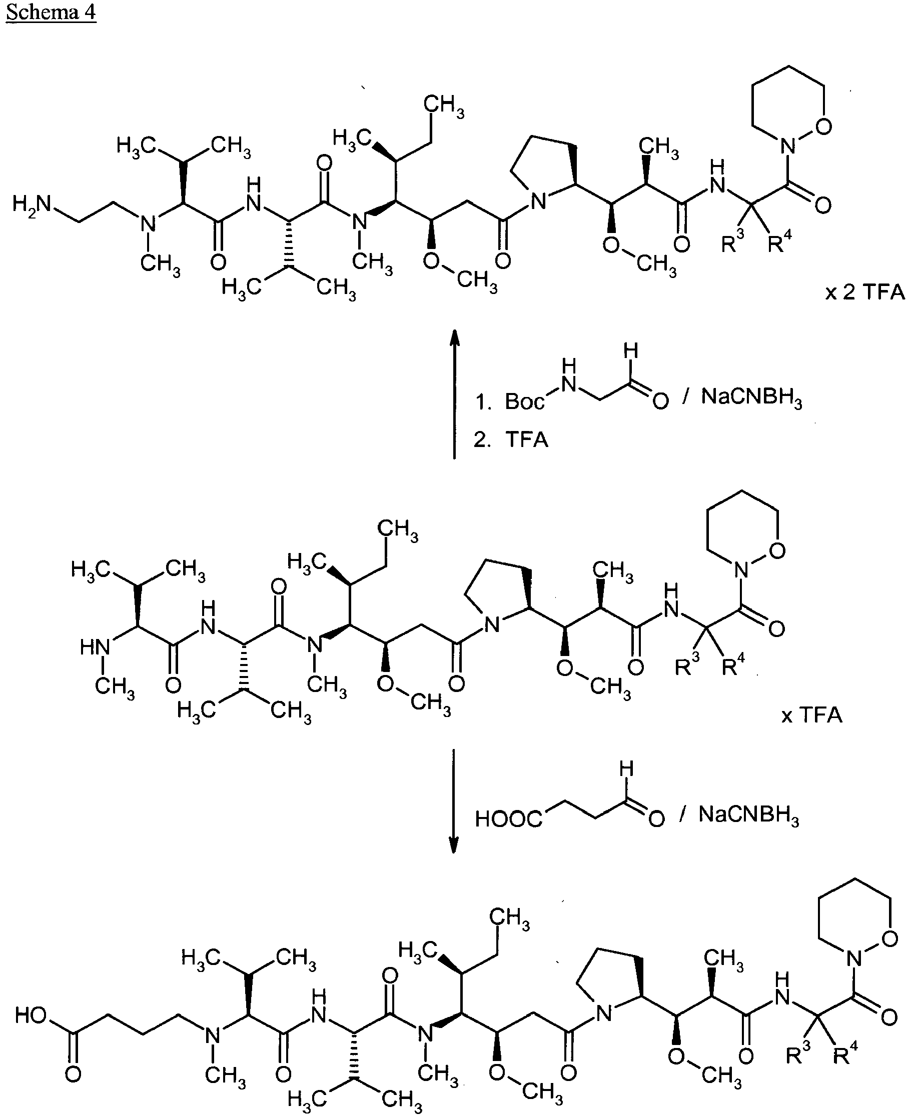

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen weisen im Vergleich zu Monomethylauristatin F (MMAF) eine deutlich stärkere cytotoxische Aktivität auf und besitzen zudem gegenüber bekannten Ester-Derivaten von MMAF, wie MMAF-OMe, eine signifikant erhöhte Stabilität in Plasma. Die erfindungsgemäßen Verbindungen sind aufgrund dieses Eigenschaftsprofils daher in besonderem Maße zur Behandlung von hyperproliferativen Erkrankungen beim Menschen und bei Säugetieren allgemein geeignet. Die Verbindungen können einerseits die Zellproliferation und Zellteilung hemmen, blockieren, verringern oder senken und andererseits die Apoptose verstärken.

Zu den hyperproliferativen Erkrankungen, zu deren Behandlung die erfindungsgemäßen Verbindungen eingesetzt werden können, zählt insbesondere die Gruppe der Krebs- und Tumorerkrankungen. Hierunter werden im Rahmen der vorliegenden Erfindung insbesondere die folgenden Erkrankungen verstanden, ohne jedoch auf sie beschränkt zu sein: Brustkarzinome und Brusttumore (ductale und lobuläre Formen, auch *in situ*), Atemwegstumore (kleinzelliges und nicht-kleinzelliges Karzinom, Bronchialkarzinom), Hirntumore (z.B. des Hirnstamms und des Hypothalamus, Astrocytoma, Medulloblastoma, Ependymoma sowie neuro-ectodermale und pineale Tumore), Tumore der Verdauungsorgane (Speiseröhre, Magen, Gallenblase, Dünndarm, Dickdarm, Rektum), Lebertumore (u.a. hepatozelluläres Karzinom, Cholangiokarzinom und gemischt-hepatozelluläres Cholangiokarzinom), Tumore des Kopf- und Halsbereiches (Larynx, Hypopharynx, Nasopharynx, Oropharynx, Lippen und Mundhöhle), Hauttumore (Plattenepithelkarzinom, Kaposi-Sarkom, malignes Melanom, Merkelzell-Hautkrebs und nicht-melanomartiger Hautkrebs), Tumore der Weichteile (u.a. Weichteilsarkome, Osteosarkome, maligne fibröse Histiozytome, Lymphosarkome und Rhabdomyosarkome), Tumore der Augen (u.a. intraokuläres Melanom und Retinoblastom), Tumore der endokrinen und exokrinen Drüsen (z.B. thyroide und parathyroide Drüsen, Bauchspeicheldrüse und Speicheldrüse), Tumore des Harntrakts (Blasen-, Penis-, Nieren-, Nierenbecken- und Harnleitertumore) sowie Tumore der reproduktiven Organe (Endometrium-, Zervix-, Ovarial-, Vaginal-, Vulva- und Uteruskarzinome der Frau sowie Prostata- und Hodenkarzinome des Mannes). Dazu gehören auch proliferative Bluterkrankungen in solider Form und als zirkulierende Blutzellen, wie Lymphome, Leukämien und myeloproliferative Erkrankungen, z.B. akute myeloide, akute lymphoblastische, chronisch-lymphozytische, chronisch-myelogene und Haarzell-Leukämie, sowie AIDS-korrelierte Lymphome, Hodgkin-Lymphome, Non-Hodgkin-Lymphome, kutane T-Zell-Lymphome, Burkitt-Lymphome und Lymphome im zentralen Nervensystem.

Diese gut beschriebenen Krankheiten des Menschen können mit vergleichbarer Ätiologie auch in anderen Säugetieren vorkommen und dort mit den Verbindungen der vorliegenden Erfindung behandelt werden.

Der Begriff "Behandlung" oder "behandeln" wird im Rahmen dieser Erfindung konventionell verwendet und bedeutet die Versorgung, Pflege und Betreuung eines Patienten mit dem Ziel, eine Krankheit oder gesundheitliche Abweichung zu bekämpfen, zu verringern, abzuschwächen oder zu erleichtern und die Lebensbedingungen zu verbessern, die durch diese Krankheit beeinträchtigt werden, wie beispielsweise bei einer Krebserkrankung.

Weiterer Gegenstand der vorliegenden Erfindung ist somit die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Beispielsweise können die Verbindungen der vorliegenden Erfindung mit bekannten anti-hyperproliferativen, zytostatischen oder zytotoxischen Substanzen zur Behandlung von Krebserkrankungen kombiniert werden. Als geeignete Kombinationswirkstoffe seien beispielhaft genannt:

Aldesleukin, Alendronsäure, Alfaferon, Alitretinoin, Allopurinol, Aloprim, Aloxi, Altretamin, Aminoglutethimid, Amifostin, Amrubicin, Amsacrin, Anastrozol, Anzmet, Aranesp, Arglabin, Arsentrioxid, Aromasin, 5-Azacytidin, Azathioprin, BCG oder tice-BCG, Bestatin, Betamethason-Acetat, Betamethason-Natriumphosphat, Bexaroten, Bleomycin-Sulfat, Broxuridin, Bortezomib, Busulfan, Calcitonin, Campath, Capecitabin, Carboplatin, Casodex, Cefeson, Celmoleukin, Cerubidin, Chlorambucil, Cisplatin, Cladribin, Clodronsäure, Cyclophosphamid, Cytarabin, Dacarbazin, Dactinomycin, DaunoXome, Decadron, Decadron-Phosphat, Delestrogen, Denileukin Diftitox, Depomedrol, Deslorelin, Dexrazoxan, Diethylstilbestrol, Diflucan, Docetaxel, Doxifluridin, Doxorubicin, Dronabinol, DW-166HC, Eligard, Elitek, Ellence, Emend, Epirubicin, Epoetin-alfa, Epogen, Eptaplatin, Ergamisol, Estrace, Estradiol, Estramustin-Natriumphosphat, Ethinylestradiol, Ethyol, Etidronsäure, Etopophos, Etoposid, Fadrozol, Farston, Filgrastim, Finasterid, Fligrastim, Floxuridin, Fluconazol, Fludarabin, 5-Fluordeoxyuridin-Monophosphat, 5-Fluoruracil (5-FU), Fluoxymesteron, Flutamid, Formestan, Fosteabin, Fotemustin, Fulvestrant, Gammagard, Gemcitabin, Gemtuzumab, Gleevec, Gliadel, Goserelin, Granisetron-Hydrochlorid, Histrelin, Hycamtin, Hydrocorton, erythro-Hydroxynonyladenin, Hydroxyharnstoff, Ibritumomab Tiuxetan, Idarubicin, Ifosfamid, Interferon-alpha, Interferon-alpha-2, Interferon-alpha-2a, Interferon-alpha-2β, Interferon-alpha-n1, Interferon-alpha-n3, Interferon-beta, Interferon-gamma-1α, Interleukin-2, Intron A, Iressa, Irinotecan, Kytril, Lentinan-Sulfat, Letrozol, Leucovorin, Leuprolid, Leuprolid-Acetat, Levamisol, Levofolinsäure-Calciumsalz, Levothroid, Levoxyl, Lomustin, Lonidamin, Marinol, Mechlorethamin, Mecobalamin, Medroxyprogesteron-Acetat, Megestrol-Acetat, Melphalan, Menest, 6-Mercaptopurin, Mesna, Methotrexat, Metvix, Miltefosin, Minocyclin, Mitomycin C, Mitotan, Mitoxantron, Modrenal, Myocet, Nedaplatin, Neulasta, Neumega, Neupogen, Nilutamid, Nolvadex, NSC-631570, OCT-43, Octreotid, Ondansetron-Hydrochlorid, Orapred, Oxaliplatin, Paclitaxel, Pediapred, Pegaspargase, Pegasys, Pentostatin, Picibanil, Pilocarpin-Hydrochlorid, Pirarubicin, Plicamycin, Porfimer-Natrium, Prednimustin, Prednisolon, Prednison, Premarin, Procarbazin, Procrit, Raltitrexed, Rebif, Rhenium-186-Etidronat, Rituximab, Roferon-A, Romurtid, Salagen, Sandostatin, Sargramostim, Semustin, Sizofiran, Sobuzoxan, Solu-Medrol, Streptozocin, Strontium-89-chlorid, Synthroid, Tamoxifen, Tamsulosin, Tasonermin, Tastolacton, Taxoter, Teceleukin, Temozolomid, Teniposid, Testosteron-Propionat, Testred, Thioguanin, Thiotepa, Thyrotropin, Tiludronsäure, Topotecan, Toremifen, Tositumomab, Tastuzumab, Teosulfan, Tretinoin, Trexall, Trimethylmelamin, Trimetrexat, Triptorelin-Acetat, Triptorelin-Pamoat, UFT, Uridin, Valrubicin, Vesnarinon, Vinblastin, Vincristin, Vindesin, Vinorelbin, Virulizin, Zinecard, Zinostatin-Stimalamer, Zofran; ABI-007, Acolbifen, Actimmun, Affinitak, Aminopterin, Arzoxifen, Asoprisnil, Atamestan, Atrasentan, Avastin, BAY 43-9006 (Sorafenib), CCI-779, CDC-501, Celebrex, Cetuximab, Crisnatol, Cyproteron-Acetat, Decitabin, DN-101, Doxorubicin-MTC, dSLIM, Dutasterid, Edotecarin, Eflornithin, Exatecan, Fenretinid, Histamin-Dihydrochlorid, Histrelin-Hydrogel-Implant, Holmium-166-DOTMP, Ibandronsäure, Interferon-gamma, Intron-PEG, Ixabepilon, Keyhole Limpet-Hemocyanin, L-651582, Lanreotid, Lasofoxifen, Libra, Lonafarnib, Miproxifen, Minodronat, MS-209, liposomales MTP-PE, MX-6, Nafarelin, Nemorubicin, Neovastat, Nolatrexed, Oblimersen, Onko-TCS, Osidem, Paclitaxel-Polyglutamat, Pamidronat-Dinatrium, PN-401, QS-21, Quazepam, R-1549, Raloxifen, Ranpirnas, 13-*cis*-Retinsäure, Satraplatin, Seocalcitol, T-138067, Tarceva, Taxoprexin, Thymosin-alpha-1, Tiazofurin, Tipifarnib, Tirapazamin, TLK-286, Toremifen, TransMID-107R, Valspodar, Vapreotid, Vatalanib, Verteporfin, Vinflunin, Z-100, Zoledronsäure, sowie Kombinationen hiervon.

In einer bevorzugten Ausführungsform können die Verbindungen der vorliegenden Erfindung mit anti-hyperproliferativen Agentien kombiniert werden, welche beispielhaft - ohne dass diese Aufzählung abschließend wäre - sein können:

Aminoglutethimid, L-Asparaginase, Azathioprin, 5-Azacytidin, Bleomycin, Busulfan, Carboplatin, Carmustin, Chlorambucil, Cisplatin, Colaspase, Cyclophosphamid, Cytarabin, Dacarbazin, Dactinomycin, Daunorubicin, Diethylstilbestrol, 2',2'-Difluordeoxycytidin, Docetaxel, Doxorubicin (Adriamycin), Epirubicin, Epothilon und seine Derivate, erythro-Hydroxynonyladenin, Ethinylestradiol, Etoposid, Fludarabin-Phosphat, 5-Fluordeoxyuridin, 5-Fluordeoxyuridin-Monophosphat, 5-Fluoruracil, Fluoxymesteron, Flutamid, Hexamethylmelamin, Hydroxyharnstoff, Hydroxyprogesteron-Caproat, Idarubicin, Ifosfamid, Interferon, Irinotecan, Leucovorin, Lomustin, Mechlorethamin, Medroxyprogesteron-Acetat, Megestrol-Acetat, Melphalan, 6-Mercaptopurin, Mesna, Methotrexat, Mitomycin C, Mitotan, Mitoxantron, Paclitaxel, Pentostatin, *N*-Phosphonoacetyl-L-aspartat (PALA), Plicamycin, Prednisolon, Prednison, Procarbazin, Raloxifen, Semustin, Streptozocin, Tamoxifen, Teniposid, Testosteron-Propionat, Thioguanin, Thiotepa, Topotecan, Trimethylmelamin, Uridin, Vinblastin, Vincristin, Vindesin und Vinorelbin.

In viel versprechender Weise lassen sich die erfindungsgemäßen Verbindungen auch mit biologischen Therapeutika wie Antikörpern (z.B. Avastin, Rituxan, Erbitux, Herceptin) kombinieren. Die erfindungsgemäßen Verbindungen können auch in Kombination mit gegen die Angiogenese gerichteten Therapien positive Effekte erzielen, wie zum Beispiel mit Avastin, Axitinib, Recentin, Regorafenib, Sorafenib oder Sunitinib. Kombinationen mit Inhibitoren des Proteasoms und von mTOR sowie Kombinationen mit Antihormonen und steroidalen metabolischen Enzyminhibitoren sind wegen ihres günstigen Nebenwirkungsprofils ebenfalls besonders geeignet.

Generell können mit der Kombination von Verbindungen der vorliegenden Erfindung mit anderen, zytostatisch oder zytotoxisch wirksamen Agentien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Kombination mit einer Strahlentherapie und/oder einer chirurgischen Intervention eingesetzt werden.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie beispielsweise oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Ac: Acetyl
- aq.: wässrig, wässrige Lösung
- Boc: *tert*.-Butoxycarbonyl
- br.: breit (bei NMR)
- Bsp.: Beispiel
- *ca.*: *circa,* ungefähr
- CI: chemische Ionisation (bei MS)
- d: Dublett (bei NMR)
- d: Tag(e)
- DC: Dünnschichtchromatographie
- DCI: direkte chemische Ionisation (bei MS)
- dd: Dublett von Dublett (bei NMR)
- DMAP: 4-*N*,*N*-Dimethylaminopyridin
- DME: 1,2-Dimethoxyethan
- DMF: *N*,*N*-Dimethylformamid
- DMSO: Dimethylsulfoxid
- DPBS: Dulbecco's Phosphat-gepufferte Salz-Lösung
- dt: Dublett von Triplett (bei NMR)
- d. Th.: der Theorie (bei chemischer Ausbeute)
- EDC: *N'*-(3-Dimethylaminopropyl)-*N*-ethylcarbodiimid-Hydrochlorid
- EI: Elektronenstoß-Ionisation (bei MS)
- eq.: Äquivalent(e)
- ESI: Elektrospray-Ionisation (bei MS)
- FCS: fötales Kälberserum
- Fmoc: (9*H*-Fluoren-9-ylmethoxy)carbonyl
- ges.: gesättigt
- GTP: Guanosin-5'-triphosphat
- h: Stunde(n)
- HATU: *O-*(7-Azabenzotriazol-1-yl)-*N,N,N',N'*-tetramethyluronium-hexafluorophosphat
- HEPES: 4-(2-Hydroxyethyl)piperazin-1-ethansulfonsäure
- HOAc: Essigsäure
- HOBt: 1-Hydroxy-1*H*-benzotriazol-Hydrat
- HOSu: *N*-Hydroxysuccinimid
- HPLC: Hochdruck-, Hochleistungsflüssigchromatographie
- konz.: konzentriert
- LC-MS: Flüssigchromatographie-gekoppelte Massenspektrometrie
- m: Multiplett (bei NMR)
- min: Minute(n)
- MS: Massenspektrometrie
- MTT: 3-(4,5-Dimethylthiazol-2-yl)-2,5-diphenyl-2*H*-tetrazoliumbromid
- NMM: *N*-Methylmorpholin
- NMP: *N*-Methyl-2-pyrrolidinon
- NMR: Kermesonanzspektrometrie
- PBS: Phosphat-gepufferte Salz-Lösung
- Pd/C: Palladium auf Aktivkohle
- quant.: quantitativ (bei Ausbeute)
- quart: Quartett (bei NMR)
- quint: Quintett (bei NMR)
- R_{f}: Retentionsindex (bei DC)
- RT: Raumtemperatur
- Rₜ: Retentionszeit (bei HPLC)
- s: Singulett (bei NMR)
- t: Triplett (bei NMR)
- *tert.*: tertiär
- TFA: Trifluoressigsäure
- THF: Tetrahydrofuran
- UV: Ultraviolett-Spektrometrie
- v/v: Volumen zu Volumen-Verhältnis (einer Lösung)
- Z: Benzyloxycarbonyl
- zus.: zusammen

### HPLC- und LC-MS-Methoden:

### Methode 1 (LC-MS):

Instrument: Waters Acquity SQD UPLC System; Säule: Waters Acquity UPLC HSS T3 1.8µ 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 2 (LC-MS):

Instrument: Micromass QuattroPremier mit Waters UPLC Acquity; Säule: Thermo Hypersil GOLD 1.9µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 3 (LC-MS):

Instrument: Micromass Quattro Micro MS mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 4 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5 (HPLC):

Gerät: HP 1090 Serie II; Säule: Merck Chromolith SpeedROD RP-18e, 50 mm x 4.6 mm; Vorsäule: Merck Chromolith Guard Cartridge Kit RP-18e, 5 mm x 4.6 mm; Injektionsvolumen: 5 µl; Eluent A: 70% HClO₄ in Wasser (4 ml/Liter), Eluent B: Acetonitril; Gradient: 0.00 min 20% B → 0.50 min 20% B → 3.00 min 90% B → 3.50 min 90% B → 3.51 min 20% B → 4.00 min 20% B; Fluss: 5 ml/min; Säulentemperatur: 40°C.

### Methode 6 (HPLC):

Gerät: Waters 2695 mit DAD 996; Säule: Merck Chromolith SpeedROD RP-18e, 50 mm x 4.6 mm; Vorsäule: Merck Chromolith Guard Cartridge Kit RP-18e, 5 mm x 4.6 mm; Eluent A: 70% HClO₄ in Wasser (4 ml/Liter), Eluent B: Acetonitril; Gradient: 0.00 min 5% B → 0.50 min 5% B → 3.00 min 95% B → 4.00 min 95% B; Fluss: 5 ml/min.

### Methode 7 (LC-MS):

Gerätetyp MS: Waters ZQ; Gerätetyp HPLC: Agilent 1100 Series; UV DAD; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

### Methode 8 (LC-MS):

Gerätetyp MS: Waters ZQ; Gerätetyp HPLC: Agilent 1100 Series; UV DAD; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 2.0 min 60% A → 2.3 min 40% A → 3.0 min 20% A → 4.0 min 10% A → 4.2 min 100% A (Fluss 2.5 ml/min); Ofen: 55°C; Fluss: 2 ml/min; UV-Detektion: 210 nm.

### Methode 9 (LC-MS):

Instrument: Waters Acquity SQD UPLC System; Säule: Waters Acquity UPLC HSS T3 1.8µ 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 95% A → 6.0 min 5% A → 7.5 min 5% A; Ofen: 50°C; Fluss: 0.35 ml/min; UV-Detektion: 210-400 nm.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

### Ausgangsverbindungen und Intermediate:

### Ausgangsverbindung 1

### (2R,3R)-3-[(2S)-1-(tert.-Butoxycarbonyl)pyrrolidin-2-yl]-3-methoxy-2-methylpropansäure (Boc-Dolaproin)

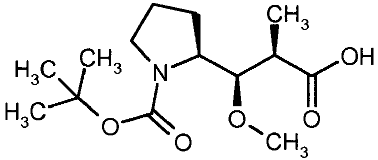

Die Titelverbindung kann auf verschiedenen Wegen nach Literaturvorschriften hergestellt werden, siehe z.B. Pettit et al., Synthesis 1996, 719; Shioiri et al., Tetrahedron Lett. 1991, 32, 931; Shioiri et al., Tetrahedron 1993, 49, 1913; Koga et al., Tetrahedron Lett. 1991, 32, 2395; Vidal et al., Tetrahedron 2004, 60, 9715; Poncet et al., Tetrahedron 1994, 50, 5345. Sie wurde hier entweder als freie Säure (wie abgebildet) oder in Form des entsprechenden Dicyclohexylamin-Salzes hergestellt.

### Ausgangsverbindung 2

### tert.-Butyl-(3R,4S,5S)-3-methoxy-5-methyl-4-(methylamino)heptanoat-Hydrochlorid (Dolaisoleucin-OtBu x HCl)

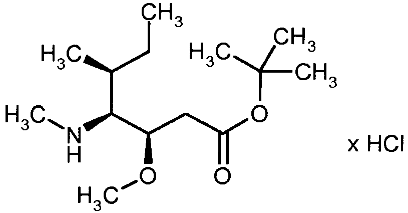

Die Titelverbindung kann auf verschiedenen Wegen nach Literaturvorschriften hergestellt werden, siehe z.B. Pettit et al., J. Org. Chem. 1994, 59, 1796; Koga et al., Tetrahedron Lett. 1991, 32, 2395; Shioiri et al., Tetrahedron Lett. 1991, 32, 931*;* Shioiri et al., Tetrahedron 1993, 49, 1913.

### Ausgangsverbindung 3

### N^{α}-(tert.-Butoxycarbonyl)-N-hydroxy-L-phenylalaninamid

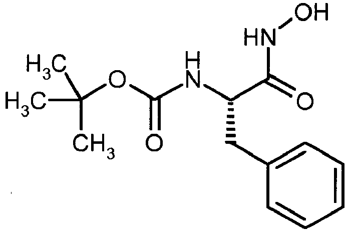

Die Titelverbindung wurde nach Literaturvorschrift hergestellt (A. Ritter et al., J. Org. Chem. 1994, 59, 4602).
Ausbeute: 750 mg (75% d. Th.)
LC-MS (Methode 3): Rₜ = 1.67 min; MS (ESIpos): m/z = 281 (M+H)⁺.

### Ausgangsverbindung 4

### 1,2-Oxazolidin-Hydrochlorid

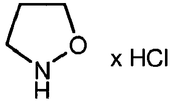

Die Titelverbindung kann nach Literaturvorschriften hergestellt werden, siehe z.B. H. King, J. Chem. Soc. 1942, 432; sie ist auch kommerziell erhältlich.

### Ausgangsverbindung 5

### 1,2-Oxazinan-Hydrochlorid

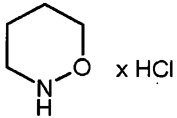

Die Titelverbindung kann nach Literaturvorschriften hergestellt werden, siehe z.B. H. King, J. Chem. Soc. 1942, 432.

### Ausgangsverbindung 6

### 2-Oxa-3-azabicyclo[2.2.2]oct-5-en

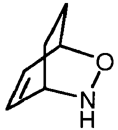

Die Titelverbindung kann in Boc-geschützter Form nach Literaturvorschrift hergestellt werden (siehe z.B. C. Johnson et al., Tetrahedron Lett. 1998, 39, 2059); die Entschützung erfolgte auf übliche Weise durch Behandlung mit Trifluoressigsäure und anschließende Neutralisation.

Ausbeute: 149 mg (89% d. Th.).

### Ausgangsverbindung 7

### 1,2-Oxazolidin-4-ol

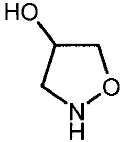

Die Titelverbindung kann nach Literaturvorschriften hergestellt werden, siehe z.B. N. Amlaiky, Synthesis 1982, 5, 426.

### Ausgangsverbindung 8

### tert.-Butyl-[(1S,2R)-1-(hydroxycarbamoyl)-2-phenylcyclopropyl]carbamat

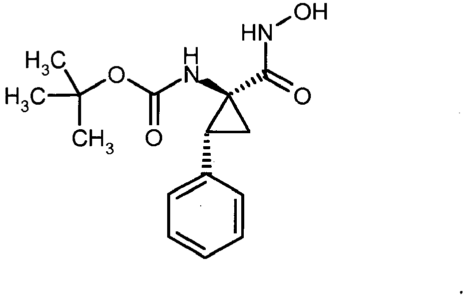

Die Titelverbindung wurde gemäß einer Literaturvorschrift (A. Ritter et al., J. Org. Chem. 1994, 59, 4602) hergestellt ausgehend von kommerziell erhältlicher (1*S*,2*R*)-1-[(*tert*.-Butoxycarbonyl)amino]-2-phenylcyclopropancarbonsäure (C. Cativiela et al., Chirality 1999, 11, 583).

Ausbeute: 339 mg (59% d. Th.)

LC-MS (Methode 1): Rₜ = 0.82 min; MS (ESIpos): m/z = 293 (M+H)⁺.

### Intermediat 1

### tert.-Butyl-(3R,4S,5S)-4-[{N-[(benzyloxy)carbonyl]-L-valyl}(methyl)amino]-3-methoxy-5-methyl-heptanoat

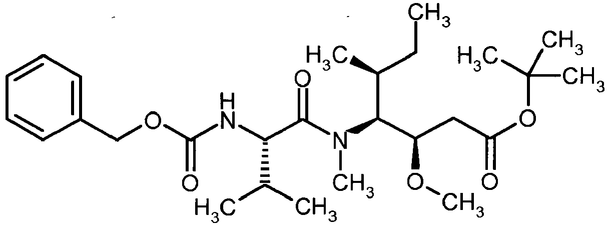

425 mg (1.7 mmol) *N*-[(Benzyloxy)carbonyl]-L-valin wurden in 50 ml DMF gelöst und nacheinander mit 500 mg (1.7 mmol) *tert*.-Butyl-(3*R*,4*S*,5*S*)-3-methoxy-5-methyl-4-(methylamino)-heptanoat-Hydrochlorid (Ausgangsverbindung 2), 356 mg (1.9 mmol) 1-(3-Dimethylaminopropyl)-3-ethylcarbodiimid-Hydrochlorid, 285 mg (1.9 mmol) 1-Hydroxy-1*H*-benzotriazol-Hydrat sowie 655 mg (5.1 mmol) *N*,*N*-Diisopropylethylamin versetzt. Die Mischung wurde 20 h bei RT gerührt. Anschließend wurden nochmals 142 mg (0.5 mmol) *N*-[(Benzyloxy)carbonyl]-L-valin, 119 mg (0.6 mmol) 1-(3-Dimethylaminopropyl)-3-ethylcarbodiimid-Hydrochlorid, 95 mg (0.6 mmol) 1-Hydroxy-1*H*-benzotriazol-Hydrat sowie 218 mg (1.7 mmol) *N*,*N*-Diisopropylethylamin zugesetzt und die Mischung 90 min lang mit Ultraschall behandelt. Der Ansatz wurde dann in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Der Rückstand wurde durch präparative HPLC gereinigt. Es wurden 329 mg (40% d. Th.) der Titelverbindung als farbloses Öl erhalten.

HPLC (Methode 5): Rₜ = 2.5 min;

LC-MS (Methode 1): Rₜ = 1.45 min; MS (ESIpos): m/z = 493 (M+H)⁺.

### Intermediat 2

### tert.-Butyl-(3R,4S,5S)-3-methoxy-5-methyl-4-[methyl(L-valyl)amino]heptanoat

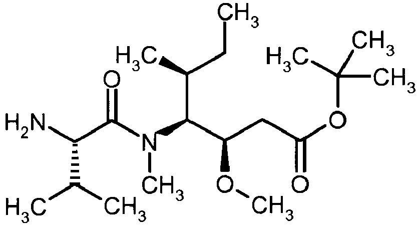

500 mg (1 mmol) *tert*.-Butyl-(3*R*,4*S*,5*S*)-4-[{*N*-[(benzyloxy)carbonyl]-L-valyl}(methyl)amino]-3-methoxy-5-methylheptanoat (Intermediat 1) wurden in 50 ml Methanol gelöst und nach Zugabe von 100 mg 10%-igem Palladium auf Aktivkohle 1 h lang bei RT unter Normaldruck hydriert. Der Katalysator wurde danach abfiltriert und das Lösungsmittel im Vakuum entfernt. Man erhielt 370 mg (quant.) der Titelverbindung als nahezu farbloses Öl.

HPLC (Methode 5): Rₜ = 1.59 min;

LC-MS (Methode 1): Rₜ = 0.74 min; MS (ESIpos): m/z = 359 (M+H)⁺.

### Intermediat 3

### N-[(9H-Fluoren-9-ylmethoxy)carbonyl]-N-methyl-L-valyl-N-[(3R,4S,5S)-1-tert.-butoxy-3-methoxy-5-methyl-1-oxoheptan-4-yl]-N-methyl-L-valinamid

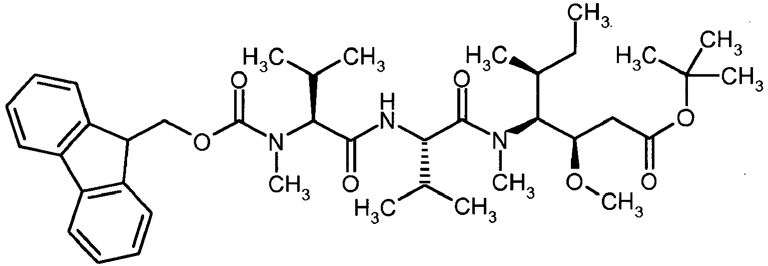

396 mg (1.1 mmol) *N*-[(9*H*-Fluoren-9-ylmethoxy)carbonyl]-*N*-methyl-L-valin wurden in 20 ml DMF gelöst und nacheinander mit 365 mg (1 mmol) *tert*.-Butyl-(3*R*,4*S*,5*S*)-3-methoxy-5-methyl-4-[methyl(L-valyl)amino]heptanoat (Intermediat 2), 234 mg (1.2 mmol) 1-(3-Dimethylaminopropyl)-3-ethylcarbodiimid-Hydrochlorid sowie 187 mg (1.2 mmol) 1-Hydroxy-1*H*-benzotriazol-Hydrat versetzt. Die Mischung wurde über Nacht bei RT gerührt. Der Ansatz wurde danach in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Der Rückstand wurde direkt, ohne weitere Reinigung, in der nächsten Stufe eingesetzt.

Ausbeute: 660 mg (68% d. Th.)

HPLC (Methode 5): Rₜ = 3.0 min;

LC-MS (Methode 1): Rₜ = 1.61 min; MS (ESIpos): m/z = 694 (M+H)⁺.

### Intermediat 4

### N-[(9H-Fluoren-9-ylmethoxy)carbonyl]-N-methyl-L-valyl-N-[(2R,3S,4S)-1-carboxy-2-methoxy-4-methylhexan-3-yl]-N-methyl-L-valinamid

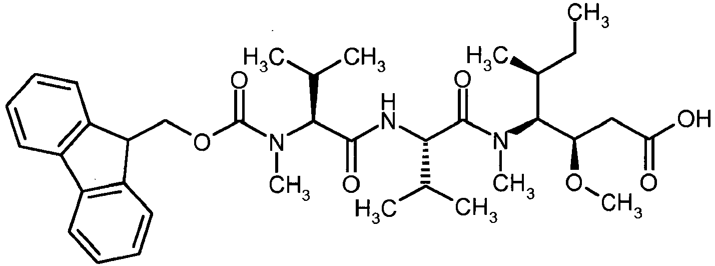

650 mg (0.94 mmol) *N*-[(9*H*-Fluoren-9-ylmethoxy)carbonyl]-*N*-methyl-L-valyl-*N*-[(3*R*,4*S*,5*S*)-1-*tert*.-butoxy-3-methoxy-5-methyl-1-oxoheptan-4-yl]-*N*-methyl-L-valinamid (Intermediat 3) wurden in 5 ml Dichlormethan aufgenommen, mit 5 ml Trifluoressigsäure versetzt und über Nacht bei RT gerührt. Anschließend wurde im Vakuum eingeengt und der verbliebene Rückstand durch präparative HPLC gereinigt. Es wurden 430 mg (72% d. Th.) der Titelverbindung als farbloser Schaum erhalten.

HPLC (Methode 5): Rₜ = 2.4 min;

LC-MS (Methode 2): Rₜ = 1.51 min; MS (ESIpos): m/z = 638 (M+H)⁺.

### Intermediat 5

### tert.-Butyl-[(2S)-1-(1,2-oxazinan-2-yl)-1-oxo-3-phenylpropan-2-yl]carbamat

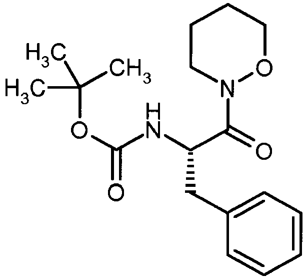

500 mg (1.9 mmol) *N*-(*tert*.-Butoxycarbonyl)-L-phenylalanin wurden in 10 ml DMF gelöst und nacheinander mit 466 mg (3.8 mmol) 1,2-Oxazinan-Hydrochlorid (Ausgangsverbindung 5), 433 mg (2.3 mmol) 1-(3-Dimethylaminopropyl)-3-ethylcarbodiimid-Hydrochlorid, 382 mg (2.8 mmol) 1-Hydroxy-1*H*-benzotriazol-Hydrat sowie 731 mg (5.7 mmol) *N*,*N*-Diisopropylethylamin versetzt. Die Mischung wurde über Nacht bei RT gerührt. Der Ansatz wurde danach in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Man erhielt 620 mg (98% d. Th.) der Titelverbindung.

HPLC (Methode 5): Rₜ = 1.8 min;

LC-MS (Methode 2): Rₜ = 1.62 min; MS (ESIpos): m/z = 235 (M-C₄H₈-CO₂+H)⁺.

### Intermediat 6

### (2S)-2-Amino-1-(1,2-oxazinan-2-yl)-3-phenylpropan-1-on Trifluoressigsäure-Salz

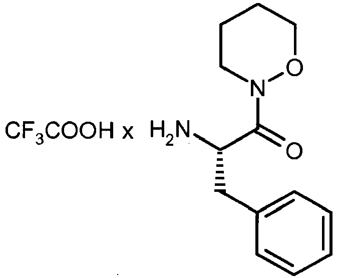

620 mg (1.85 mmol) *tert*.-Butyl-[(2*S*)-1-(1,2-oxazinan-2-yl)-1-oxo-3-phenylpropan-2-yl]carbamat (Intermediat 5) wurden in 5 ml Dichlormethan aufgenommen, mit 10 ml Trifluoressigsäure versetzt und 30 min bei RT gerührt. Anschließend wurde im Vakuum eingeengt und der verbliebene Rückstand aus Wasser/Acetonitril lyophilisiert. Auf diese Weise wurden 750 mg der Titelverbindung als farbloser Schaum erhalten.

HPLC (Methode 5): Rₜ = 0.45 min;

LC-MS (Methode 3): Rₜ = 1.09 min; MS (ESIpos): m/z = 235 (M+H)⁺.

### Intermediat 7

### (2R,3R)-3-Methoxy-2-methyl-N-[(2S)-1-(1,2-oxazinan-2-yl)-1-oxo-3-phenylpropan-2-yl]-3-[(2S)-pyrrolidin-2-yl]propanamid Trifluoressigsäure-Salz

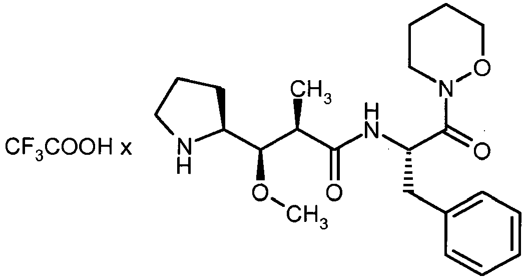

Zunächst wurde (2*R*,3*R*)-3-[(2*S*)-1-(*tert*.-Butoxycarbonyl)pyrrolidin-2-yl]-3-methoxy-2-methylpro-pansäure (Ausgangsverbindung 1) aus 50 mg (0.11 mmol) ihres Dicyclohexylamin-Salzes durch Aufnehmen in Ethylacetat und Ausschütteln mit wässriger Kaliumhydrogensulfat-Lösung freigesetzt. Die organische Phase wurde über Magnesiumsulfat getrocknet, filtriert und eingeengt. Der Rückstand wurde in 10 ml DMF aufgenommen und nacheinander mit 49 mg (0.11 mmol) (2*S*)-2-Amino-1-(1,2-oxazinan-2-yl)-3-phenylpropan-1-on Trifluoressigsäure-Salz (Intermediat 6), 61 mg (0.16 mmol) *O*-(7-Azabenzotriazol-1-yl)-*N*,*N*,*N'*,*N'*-tetramethyluronium-hexafluorophosphat (HATU) sowie 56 µl (0.16 mmol) *N*,*N*-Diisopropylethylamin versetzt. Die Mischung wurde 1 h bei RT gerührt. Der Ansatz wurde danach eingeengt und der Rückstand durch präparative HPLC gereinigt. Man erhielt so 44 mg (82% d. Th.) des Boc-geschützten Intermediats *tert*.-Butyl-(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-carboxylat.

HPLC (Methode 5): Rₜ = 1.9 min;

LC-MS (Methode 2): Rₜ = 1.27 min; MS (ESIpos): m/z = 504 (M+H)⁺.

44 mg (0.09 mmol) dieses Intermediats wurden in 3 ml Dichlormethan aufgenommen, mit 1 ml Trifluoressigsäure versetzt und 30 min bei RT gerührt. Anschließend wurde im Vakuum eingeengt und der verbliebene Rückstand aus Wasser/Acetonitril lyophilisiert. Auf diese Weise wurden 39 mg (86% d. Th.) der Titelverbindung als farbloser Schaum erhalten.

HPLC (Methode 5): Rₜ = 0.94 min;

LC-MS (Methode 1): Rₜ = 0.65 min; MS (ESIpos): m/z = 404 (M+H)⁺.

### Intermediat 8

### (2S)-2-Amino-1-(2-oxa-3-azabicyclo[2.2.2]oct-5-en-3-yl)-3-phenylpropan-1-on Trifluoressigsäure-Salz

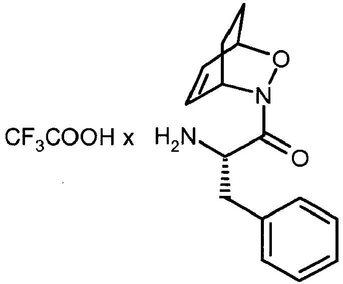

41 mg (0.37 mmol) 2,5-Dioxopyrrolidin-1-yl-*N*-(*tert*.-butoxycarbonyl)-L-phenylalaninat wurden in 10 ml DMF aufgenommen und mit 149 mg (0.41 mmol) 2-Oxa-3-azabicyclo[2.2.2]oct-5-en (Ausgangsverbindung 6) sowie 72 µl (0.41 mmol) *N*,*N*-Diisopropylethylamin versetzt. Die Mischung wurde 1 h bei RT gerührt. Das Lösungsmittel wurde danach im Vakuum entfernt, der Rückstand in Ethylacetat aufgenommen und mit 5%-iger Zitronensäure-Lösung und anschließend mit 5%-iger Natriumhydrogencarbonat-Lösung ausgeschüttelt. Die organische Phase wurde eingeengt und der Rückstand durch Flash-Chromatographie an Kieselgel mit Toluol/Ethanol 10:1 als Eluent gereinigt. Die entsprechenden Fraktionen wurden vereinigt und das Lösungsmittel im Vakuum entfernt. Nach Trocknen des Rückstands im Hochvakuum wurden so 69 mg (47% d. Th.) des Boc-geschützten Intermediats *tert*.-Butyl-[(2*S*)-1-(2-oxa-3-azabicyclo[2.2.2]oct-5-en-3-yl)-1-oxo-3-phenylpropan-2-yl]carbamat als Diastereomerengemisch erhalten.

LC-MS (Methode 1): Rₜ = 1.1 min; MS (ESIpos): m/z = 359 (M+H)⁺.

64 mg (0.18 mmol) dieses Intermediats wurden in 10 ml Dichlormethan aufgenommen, mit 1 ml Trifluoressigsäure versetzt und 30 min bei RT gerührt. Anschließend wurde im Vakuum eingeengt und der verbliebene Rückstand aus Wasser/Dioxan lyophilisiert. Auf diese Weise wurden 66 mg (quant.) der Titelverbindung als farbloser Schaum erhalten.

HPLC (Methode 6): Rₜ = 1.45 min;

LC-MS (Methode 3): Rₜ = 1.12 min; MS (ESIpos): m/z = 259 (M+H)⁺.

### Intermediat 9

### (2R,3R)-3-Methoxy-2-methyl-N-[(2S)-1-(2-oxa-3-azabicyclo[2.2.2]oct-5-en-3-yl)-1-oxo-3-phenyl-propan-2-yl]-3-[(2S)-pyrrolidin-2-yl]propanamid Trifluoressigsäure-Salz

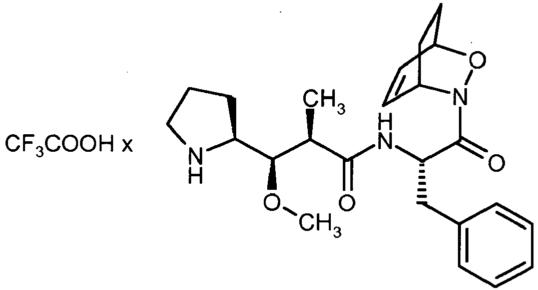

Zunächst wurde (2*R*,3*R*)-3-[(2*S*)-1-(*tert*.-Butoxycarbonyl)pyrrolidin-2-yl]-3-methoxy-2-methylpro-pansäure (Ausgangsverbindung 1) aus 83 mg (0.18 mmol) ihres Dicyclohexylamin-Salzes durch Aufnehmen in Ethylacetat und Ausschütteln mit wässriger Kaliumhydrogensulfat-Lösung freigesetzt. Die organische Phase wurde über Magnesiumsulfat getrocknet, filtriert und eingeengt. Der Rückstand wurde in 10 ml DMF aufgenommen und nacheinander mit 66 mg (0.18 mmol) (2*S*)-2-Amino-1-(2-oxa-3-azabicyclo[2.2.2]oct-5-en-3-yl)-3-phenylpropan-1-on Trifluoressigsäure-Salz (Intermediat 8), 101 mg (0.266 mmol) *O*-(7-Azabenzotriazol-1-yl)-*N*,*N*,*N',N'*-tetramethyluronium-hexafluorophosphat (HATU) sowie 93 µl (0.53 mmol) *N*,*N*-Diisopropylethylamin versetzt. Die Mischung wurde 30 min bei RT gerührt. Der Ansatz wurde danach eingeengt und der Rückstand durch präparative HPLC gereinigt. Man erhielt so 52 mg (56% d. Th.) des Boc-geschützten Intermediats *tert*.-Butyl-(2*S*)-2-[(1*R*,2*R*)-1-methoxy-2-methyl-3-{[(2*S*)-1-(2-oxa-3-azabicyclo[2.2.2]oct-5-en-3-yl)-1-oxo-3-phenylpropan-2-yl]amino}-3-oxopropyl]pyrrolidin-1-carboxylat.

HPLC (Methode 6): Rₜ = 2.13 min;

LC-MS (Methode 1): Rₜ = 1.13 min; MS (ESIpos): m/z = 528 (M+H)⁺.

52 mg (0.1 mmol) dieses Intermediats wurden in 10 ml Dichlormethan aufgenommen, mit 1 ml Trifluoressigsäure versetzt und 20 min bei RT gerührt. Anschließend wurde im Vakuum eingeengt und der verbliebene Rückstand mit 20 ml Diethylether verrührt. Nach 10 min wurde abfiltriert und der Filter-Rückstand im Hochvakuum getrocknet. Auf diese Weise wurden 39 mg (72% d. Th.) der Titelverbindung erhalten.

HPLC (Methode 6): Rₜ = 1.62 min;

LC-MS (Methode 1): Rₜ = 0.68 min; MS (ESIpos): m/z = 428 (M+H)⁺.

### Intermediat 10

### (2S)-2-Amino-1-(1,4-dihydro-3H-2,3-benzoxazin-3-yl)-3-phenylpropan-1-on Trifluoressigsäure-Salz

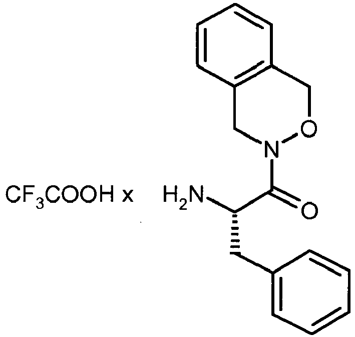

Zunächst wurde ausgehend von 75 mg (0.27 mmol) N^{α}-(*tert*.-Butoxycarbonyl)-*N*-hydroxy-L-phenyl-alaninamid (Ausgangsverbindung 3) durch Reaktion mit 1,2-Bis(brommethyl)benzol analog einer Literaturvorschrift (siehe H. King, J. Chem. Soc. 1942, 432) das Boc-geschützte Intermediat *tert.-*Butyl-[(2*S*)-1-(1,4-dihydro-3*H*-2,3-benzoxazin-3-yl)-1-oxo-3-phenylpropan-2-yl]carbamat hergestellt.

Ausbeute: 36 mg (35% d. Th.)

LC-MS (Methode 3): Rₜ = 2.49 min; MS (ESIpos): m/z = 383 (M+H)⁺.

36 mg (0.094 mmol) dieses Intermediats wurden in 5 ml Dichlormethan aufgenommen, mit 0.5 ml Trifluoressigsäure versetzt und 20 min bei RT gerührt. Anschließend wurde im Vakuum eingeengt und der verbliebene Rückstand durch präparative HPLC gereinigt. Die entsprechenden Fraktionen wurden vereinigt, im Vakuum auf ein Volumen von ca. 15 ml aufkonzentriert und anschließend lyophilisiert. Es wurden 16 mg (43% d. Th.) der Titelverbindung als farbloser Schaum erhalten.

HPLC (Methode 6): Rₜ = 1.7 min;

LC-MS (Methode 1): Rₜ = 0.69 min; MS (ESIpos): m/z = 283 (M+H)⁺.

### Intermediat 11

### (2R,3R)-N-[(2S)-1-(1,4-Dihydro-3H-2,3-benzoxazin-3-yl)-1-oxo-3-phenylpropan-2-yl]-3-methoxy-2-methyl-3-[(2S)-pyrrolidin-2-yl]propanamid Trifluoressigsäure-Salz

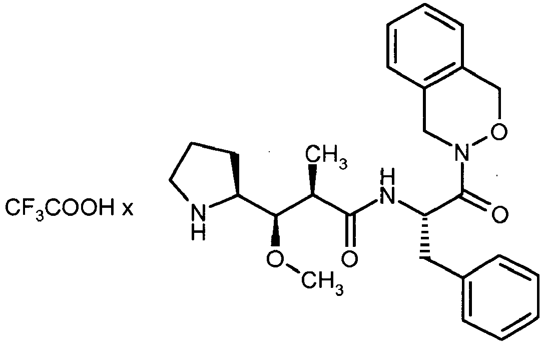

Zunächst wurde (2*R*,3*R*)-3-[(2*S*)-1-(*tert*.-Butoxycarbonyl)pyrrolidin-2-yl]-3-methoxy-2-methylpropansäure (Ausgangsverbindung 1) aus 15 mg (0.031 mmol) ihres Dicyclohexylamin-Salzes durch Aufnehmen in Ethylacetat und Ausschütteln mit wässriger Kaliumhydrogensulfat-Lösung freigesetzt. Die organische Phase wurde über Magnesiumsulfat getrocknet, filtriert und eingeengt. Der Rückstand wurde in 10 ml DMF aufgenommen und nacheinander mit 16 mg (0.031 mmol) (2*S*)-2-Amino-1-(1,4-dihydro-3*H*-2,3-benzoxazin-3-yl)-3-phenylpropan-1-on Trifluoressigsäure-Salz (Intermediat 10), 18 mg (0.047 mmol) *O*-(7-Azabenzotriazol-1-yl)-*N*,*N*,*N'*,*N'*-tetramethyluronium-hexa-fluorophosphat (HATU) sowie 16 µl (0.53 mmol) *N*,*N*-Diisopropylethylamin versetzt. Die Mischung wurde 16 h bei RT gerührt. Der Ansatz wurde danach eingeengt und der Rückstand durch präparative HPLC gereinigt. Man erhielt so 14 mg (81% d. Th.) des Boc-geschützten Intermediats *tert*.-Butyl-(2*S*)-2-[(1*R*,2*R*)-3-{[(2*S*)-1-(1,4-dihydro-3*H*-2,3-benzoxazin-3-yl)-1-oxo-3-phenylpropan-2-yl]amino}-1-methoxy-2-methyl-3-oxopropyl]pyrrolidin-1-carboxylat.

HPLC (Methode 6): Rₜ = 2.35 min;

LC-MS (Methode 2): Rₜ = 1.39 min; MS (ESIpos): m/z = 552 (M+H)⁺.

14 mg (0.025 mmol) dieses Intermediats wurden in 5 ml Dichlormethan aufgenommen, mit 1 ml Trifluoressigsäure versetzt und 20 min bei RT gerührt. Anschließend wurde im Vakuum eingeengt und der verbliebene Rückstand aus Dioxan lyophilisiert. Auf diese Weise wurden 14 mg (98% d. Th.) der Titelverbindung erhalten.

HPLC (Methode 6): Rₜ = 1.83 min;

LC-MS (Methode 1): Rₜ = 0.78 min; MS (ESIpos): m/z = 452 (M+H)⁺.

### Intermediat 12

### (2S)-2-Amino-3-(1H-indol-3-yl)-1-(1,2-oxazinan-2-yl)propan-1-on Trifluoressigsäure-Salz

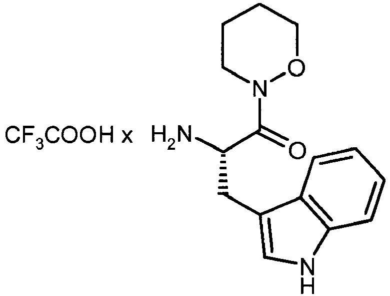

130 mg (0.324 mmol) 2,5-Dioxopyrrolidin-1-yl-*N*-(*tert*.-butoxycarbonyl)-L-tryptophanat wurden in 15 ml DMF aufgenommen und mit 50 mg (0.405 mmol) 1,2-Oxazinan-Hydrochlorid (Ausgangsverbindung 5) sowie 140 µl *N*,*N*-Diisopropylethylamin versetzt. Die Mischung wurde 24 h bei RT gerührt. Danach wurde nochmals die gleiche Menge an 1,2-Oxazinan-Hydrochlorid und *N*,*N*-Diisopropylethylamin zugesetzt. Nach weiteren 4 h Rühren bei RT wurde der Ansatz im Vakuum eingeengt, der Rückstand in Dichlormethan aufgenommen und zunächst mit gesättigter Natriumhydrogencarbonat-Lösung, dann mit Wasser ausgeschüttelt. Die organische Phase wurde eingeengt und der Rückstand durch Flash-Chromatographie an Kieselgel mit Dichlormethan/Ethylacetat 4:1 als Eluent gereinigt. Die entsprechenden Fraktionen wurden eingeengt und der Rückstand im Hochvakuum getrocknet. Man erhielt so 63 mg (52% d. Th.) des Boc-geschützten Intermediats *tert.-*Butyl-[(2*S*)-3-(1*H*-indol-3-yl)-1-(1,2-oxazinan-2-yl)-1-oxopropan-2-yl]carbamat.

HPLC (Methode 6): Rₜ = 2.05 min;

LC-MS (Methode 3): Rₜ = 2.16 min; MS (ESIpos): m/z = 374 (M+H)⁺.

63 mg (0.167 mmol) dieses Intermediats wurden in 15 ml Dichlormethan aufgenommen, mit 2 ml Trifluoressigsäure versetzt und 40 min im Ultraschallbad behandelt. Der Ansatz wurde danach im Vakuum eingeengt und der Rückstand aus Dioxan/Wasser lyophilisiert. Es wurden auf diese Weise 65 mg (90% d. Th.) der Titelverbindung erhalten.

HPLC (Methode 6): Rₜ = 1.53 min;

LC-MS (Methode 1): Rₜ = 0.61 min; MS (ESIpos): m/z = 274 (M+H)⁺.

### Intermediat 13

### (2R,3R)-N-[(2S)-3-(1H-Indol-3-yl)-1-(1,2-oxozinan-2-yl)-1-oxopropan-2-yl]-3-methoxy-2-methyl-3-[(2S)-pyrrolidin-2-yl]propanamid Trifluoressigsäure-Salz

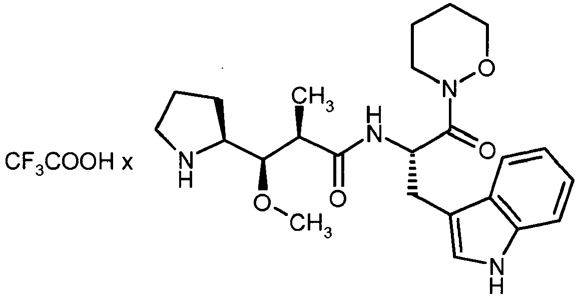

Die Titelverbindung wurde in Analogie zur Synthese von Intermediat 7 über zwei Stufen aus dem Dicyclohexylamin-Salz der Ausgangsverbindung 1 und (2*S*)-2-Amino-3-(1*H*-indol-3-yl)-1-(1,2-oxazinan-2-yl)propan-1-on Trifluoressigsäure-Salz (Intermediat 12) hergestellt.

Ausbeute über 2 Stufen: 62 mg (67% d. Th.)

HPLC (Methode 6): Rₜ = 1.65 min;

LC-MS (Methode 1): Rₜ = 0.7 min; MS (ESIpos): m/z = 443 (M+H)⁺.

### Intermediat 14

### (2S)-2-Amino-1-(1,2-oxazolidin-2-yl)-3-phenylpropan-1-on Trifluoressigsäure-Salz

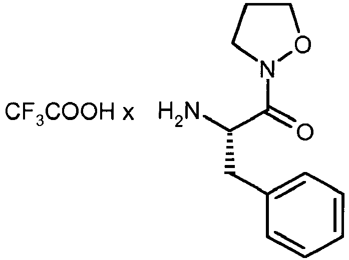

Die Titelverbindung wurde in Analogie zur Synthese der Intermediate 5 und 6 über zwei Stufen aus *N*-(*tert*.-Butoxycarbonyl)-L-phenylalanin und 1,2-Oxazolidin-Hydrochlorid (Ausgangsverbindung 4) hergestellt.

Ausbeute über 2 Stufen: 1650 mg (97% d. Th.)

HPLC (Methode 5): Rₜ = 0.29 min;

LC-MS (Methode 1): Rₜ = 0.41 min; MS (ESIpos): m/z = 221 (M+H)⁺.

### Intermediat 15

### (2R,3R)-3-Methoxy-2-methyl-N-[(2S)-1-(1,2-oxazolidin-2-yl)-1-oxo-3-phenylpropan-2-yl]-3-[(2S)-pyrrolidin-2-yl]propanamid Trifluoressigsäure-Salz

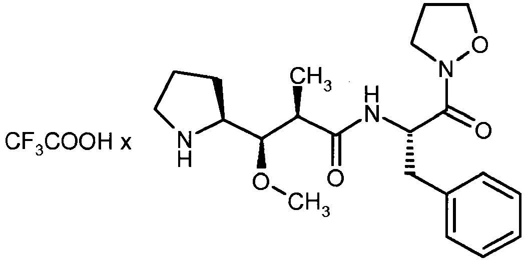

Die Titelverbindung wurde in Analogie zur Synthese von Intermediat 7 über zwei Stufen aus dem Dicyclohexylamin-Salz von Ausgangsverbindung 1 und (2*S*)-2-Anüno-1-(1,2-oxazolidin-2-yl)-3-phenylpropan-1-on Trifluoressigsäure-Salz (Intermediat 14) hergestellt.

Ausbeute über 2 Stufen: 110 mg (97% d. Th.)

HPLC (Methode 5): Rₜ = 0.52 min;

LC-MS (Methode 3): Rₜ = 1.15 min; MS (ESIpos): m/z = 390 (M+H)⁺.

### Intermediat 16

### (2S,3S)-2-Amino-1-(1,2-oxazinan-2-yl)-3-phenylbutan-1-on Trifluoressigsäure-Salz

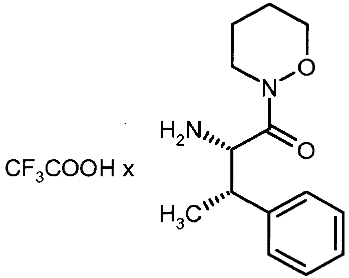

Die Titelverbindung wurde in Analogie zur Synthese der Intermediate 5 und 6 über zwei Stufen aus (β*S*)-*N*-(*tert*.-Butoxycarbonyl)-β-methyl-L-phenylalanin und 1,2-Oxazinan-Hydrochlorid (Ausgangsverbindung 5) hergestellt.

Ausbeute über 2 Stufen: 652 mg (63% d. Th.)

HPLC (Methode 5): Rₜ = 0.53 min;

LC-MS (Methode 1): Rₜ = 1.25 min; MS (ESIpos): m/z = 249 (M+H)⁺.

### Intermediat 17

### (2R,3R)-3-Methoxy-2-methyl-N-[(2S,3S)-1-(1,2-oxazinan-2-yl)-1-oxo-3-phenylbutan-2-yl]-3-[(2S)-pyrrolidin-2-yl]propanamid Trifluoressigsäure-Salz

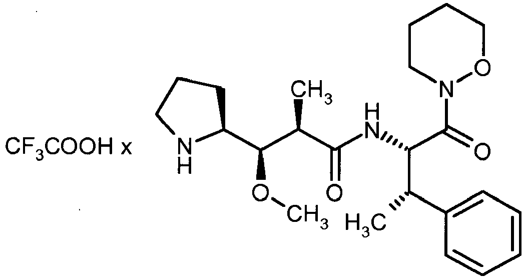

Die Titelverbindung wurde in Analogie zur Synthese von Intermediat 7 über zwei Stufen aus dem Dicyclohexylamin-Salz von Ausgangsverbindung 1 und (2*S*,3*S*)-2-Amino-1-(1,2-oxazinan-2-yl)-3-phenylbutan-1-on Trifluoressigsäure-Salz (Intermediat 16) hergestellt.

Ausbeute über 2 Stufen: 101 mg (90% d. Th.)

HPLC (Methode 5): Rₜ = 1.07 min;

LC-MS (Methode 3): Rₜ = 1.33 min; MS (ESIpos): m/z = 418 (M+H)⁺.

### Intermediat 18

### (2S)-2-Amino-3-(4-hydroxy-3-nitrophenyl)-1-(1,2-oxazinan-2-yl)propan-1-on Trifluoressigsäure-Salz

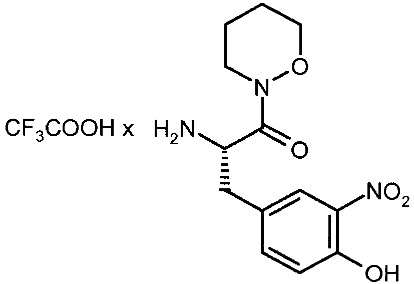

Die Titelverbindung wurde in Analogie zur Synthese der Intermediate 5 und 6 über zwei Stufen aus *N*-(*tert*.-Butoxycarbonyl)-3-nitro-L-tyrosin und 1,2-Oxazinan-Hydrochlorid (Ausgangsverbindung 5) hergestellt.

Ausbeute über 2 Stufen: 374 mg (47% d. Th.)

HPLC (Methode 5): Rₜ = 0.4 min;

LC-MS (Methode 1): Rₜ = 0.5 min; MS (ESIpos): m/z = 296 (M+H)⁺.

### Intermediat 19

### (2R,3R)-N-[(2S)-3-(4-Hydroxy-3-nitrophenyl)-1-(1,2-oxazinan-2-yl)-1-oxopropan-2-yl]-3-methoxy-2-methyl-3-[(2S)-pyrrolidin-2-yl]propanamid Trifluoressigsäure-Salz

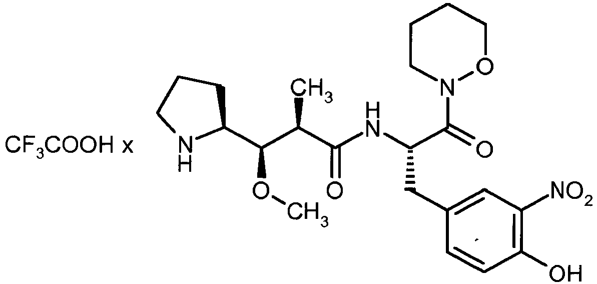

Die Titelverbindung wurde in Analogie zur Synthese von Intermediat 7 über zwei Stufen aus dem Dicyclohexylamin-Salz von Ausgangsverbindung 1 und (2*S*)-2-Amino-3-(4-hydroxy-3-nitrophenyl)-1-(1,2-oxazinan-2-yl)propan-1-on Trifluoressigsäure-Salz (Intermediat 18) hergestellt. Das in einer Reinheit von 63% erhaltene Produkt wurde als solches, ohne weitere Aufreinigung, in Folgereaktionen eingesetzt.

Ausbeute über 2 Stufen: 128 mg (61% d. Th.)

HPLC (Methode 5): Rₜ = 0.8 min;

LC-MS (Methode 3): Rₜ = 1.24 min; MS (ESIpos): m/z = 465 (M+H)⁺.

### Intermediat 20

### N-(tert.-Butoxycarbonyl)-N-methyl-L-valyl-N-[(2R,3S,4S)-1-carboxy-2-methoxy-4-methylhexan-3-yl]-N-methyl-L-valinamid

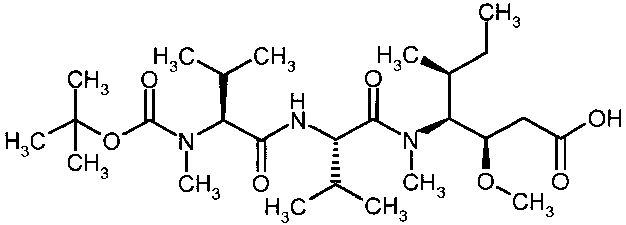

51 mg (0.08 mmol) *N*-[(9*H*-Fluoren-9-ylmethoxy)carbonyl]-*N*-methyl-L-valyl-*N*-[(2*R*,3*S*,4*S*)-1-carb-oxy-2-methoxy-4-methylhexan-3-yl]-*N*-methyl-L-valinamid (Intermediat 4) wurden in 10 ml DMF gelöst und mit 0.5 ml Piperidin versetzt. Nach 10 min Rühren bei RT wurde der Ansatz im Vakuum eingeengt und der Rückstand mit Diethylether verrührt. Die unlöslichen Bestandteile wurden abfiltriert und mehrfach mit Diethylether gewaschen. Dann wurde der Filter-Rückstand in 5 ml Dioxan/Wasser (1:1) aufgenommen und die Lösung mit 1 N Natronlauge auf pH 11 eingestellt. Unter Ultraschallbehandlung wurden in mehreren Portionen insgesamt 349 mg (1.6 mmol) Di-*tert*.-butyldicarbonat zugegeben, wobei der pH-Wert der Lösung bei 11 gehalten wurde. Nach Beendigung der Reaktion wurde das Dioxan abgedampft und die wässrige Lösung mit Zitronensäure auf einen pH-Wert von 2-3 eingestellt. Man extrahierte zweimal mit je 50 ml Ethylacetat. Die organischen Phasen wurden vereinigt, über Magnesiumsulfat getrocknet und im Vakuum eingeengt. Der Rückstand wurde in Diethylether aufgenommen und das Produkt mit Pentan ausgefällt. Durch Dekantieren wurde das Lösungsmittel abgetrennt. Der Rückstand wurde noch mehrmals mit Pentan digeriert und schließlich im Hochvakuum getrocknet. Es wurden so 31 mg (93% d. Th.) der Titelverbindung erhalten.

HPLC (Methode 6): Rₜ = 2.2 min;

LC-MS (Methode 2): Rₜ = 1.32 min; MS (ESIpos): m/z = 516 (M+H)⁺.

### Intermediat 21

### tert.-Butyl-(2S)-2-[(1R,2R)-3-{[(2S)-3,3-dimethyl-1-(1,2-oxazinan-2-yl)-1-oxobutan-2-yl]amino}-1-methoxy-2-methyl-3-oxopropyl]pyrrolidin- 1 -carboxylat

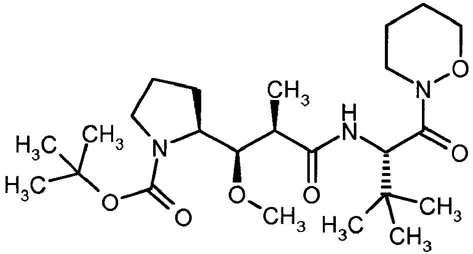

Die Titelverbindung wurde in Analogie zur Synthese der Intermediate 5 und 6 über drei Stufen durch Kupplung von kommerziell erhältlichem *N*-(*tert*.-Butoxycarbonyl)-3-methyl-L-valin mit 1,2-Oxazinan-Hydrochlorid (Ausgangsverbindung 5), anschließende Entschützung mit Trifluoressigsäure und erneute Kupplung mit Ausgangsverbindung 1 hergestellt. Das Endprodukt wurde durch präparative HPLC gereinigt.

HPLC (Methode 5): Rₜ = 1.92 min;

LC-MS (Methode 7): Rₜ = 2.19 min; MS (ESIpos): m/z = 470 (M+H)⁺.

### Intermediat 22

### tert.-Butyl-(2S)-2-[(1R,2R)-1-methoxy-2-methyl-3-{[(2S)-1-(1,2-oxazinan-2-yl)-1-oxo-3,3-diphenylpropan-2-yl]amino}-3-oxopropyl]pyrrolidin-1-carboxylat

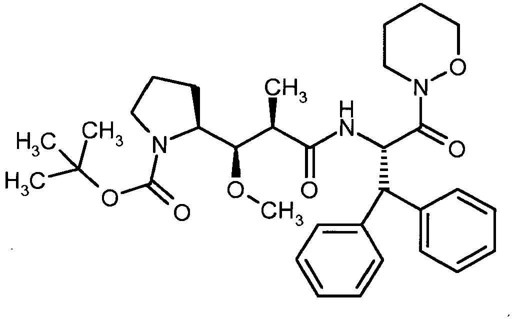

Die Titelverbindung wurde in Analogie zur Synthese der Intermediate 5 und 6 über drei Stufen durch Kupplung von kommerziell erhältlichem *N*-(*tert*.-Butoxycarbonyl)-β-phenyl-L-phenylalanin mit 1,2-Oxazinan-Hydrochlorid (Ausgangsverbindung 5), anschließende Entschützung mit Trifluoressigsäure und erneute Kupplung mit Ausgangsverbindung 1 hergestellt. Das Endprodukt wurde durch präparative HPLC gereinigt.

HPLC (Methode 5): Rₜ = 2.07 min;

LC-MS (Methode 1): Rₜ = 1.22 min; MS (ESIpos): m/z = 580 (M+H)⁺.

### Intermediat 23

### tert.-Butyl-(2S)-2-[(1R,2R)-1-methoxy-2-methyl-3-{[(1S,2S)-1-(1,2-oxazinan-2-ylcarbonyl)-2-phenylcyclopropyl]amino}-3-oxopropyl]pyrrolidin-1-carboxylat

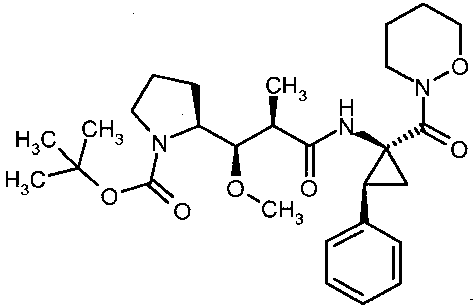

Die Titelverbindung wurde in Analogie zur Synthese der Intermediate 5 und 6 über drei Stufen durch Kupplung von kommerziell erhältlicher (1*S*,2*S*)-1-[(*tert*.-Butoxycarbonyl)amino]-2-phenyl-cyclopropancarbonsäure mit 1,2-Oxazinan-Hydrochlorid (Ausgangsverbindung 5), anschließende Entschützung mit Trifluoressigsäure und erneute Kupplung mit Ausgangsverbindung 1 hergestellt. Das Endprodukt wurde durch präparative HPLC gereinigt.

HPLC (Methode 5): Rₜ = 2.19 min;

LC-MS (Methode 3): Rₜ = 2.28 min; MS (ESIpos): m/z = 516 (M+H)⁺.

### Intermediat 24

### tert.-Butyl-(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]pyrrolidin-1-carboxylat

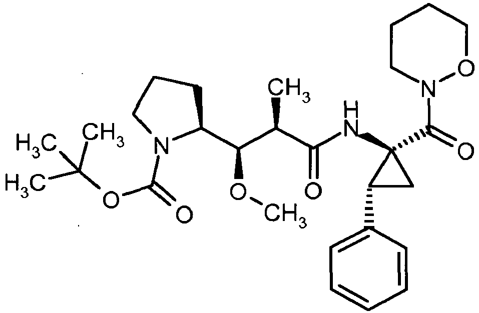

Die Titelverbindung wurde in Analogie zur Synthese der Intermediate 5 und 6 über drei Stufen durch Kupplung von kommerziell erhältlicher (1*S*,2*R*)-1-[(*tert*.-Butoxycarbonyl)amino]-2-phenyl-cyclopropancarbonsäure mit 1,2-Oxazinan-Hydrochlorid (Ausgangsverbindung 5), anschließende Entschützung mit Trifluoressigsäure und erneute Kupplung mit Ausgangsverbindung 1 hergestellt. Das Endprodukt wurde durch präparative HPLC gereinigt.

HPLC (Methode 5): Rₜ = 2.12 min;

LC-MS (Methode 2): Rₜ = 1.25 min; MS (ESIpos): m/z = 516 (M+H)⁺.

### Intermediat 25

### tert.-Butyl-[(2S)-1-(4-hydroxy-1,2-oxazolidin-2-yl)-1-oxo-3-phenylpropan-2-yl]carbamat (Diastereomer 1 und 2)

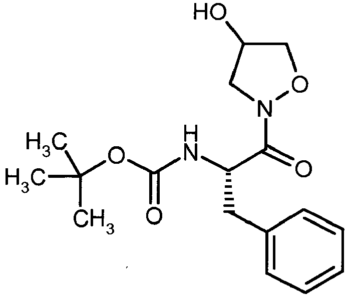

200 mg (1.59 mmol) 1,2-Oxazolidin-4-ol (Ausgangsverbindung 7) wurden in 10 ml DMF aufgenommen und mit 606 mg (1.67 mmol) 2,5-Dioxopyrrolidin-1-yl-*N*-(*tert*.-butoxycarbonyl)-L-phenylalaninat sowie 277 µl *N,N*-Diisopropylethylamin versetzt. Nach 60 h Rühren bei RT wurde der Ansatz im Vakuum eingeengt und der Rückstand in 100 ml Ethylacetat aufgenommen. Man schüttelte jeweils zweimal mit 5%-iger Zitronensäure-Lösung und 10%-iger Natriumhydrogencarbonat-Lösung aus. Anschließend wurde die organische Phase im Vakuum eingeengt. Die weitere Reinigung und Auftrennung der beiden Diastereomere erfolgte durch Flash-Chromatographie an Kieselgel mit Dichlormethan/Methanol 98:2 als Eluent. Die entsprechenden Fraktionen wurden eingeengt und der Rückstand im Hochvakuum getrocknet.

### Diastereomer 1:

Ausbeute: 154 mg [noch durch N-(*tert*.-Butoxycarbonyl)-L-phenylalanin verunreinigt, welches auf der Folgestufe abgetrennt wurde]

R_{f} = 0.44 (Dichlormethan/Methanol 95:5).

### Diastereomer 2:

Ausbeute: 86 mg (16% d. Th.)

R_{f} = 0.40 (Dichlormethan/Methanol 95:5).

### Intermediat 26

### tert.-Butyl- {(2S)-1-[4-(benzyloxy)-1,2-oxazolidin-2-yl]-1-oxo-3-phenylpropan-2-yl}carbamat (Diastereomer 1)

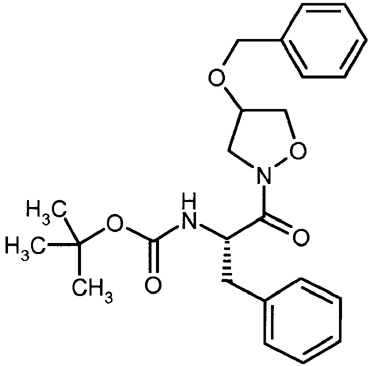

75 mg (ca. 0.22 mmol) des Diastereomers 1 von Intermediat 25 wurden in 10 ml Aceton aufgenommen und mit 228 mg (1.34 mmol) Benzylbromid, 616 mg (4.46 mmol) Kaliumcarbonat sowie einer Spatelspitze Tetra-n-butylammoniumiodid versetzt. Der Ansatz wurde 20 h unter Rückfluss erhitzt und dann im Vakuum eingeengt. Der Rückstand wurde mit 100 ml Dichlormethan verrührt, filtriert und die Dichlormethan-Phase anschließend eingeengt. Der verbliebene Rückstand wurde durch präparative HPLC gereinigt. Es wurden 68 mg (72% d. Th.) der Titelverbindung erhalten.

HPLC (Methode 6): Rₜ = 2.31 min;

LC-MS (Methode 1): Rₜ = 1.18 min; MS (ESIpos): m/z = 427 (M+H)⁺.

### Intermediat 27

### tert.-Butyl- {(2S)-1-[4-(benzyloxy)-1,2-oxazolidin-2-yl]-1-oxo-3-phenylpropan-2-yl} carbamat (Diastereomere 2)

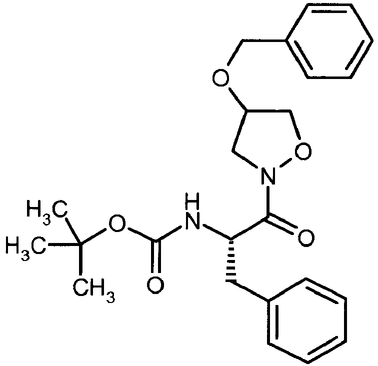

83 mg (0.25 mmol) des Diastereomers 2 von Intermediat 25 wurden in 15 ml Aceton aufgenommen und mit 127 mg (0.74 mmol) Benzylbromid, 682 mg (4.93 mmol) Kaliumcarbonat sowie einer Spatelspitze Tetra-*n*-butylammoniumiodid versetzt. Der Ansatz wurde 20 h unter Rückfluss erhitzt und dann im Vakuum eingeengt. Der Rückstand wurde mit 100 ml Dichlormethan verrührt, filtriert und die Dichlormethan-Phase anschließend eingeengt. Der verbliebene Rückstand wurde durch präparative HPLC gereinigt. Es wurden 87 mg (83% d. Th.) der Titelverbindung erhalten.

HPLC (Methode 6): Rₜ = 2.30 min;

LC-MS (Methode 1): Rₜ = 1.22 min; MS (ESIpos): m/z = 427 (M+H)⁺.

### Intermediat 28

### (2S)-2-Amino-1-[4-(benzyloxy)-1,2-oxazolidin-2-yl]-3-phenylpropan-1-on Trifluoressigsäure-Salz (Diastereomer 1)

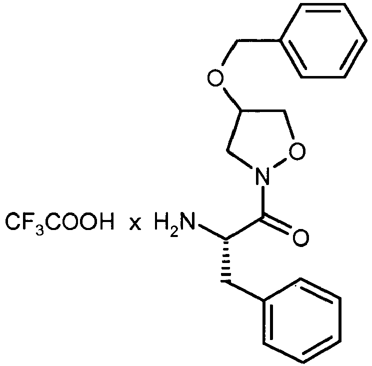

68 mg (0.16 mmol) *tert*.-Butyl-{(2*S*)-1-[4-(benzyloxy)-1,2-oxazolidin-2-yl]-1-oxo-3-phenylpropan-2-yl}carbamat (Diastereomer 1, Intermediat 26) wurden in 10 ml Dichlormethan aufgenommen, mit 1 ml Trifluoressigsäure versetzt und 30 min bei RT gerührt. Anschließend wurde im Vakuum eingeengt und der verbliebene Rückstand aus Dioxan/Wasser lyophilisiert. Es wurden 69 mg (98% d. Th.) der Titelverbindung als farbloser Schaum erhalten.

HPLC (Methode 6): Rₜ = 1.72 min;

LC-MS (Methode 3): Rₜ = 1.41 min; MS (ESIpos): m/z = 327 (M+H)⁺.

### Intermediat 29

### (2S)-2-Amino-1-[4-(benzyloxy)-1,2-oxazolidin-2-yl]-3-phenylpropan-1-on Trifluoressigsäure-Salz (Diastereomer 2)

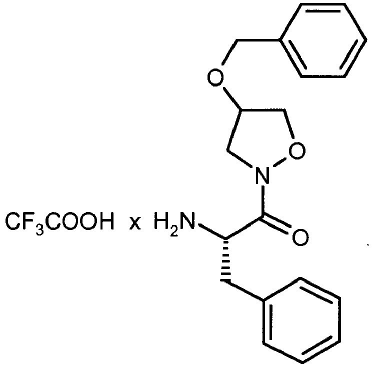

82 mg (0.19 mmol) *tert*.-Butyl-{(2*S*)-1-[4-(benzyloxy)-1,2-oxazolidin-2-yl]-1-oxo-3-phenylpropan-2-yl}carbamat (Diastereomer 2, Intermediat 27) wurden in 10 ml Dichlormethan aufgenommen, mit 1 ml Trifluoressigsäure versetzt und 30 min bei RT gerührt. Anschließend wurde im Vakuum eingeengt und der verbliebene Rückstand aus Dioxan/Wasser lyophilisiert. Es wurden 80 mg (95% d. Th.) der Titelverbindung als farbloser Schaum erhalten.

HPLC (Methode 6): Rₜ = 1.80 min;

LC-MS (Methode 3): Rₜ = 1.50 min; MS (ESIpos): m/z = 327 (M+H)⁺.

### Intermediat 30

### N-[(9H-Fluoren-9-ylmethoxy)carbonyl]-N-methyl-L-valyl-N-[(3R,4S,5S)-1-{(2S)-2-[(1R,2R)-2-carboxy-1-methoxypropyl]pyrrolidin-1-yl}-3-methoxy-5-methyl-1-oxoheptan-4-yl]-N-methyl-L-valinamid

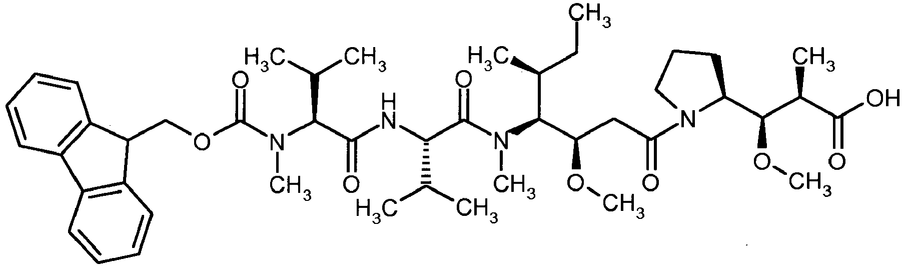

315 mg (0.494 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 12 ml DMF gelöst, mit 104 mg (0.543 mmol) 1-(3-Dimethylaminopropyl)-3-ethylcarbodimid-Hydro-chlorid sowie 83 mg (0.543 mmol) 1-Hydroxy-1*H*-benzotriazol-Hydrat versetzt und 90 min bei RT gerührt. Anschließend wurden 112 µl *N*,*N*-Diisopropylethylamin sowie 149 mg (0.494 mmol) (2*R*,3*R*)-3-Methoxy-2-methyl-3-[(2*S*)-pyrrolidin-2-yl]propansäure Trifluoressigsäure-Salz, welches zuvor aus der Ausgangsverbindung 1 durch Abspaltung der Boc-Schutzgruppe mittels Trifluoressigsäure hergestellt worden war, hinzugegeben. Die Mischung wurde 2 h bei RT gerührt und anschließend im Hochvakuum eingeengt. Der verbliebene Rückstand wurde durch zweimalige präparative HPLC aufgereinigt. Es wurden 140 mg (35% d. Th.) der Titelverbindung in Form eines farblosen Schaumes erhalten.

HPLC (Methode 5): Rₜ = 2.40 min;

LC-MS (Methode 1): Rₜ = 1.38 min; MS (ESIpos): m/z = 807 (M+H)⁺.

### Intermediat 31

### [(1S,2R)-1-Amino-2-phenylcyclopropyl](1,4-dihydro-3H-2,3-benzoxazin-3-yl)methanon Trifluoressigsäure-Salz

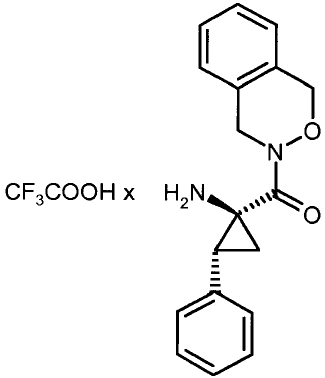

Die Titelverbindung wurde in Analogie zur Synthese von Intermediat 10 ausgehend von *tert*.-Butyl-[(1*S*,2*R*)-1-(hydroxycarbamoyl)-2-phenylcyclopropyl]carbamat (Ausgangsverbindung 8) hergestellt.

HPLC (Methode 6): Rₜ = 1.60 min;

LC-MS (Methode 1): Rₜ = 0.70 min; MS (ESIpos): m/z = 295 (M+H)⁺.

### Intermediat 32

### N-[(Benzyloxy)carbonyl]-N-methyl-L-threonyl-N-[(2R,3S,4S)-1-carboxy-2-methoxy-4-methylhexan-3-yl]-N-methyl-L-valinamid

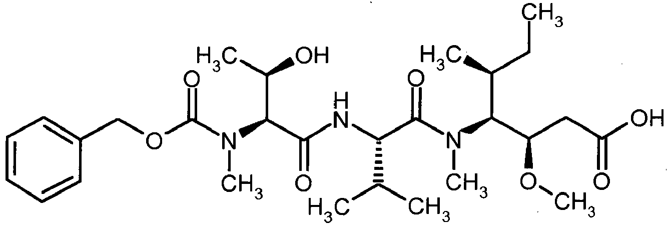

Zunächst wurde *N*-[(Benzyloxy)carbonyl]-*N*-methyl-L-threonin aus 237 mg (0.887 mmol) seines Dicyclohexylamin-Salzes durch Aufnehmen in Ethylacetat und Ausschütteln mit 5%-iger wässriger Schwefelsäure freigesetzt. Die organische Phase wurde über Magnesiumsulfat getrocknet, filtriert und eingeengt. Der Rückstand wurde in 16 ml DMF aufgenommen und nacheinander mit 365 mg (1 mmol) *tert*.-Butyl-(3*R*,4*S*,5*S*)-3-methoxy-5-methyl-4-[methyl(L-valyl)amino]heptanoat (Intermediat 2), 185 mg (0.967 mmol) 1-(3-Dimethylaminopropyl)-3-ethylcarbodiimid-Hydrochlorid sowie 148 mg (0.967 mmol) 1-Hydroxy-1*H*-benzotriazol-Hydrat versetzt. Die Mischung wurde 2 h bei RT gerührt. Der Ansatz wurde danach in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Der Rückstand wurde durch präparative HPLC aufgereinigt. Es wurden so 283 mg (53% d. Th.) des *tert*.-Butylester-Intermediats *N*-[(Benzyloxy)earbonyl]-*N*-methyl-L-threonyl-*N*-[(3*R*,4*S*,5*S*)-1-*tert*.-butoxy-3-methoxy-5-methyl-1-oxoheptan-4-yl]-*N*-methyl-L-valinamid erhalten.

HPLC (Methode 5): Rₜ = 2.17 min.

283 mg (0.466 mmol) dieses Intermediats wurden in 5 ml Dichlormethan aufgenommen, mit 5 ml wasserfreier Trifluoressigsäure versetzt und 2 h bei RT gerührt. Anschließend wurde der Ansatz im Hochvakuum eingeengt und der verbliebene Rückstand mittels präparativer HPLC gereinigt. Man erhielt 156 mg (61% d. Th.) der Titelverbindung als farblosen Schaum.

HPLC (Methode 5): Rₜ = 1.50 min;

LC-MS (Methode 2): Rₜ = 1.09 min; MS (ESIpos): m/z = 552 (M+H)⁺.

### Intermediat 33

### (2R)-2-Amino-1-(1,2-oxazinan-2-yl)-3-phenylpropan-1-on Trifluoressigsäure-Salz

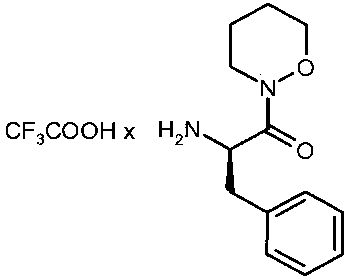

Die Titelverbindung wurde in Analogie zur Synthese von Intermediat 6 ausgehend von *N*-(*tert.-*Butoxycarbonyl)-D-phenylalanin hergestellt.

HPLC (Methode 6): Rₜ = 1.40 min;

LC-MS (Methode 1): Rₜ = 0.50 min; MS (ESIpos): m/z = 235 (M+H)⁺.

### Intermediat 34

### (2S)-2-Amino-2-methyl-1-(1,2-oxazinan-2-yl)-3-phenylpropan-1-on Trifluoressigsäure-Salz

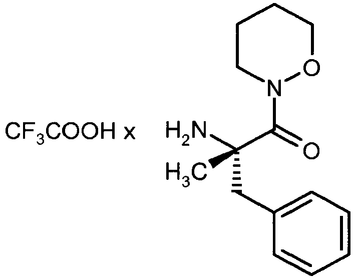

Die Titelverbindung wurde in Analogie zur Synthese von Intermediat 6 ausgehend von kommerziell erhältlichem *N*-(*tert*.-Butoxycarbonyl)-α-methyl-L-phenylalanin hergestellt.

HPLC (Methode 5): Rₜ = 0.45 min;

LC-MS (Methode 2): Rₜ = 0.61 min; MS (ESIpos): m/z = 249 (M+H)⁺.

### Ausführungsbeispiele:

### Beispiel 1

### N-Methyl-L-valyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid Trifluoressigsäure-Salz

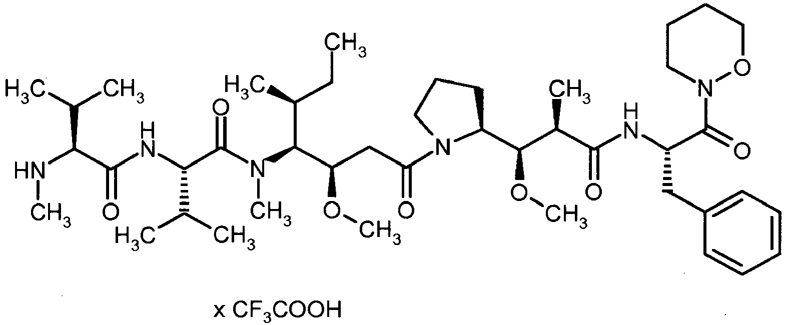

143 mg (0.223 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 15 ml DMF aufgenommen und nacheinander mit 141 mg (0.22 mmol) (2*R*,3*R*)-3-Methoxy-2-methyl-*N-*[(2*S*)-1-(1,2-oxazinan-2-yl)-1-oxo-3-phenylpropan-2-yl]-3-[(2*S*)-pyrrolidin-2-yl]propanamid Trifluoressigsäure-Salz (Intermediat 7), 102 mg (0.27 mmol) *O*-(7-Azabenzotriazol-1-yl)-*N*,*N*,*N'*,*N'-*tetramethyluronium-hexafluorophosphat (HATU) sowie 128 µl (0.74 mmol) *N,N-*Diisopropylethyl-amin versetzt. Die Mischung wurde 3 h bei RT gerührt. Der Ansatz wurde danach in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Man erhielt so 275 mg (quant.) des Fmoc-geschützten Intermediats *N*-[(9*H*-Fluoren-9-ylmethoxy)carbonyl]-*N*-methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid.

HPLC (Methode 5): Rₜ = 2.73 min;

LC-MS (Methode 4): Rₜ = 3.19 min; MS (ESIpos): m/z = 1023 (M+H)⁺.

46 mg (0.045 mmol) dieses Intermediats wurden in 4 ml DMF gelöst. Nach Zugabe von 1 ml Piperidin wurde der Ansatz 1 h bei RT gerührt. Anschließend wurde im Vakuum eingeengt und der Rückstand mittels präparativer HPLC gereinigt (Eluent: Acetonitril + 0.01% TFA / Wasser + 0.01% TFA). Es wurden 22 mg (54% d. Th.) der Titelverbindung als farbloser Schaum erhalten.

HPLC (Methode 5): Rₜ = 1.68 min;

LC-MS (Methode 2): Rₜ = 1.03 min; MS (ESIpos): m/z = 801 (M+H)⁺

¹H-NMR (600 MHz, DMSO-d₆): δ = 8.8 (m, 2H), 8.7 (m, 1H), 8.42 und 8.15 (2d, 1H), 7.3-7.1 (m, 5H), 5.12 und 4.95 (2m, 1H), 4.70 und 4.62 (2m, 1H), 4.62 und 4.50 (2t, 1H), 4.1-3.9 (m, 3H), 3.85 (m, 1H), 3.75-3.6 (m, 2H), 3.23, 3.18, 3.17, 3.14, 3.02 und 2.96 (6s, 9H), 3.1-2.9 und 2.75 (2m, 2H), 2.46 (m, 3H), 2.4-2.1 (m, 2H), 2.05 (br. m, 2H), 1.85-1.55 (br. m, 6H), 1.5-1.2 (br. m, 3H), 1.1-0.8 (m, 18H), 0.75 (t, 3H) [weitere Signale unter H₂O-Peak verborgen].

### Beispiel 2

### N-(2-Hydroxyethyl)-N-methyl-L-valy-N-[(3R,4S,5S)-3-methoxy-1-{(2S)-2-[1R,2R)-1-methoxy-2-methyl-3- {[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid

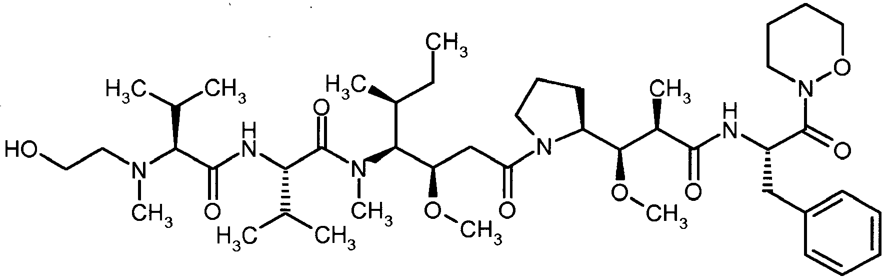

50 mg (0.0411 mmol) *N*-Methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxaziman-2-yl)-1-oxo-3-phenylpropan-2-yl]amino}-3-oxopropyl]pyrrolidin-1-yl}-5-methyl-1-oxoheptan-4-yl]-*N*-methyl-L-valinamid Trifluoressigsäure-Salz (Beispiel 1) wurden in 3 ml Dioxan/Wasser (1:1) gelöst und mit 4.9 mg (0.081 mmol) dimerem Glycolaldehyd versetzt. Anschließend wurden 2.8 mg (0.045 mmol) Natriumcyanoborhydrid zugegeben. Die Mischung wurde dann mit 50 µl 0.1 N Salzsäure auf pH 4-5 eingestellt und 1 h bei 100°C gerührt. Der Ansatz wurde danach in eine Mischung aus halbgesättigter wässriger Natriumhydrogencarbonat-Lösung und Ethylacetat gegossen. Die organische Phase wurde abgetrennt, mit gesättigter Natriumchlorid-Lösung gewaschen, über Magnesiumsulfat getrocknet, filtriert und eingeengt. Der Rückstand wurde durch präparative HPLC gereinigt. Es wurden 21 mg (61% d. Th.) der Titelverbindung in Form eines farblosen Schaumes erhalten.

HPLC (Methode 5): Rₜ = 1.69 min;

LC-MS (Methode 1): Rₜ = 0.92 min; MS (ESIpos): m/z = 845 (M+H)⁺

¹H-NMR (600 MHz, DMSO-d₆): δ = 9.2 (m, 1H), 8.9 (m, 1H), 8.4 und 8.15 (2d, 1H), 7.3-7.1 (m, 5H), 5.12 und 4.95 (2m, 1H), 4.70 und 4.62 (2m, 1H), 4.62 und 4.55 (2t, 1H), 4.1-3.9 (m, 3H), 3.9-3.7 (m, 5H), 3.23, 3.18, 3.17, 3.15, 3.02 und 2.98 (6s, 9H), 2.95 und 2.75 (2m, 2H), 2.8 (m, 3H), 2.46-2.00 (m, 4H), 1.85-1.55 (br. m, 6H), 1.5-1.2 (br. m, 3H), 1.1-0.8 (m, 18H), 0.75 (t, 3H) [weitere Signale unter H₂O-Peak verborgen].

### Beispiel 3

### N,N-Dimethyl-L-valyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid

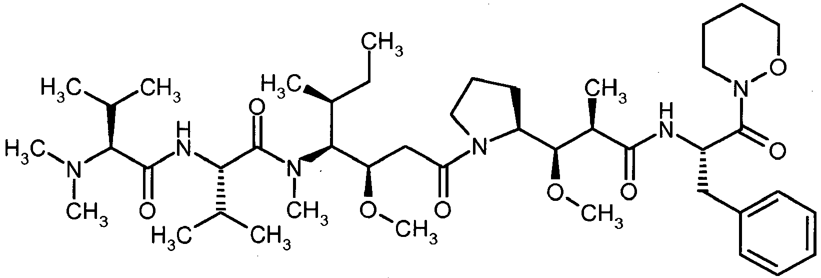

20 mg (0.022 mmol) *N*-Methyl-L-valyl-*N*-[(3*R*,4*S*,5*S*)-3-methoxy-1-{(2*S*)-2-[(1*R*,2R)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid Trifluoressigsäure-Salz (Beispiel 1) wurden in 1 ml DMF gelöst, mit 3.4 mg (1 µl) Iodmethan sowie 7.6 mg (0.055 mmol) Kaliumcarbonat versetzt und 1 h bei RT gerührt. Anschließend wurde nochmals die gleiche Menge an Kaliumcarbonat zugegeben und der Ansatz 10 min im Ultraschallbad behandelt. Das Lösungsmittel wurde danach im Vakuum abdestilliert und der verbliebene Rückstand durch präparative HPLC gereinigt. Es wurden 8 mg (44% d. Th.) der Titelverbindung erhalten.

HPLC (Methode 5): Rₜ = 1.71 min;

LC-MS (Methode 2): Rₜ = 1.04 min; MS (ESIpos): m/z = 815 (M+H)⁺

¹H-NMR (600 MHz, DMSO-d₆): δ = 9.55 (m, 1H), 8.9-8.7 (m, 1H), 8.45 und 8.15 (2d, 1H), 7.3-7.1 (m, 5H), 5.12 und 4.95 (2m, 1H), 4.70 und 4.62 (2m, 1H), 4.62 und 4.55 (2t, 1H), 4.1-3.9 (m, 3H), 3.9-3.6 (m, 3H), 3.55 (m, 2H), 3.23, 3.18, 3.17, 3.15, 3.02 und 2.98 (6s, 9H), 2.95 und 2.7 (2m, 2H), 2.8-2.7 (2 br. s, 6H), 2.46-2.00 (m, 4H), 1.85-1.55 (br. m, 6H), 1.5-1.2 (br. m, 3H), 1.1-0.8 (m, 18H), 0.75 (t, 3H) [weitere Signale unter H₂O-Peak verborgen].

### Beispiel 4

### N-Methyl-L-valyl-N-[(3R,4S,5S)-3-methoxy-1-{(2S)-2-[(1R,2R)-1-methoxy-2-methyl-3-{[(2S,3S)-1-(1,2-oxazinan-2-yl)-1-oxo-3-phenylbutan-2-yl]amino}-3-oxopropyl]pyrrolidin-1-yl}-5-methyl-1-oxoheptan-4-yl]-N-methyl-L-valinamid Trifluoressigsäure-Salz

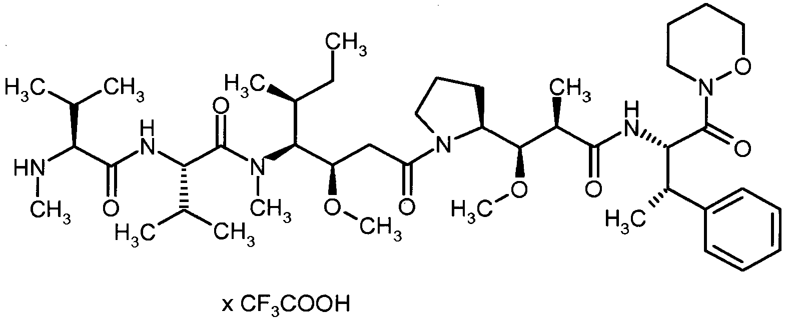

126 mg (0.198 mmol) *N*-[(9*H*-Fluoren-9-yhnethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 10 ml DMF aufgenommen und nacheinander mit 105 mg (0.198 mmol) (2*R*,3*R*)-3-Methoxy-2-methyl-*N-*[(2*S*,3*S*)-1-(1,2-oxazinan-2-yl)-1-oxo-3-phenylbutan-2-yl]-3-[(2*S*)-pyrrolidin-2-yl]propanamid Trifluoressigsäure-Salz (Intermediat 17), 41.6 mg (0.217 mmol) 1-(3-Dimethylaminopropyl)-3-ethyl-carbodiimid-Hydrochlorid, 33 mg (0.217 mmol) 1-Hydroxy-1*H*-benzotriazol-Hydrat sowie 79 µl (0.454 mmol) *N*,*N*-Diisopropylethylamin versetzt. Die Mischung wurde über Nacht bei RT gerührt. Der Ansatz wurde danach in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Es wurden so 220 mg (quant.) des Fmoc-geschützten Intermediats *N*-[(9*H*-Fluoren-9-ylmethoxy)carbonyl]-*N*-methyl-L-valyl-*N*-[(3R,4S,5S)-3-methoxy-1-{(2*S*)-2-[(1*R*,2*R*)-1-methoxy-2-methyl-3-{[(2*S*,3*S*)-1-(1,2-oxazinan-2-yl)-1-oxo-3-phenylbutan-2-yl]amino}-3-oxopropyl]pyrrolidin-1-yl}-5-methyl-1-oxoheptan-4-yl]-*N*-methyl-L-valinamid erhalten.

HPLC (Methode 5): Rₜ = 2.77 min;

LC-MS (Methode 1): Rₜ = 1.5 min; MS (ESIpos): m/z = 1037 (M+H)⁺.

220 mg (0.212 mmol) dieses Intermediats wurden in 5 ml DMF gelöst. Nach Zugabe von 1 ml Piperidin wurde der Ansatz 1 h bei RT gerührt. Anschließend wurde im Vakuum eingeengt und der Rückstand mittels präparativer HPLC gereinigt (Eluent: Acetonitril + 0.01% TFA / Wasser + 0.01% TFA). Es wurden 91 mg (46% d. Th.) der Titelverbindung als farbloser Schaum erhalten.

HPLC (Methode 5): Rₜ = 1.71 min;

LC-MS (Methode 1): Rₜ = 0.9 min; MS (ESIpos): m/z = 815 (M+H)⁺

¹H-NMR (600 MHz, DMSO-d₆): δ = 8.87 und 8.80 (2d, 2H), 8.75 (m, 1H), 8.40 und 7.98 (2d, 1H), 7.3-7.1 (m, 5H), 5.45 und 5.2 (2t, 1H), 4.78 und 4.62 (2m, 1H), 4.73 und 4.58 (2t, 1H), 4.2-4.0 (m, 3H), 3.7-3.6 (m, 1H), 3.35, 3.20, 3.18, 3.14, 3.12 und 3.00 (6s, 9H), 3.1 und 2.95 (2m, 2H), 2.46 (m, 3H), 2.4-2.0 (m, 4H), 1.9-1.6 (m, 4H), 1.6-1.2 (m, 5H), 1.1-0.75 (m, 21H), 0.80 (t, 3H) [weitere Signale unter H₂O-Peak verborgen].

### Beispiel 5

### N-Methyl-L-valyl-N-[(3R,4S,5S)-3-methoxy-1-{(2S)-2-[(1R,2R)-1-methoxy-2-methyl-3-{[(2S)-1-(1,2-oxazolidin-2-yl)-1-oxo-3-phenylpropan-2-yl]amino}-3-oxopropyl]pyrrolidin-1-yl}-5-methyl-1-oxoheptan-4-yl]-N-methyl-L-valinamid Trifluoressigsäure-Salz

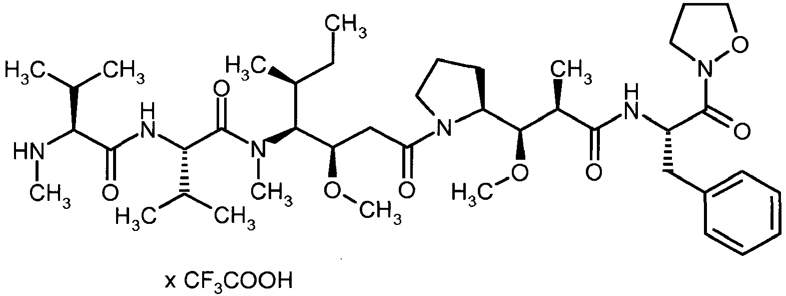

138 mg (0.216 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 14 ml DMF aufgenommen und nacheinander mit 109 mg (0.216 mmol) (2*R*,3*R*)-3-Methoxy-2-methyl-*N-*[(2*S*)-1-(1,2-oxazolidin-2-yl)-1-oxo-3-phenylpropan-2-yl]-3-[(2*S*)-pyrrolidin-2-yl]propanamid Trifluoressigsäure-Salz (Intermediat 15), 45.6 mg (0.238 mmol) 1-(3-Dimethylaminopropyl)-3-ethyl-carbodiimid-Hydrochlorid, 36.5 mg (0.38 mmol) 1-Hydroxy-1*H*-benzotriazol-Hydrat sowie 49 µl (0.281 mmol) *N*,*N*-Diisopropylethylamin versetzt. Die Mischung wurde über Nacht bei RT gerührt. Der Ansatz wurde danach auf eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Es wurden so 170 mg (78% d. Th.) des Fmoc-geschützten Intermediats *N*-[(9H-Fluoren-9-ylmethoxy)carbonyl]-*N*-methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxazolidin-2-yl)-1-oxo-3-phenylpropan-2-yl]amino}-3-oxopropyl]pyrrolidin-1-yl}-5-methyl-1-oxoheptan-4-yl]-*N*-methyl-L-valinamid erhalten.

HPLC (Methode 5): Rₜ = 2.64 min;

LC-MS (Methode 1): Rₜ = 1.44 min; MS (ESIpos): m/z = 1009 (M+H)⁺.

170 mg (0.168 mmol) dieses Intermediats wurden in 5 ml DMF gelöst. Nach Zugabe von 1 ml Piperidin wurde der Ansatz 1 h bei RT gerührt. Anschließend wurde im Vakuum eingeengt und der Rückstand mittels präparativer HPLC gereinigt (Eluent: Acetonitril + 0.01% TFA / Wasser + 0.01% TFA). Es wurden 37 mg (24% d. Th.) der Titelverbindung als farbloser Schaum erhalten.

HPLC (Methode 5): Rₜ = 1.57 min;

LC-MS (Methode 1): Rₜ = 0.84 min; MS (ESIpos): m/z = 787 (M+H)⁺

¹H-NMR (600 MHz, DMSO-d₆): δ = 8.9-8.7 (m, 3H), 8.5 und 8.2 (2m, 1H), 7.3-7.1 (m, 5H), 5.6 und 5.4 (2m, 1H), 4.7 und 4.6 (2m, 1H), 4.65 und 4.55 (2t, 1H), 4.0-3.9 (m, 3H), 3.75-3.6 (m, 2H), 3.23, 3.20, 3.15, 3.05 und 2.98 (5s, 9H), 3.0 und 2.7 (2m, 2H), 2.46 (m, 3H), 2.35-2.15 (m, 4H), 2.1-2.0 (m, 2H), 1.85-1.6 (m, 3H), 1.45 (m, 1H), 1.25 (m, 1H), 1.1-0.85 (m, 18H), 0.75 (t, 3H) [weitere Signale unter H₂O-Peak verborgen].

### Beispiel 6

*N*-Methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(2-oxa-3-azabicyclo[2.2.2]oct-5-en-3-yl)-1-oxo-3-phenylpropan-2-yl]amino}-3-oxopropyl]pyrrolidin-1-yl}-5-methyl-1-oxoheptan-4-yl]-*N*-methyl-L-valinamid Trifluoressigsäure-Salz

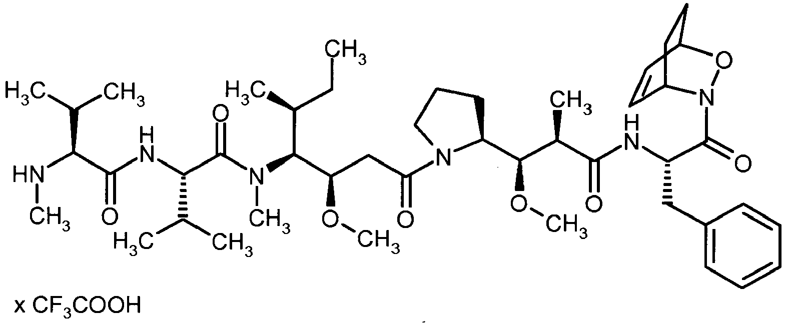

44.5 mg (0.071 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 10 ml DMF aufgenommen und nacheinander mit 38.6 mg (0.071 mmol) (2*R*,3*R*)-3-Methoxy-2-methyl-*N-*[(2*S*)-1-(2-oxa-3-azabicyclo[2.2.2]oct-5-en-3-yl)-1-oxo-3-phenylpropan-2-yl]-3-[(2*S*)-pyrrolidin-2-yl]propanamid Trifluoressigsäure-Salz (Intermediat 9), 32.5 mg (0.086 mmol) *O*-(7-Azabenzotriazol-1-yl)-*N*,*N*,*N'*,*N'*-tetramethyluronium-hexafluorophosphat (HATU) sowie 41 µl (0.235 mmol) *N,N-*Diisopropylethylamin versetzt. Die Mischung wurde 1 h bei RT gerührt. Der Ansatz wurde dann im Vakuum eingeengt und der Rückstand in Ethylacetat aufgenommen. Die organische Phase wurde nacheinander mit 5%-iger Zitronensäure-Lösung und 5%-iger Natriumhydrogencarbonat-Lösung gewaschen, über Magnesiumsulfat getrocknet, filtriert und eingeengt. Es wurden so 73 mg (98% d. Th.) des Fmoc-geschützten Intermediats *N*-[(9*H*-Fluoren-9-ylmethoxy)carbonyl]-*N*-methyl-L-valyl-*N*-[(3*R*,4*S*,5*S*)-3-methoxy-1-{(2*S*)-2-[(1*R*,2*R*)-1-methoxy-2-methyl-3-{[(2S)-1-(2-oxa-3-azabicyclo[2.2.2]oct-5-en-3-yl)-1-oxo-3-phenylpropan-2-yl]amino}-3-oxopropyl]pyrrolidin-1-yl}-5-methyl-1-oxoheptan-4-yl]-*N*-methyl-L-valinamid erhalten.

HPLC (Methode 6): Rₜ = 2.78 min;

LC-MS (Methode 3): Rₜ = 2.96 min; MS (ESIpos): m/z = 1047 (M+H)⁺.

73 mg (0.071 mmol) dieses Intermediats wurden in 5 ml DMF gelöst. Nach Zugabe von 0.5 ml Piperidin wurde der Ansatz 10 min bei RT gerührt. Anschließend wurde im Vakuum eingeengt und der Rückstand mehrfach mit Diethylether digeriert. Nach Abdekantieren des Ethers wurde der Rückstand durch präparative HPLC gereinigt (Eluent: Acetonitril / 0.1% aq. TFA). Es wurden 16 mg (26% d. Th.) der Titelverbindung als farbloser Schaum erhalten.

HPLC (Methode 6): Rₜ = 1.94 min;

LC-MS (Methode 3): Rₜ = 1.71 min; MS (ESIpos): m/z = 825 (M+H)⁺

¹H-NMR (400 MHz, DMSO-d₆): δ = 8.9-8.6 (m, 3H), 8.4, 8.3, 8.1 und 8.0 (4d, 1H), 7.3-7.1 (m, 5H), 6.7-6.5 (m, 2H), 5.2-4.8 (m, 3H), 4.75-4.55 (m, 3H), 4.05-3.95 (m, 1H), 3.7-3.4 (m, 4H), 3.22, 3.17, 3.15, 3.05, 3.02 und 2.95 (6s, 9H), 3.0 und 2.7 (2 br. m, 2H), 2.46 (m, 3H), 2.4-1.2 (br. m, 13H), 1.1-0.85 (m, 18H), 0.75 (m, 3H) [weitere Signale unter H₂O-Peak verborgen].

### Beispiel 7

### N-Methyl-L-valyl-N-[(3R,4S,5S)-1-{(2S)-2-[(1R,2R)-3-{[(2S)-3-(4-hydroxy-3-nitrophenyl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

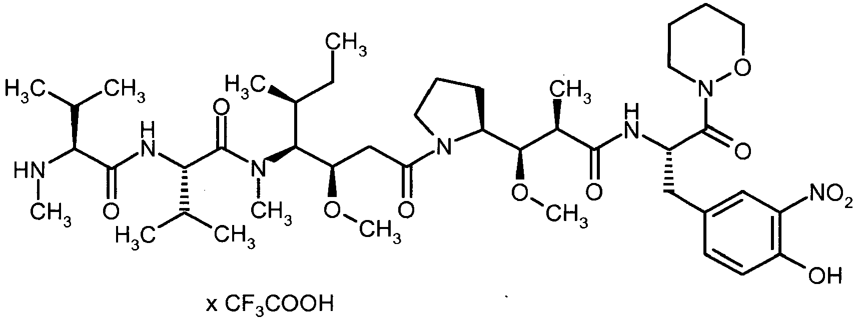

160 mg (0.251 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 13 ml DMF aufgenommen und nacheinander mit 145 mg (0.251 mmol) (2*R*,3*R*)-*N*-[(2*S*)-3-(4-Hydroxy-3-nitrophenyl)-1-(1,2-oxazinan-2-yl)-1-oxopropan-2-yl]-3-methoxy-2-methyl-3-[(2S)-pyrrolidin-2-yl]-propanamid Trifluoressigsäure-Salz (Intermediat 19), 53 mg (0.276 mmol) 1-(3-Dimethylamino-propyl)-3-ethylcarbodiimid-Hydrochlorid, 42.2 mg (0.276 mmol) 1-Hydroxy-1*H-*benzotriazol-Hydrat sowie 65 µl (0.376 mmol) *N,N*-Diisopropylethylamin versetzt. Die Mischung wurde über Nacht bei RT gerührt. Der Ansatz wurde dann in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Es wurden 260 mg (96% d. Th.) des rohen Fmoc-geschützten Intermediats *N*-[(9*H*-Fluoren-9-ylmethoxy)carbonyl]-*N*-methyl-L-valyl-*N*-[(3*R*,4*S*,5*S*)-1-{(2*S*)-2-[(1*R*,2*R*)-3-{[(2*S*)-3-(4-hydroxy-3-nitrophenyl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid erhalten, welches ohne weitere Reinigung in der nächsten Stufe eingesetzt wurde.

260 mg (0.24 mmol) dieses Intermediats wurden in 3 ml DMF gelöst. Nach Zugabe von 0.6 ml Piperidin wurde der Ansatz 1 h bei RT gerührt. Anschließend wurde im Vakuum eingeengt und der Rückstand durch präparative HPLC gereinigt (Eluent: Acetonitril + 0.01% TFA / Wasser + 0.01% TFA). Es wurden 105 mg (45% d. Th.) der Titelverbindung als leicht gelblicher Schaum erhalten.

HPLC (Methode 5): Rₜ = 1.62 min;

LC-MS (Methode 1): Rₜ = 0.87 min; MS (ESIpos): m/z = 862 (M+H)⁺

¹H-NMR (500 MHz, DMSO-d₆): δ = 10.85 und 10.72 (2s, 1H), 8.9-8.6 (m, 3H), 8.45 und 8.2 (2d, 1H), 7.70 und 7.72 (2s, 1H), 7.40 und 7.32 (2d, 1H), 7.01 und 7.00 (2d, 1H), 5.0-4.85 (m, 1H), 4.7-4.5 (m, 2H), 4.15-4.0 (m, 2H), 3.95-3.75 (m, 2H), 3.7-3.6 (m, 2H), 3.28, 3.21, 3.16, 3.15, 3.02 und 2.96 (6s, 9H), 2.9 und 2.75 (2m, 2H), 2.46 (m, 3H), 2.4-2.3 (m, 1H), 2.3-2.2 (m, 1H), 2.1-1.95 (br. m, 2H), 1.85-1.7 (m, 4H), 1.7-1.55 (m, 2H), 1.55-1.35 (m, 2H), 1.35 (m, 1H), 1.1-0.8 (m, 18H), 0.75 (t, 3H) [weitere Signale unter H₂O-Peak verborgen].

### Beispiel 8

### N-Methyl-L-valyl-N-[(3R,4S,5S)-1-{(2S)-2-[(1R,2R)-3-{[(2S)-1-(1,4-dihydro-3H-2,3-benzoxazin-3-yl)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

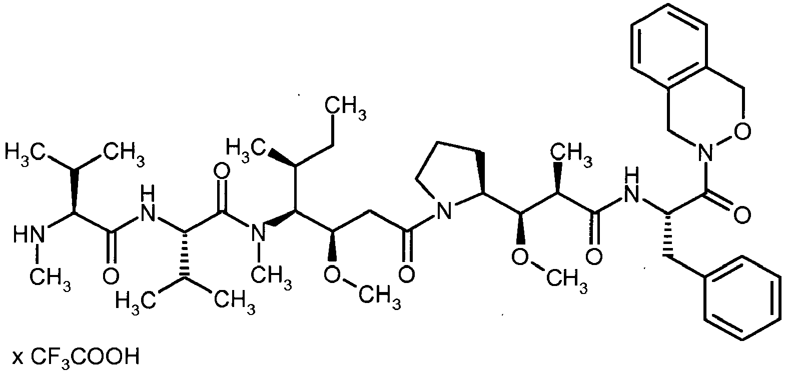

15.8 mg (0.025 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 5 ml DMF aufgenommen und nacheinander mit 14 mg (0.025 mmol) (2*R*,3*R*)-*N*-[(2*S*)-1-(1,4-Dihydro-3*H*-2,3-benzoxazin-3-yl)-1-oxo-3-phenylpropan-2-yl]-3-methoxy-2-methyl-3-[(2*S*)-pyrrolidin-2-yl]-propanamid Trifluoressigsäure-Salz (Intermediat 11), 11.3 mg (0.03 mmol) *O*-(7-Azabenzotriazol-1-yl)-*N*,*N*,*N*',*N'*-tetramethyluronium-hexafluorophosphat (HATU) sowie 14 µl (0.082 mmol) *N,N-*Diisopropylethylamin versetzt. Die Mischung wurde 10 min bei RT gerührt. Der Ansatz wurde dann im Vakuum eingeengt und der Rückstand direkt durch präparative HPLC gereinigt. Man erhielt so 13 mg (49% d. Th.) des Fmoc-geschützten Intermediats *N*-[(9*H*-Fluoren-9-ylmethoxy)carbonyl]-N-methyl-L-valyl-*N*-[(3*R*,4*S*,5*S*)-1-{(2*S*)-2-[(1*R*,2*R*)-3-{[(2*S*)-1-(1,4-dihydro-3*H*-2,3-benzoxazin-3-yl)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid.

HPLC (Methode 6): Rₜ = 2.93 min;

LC-MS (Methode 3): Rₜ = 3.10 min; MS (ESIpos): m/z = 1071 (M+H)⁺.

13 mg (0.012 mmol) dieses Intermediats wurden in 2 ml DMF gelöst. Nach Zugabe von 0.5 ml Piperidin wurde der Ansatz 10 min bei RT gerührt. Anschließend wurde im Vakuum eingeengt und der Rückstand durch präparative HPLC gereinigt (Eluent: Acetonitril / 0.1 % aq. TFA). Es wurden 10 mg (97% d. Th.) der Titelverbindung erhalten.

HPLC (Methode 6): Rₜ = 2.10 min;

LC-MS (Methode 1): Rₜ = 0.97 min; MS (ESIpos): m/z = 849 (M+H)⁺

¹H-NMR (400 MHz, DMSO-d₆): δ = 8.90-8.62 (m, 3H), 8.60 und 8.33 (2d, 1H), 7.35-7.1 (m, 9H), 5.4-5.0 (m, 4H), 4.7-4.5 (m, 3H), 3.95 (m, 1H), 3.7-3.4 (m, 3H), 3.24, 3.20, 3.18, 3.16, 3.10 und 3.08 (6s, 9H), 3.0 und 2.85 (2 br. m, 2H), 2.46 (m, 3H), 2.3 (br. m, 2H), 2.05 (br. m, 2H), 1.9-1.6 (m, 3H), 1.5-1.2 (br. m, 2H), 1.1-0.85 (m, 18H), 0.75 (m, 3H) [weitere Signale unter H₂O-Peak verborgen].

### Beispiel 9

### N-Methyl-L-valyl-N-[(3R,4S,5S)-1-{(2S)-2-[(1R,2R)-3-{[(2S)-3-(1H-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

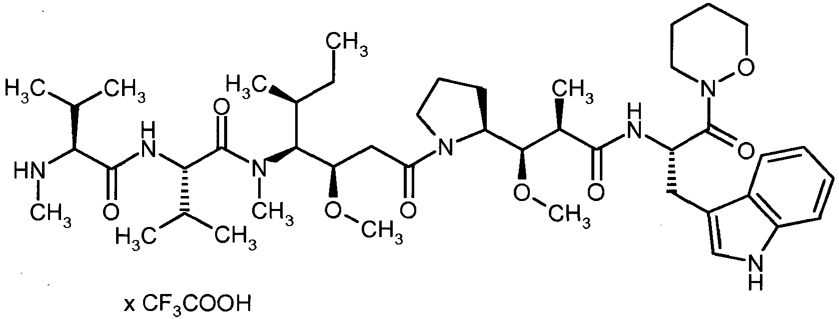

40 mg (0.076 mmol) *N*-(*tert*.-Butoxycarbonyl)-*N*-methyl-L-valyl-*N*-[(2*R*,3*S*,4*S*)-1-carboxy-2-meth-oxy-4-methylhexan-3-yl]-*N*-methyl-L-valinamid (Intermediat 20) wurden in 5 ml DMF aufgenommen und nacheinander mit 43 mg (0.078 mmol) (2*R*,3*R*)-*N*-[(2*S*)-3-(2*S*)-Indol-3-yl)-1-(1,2-oxazinan-2-yl)-1-oxopropan-2-yl]-3-methoxy-2-methyl-3-[(2*S*)-pyrrolidin-2-yl]propanamid Trifluoressigsäure-Salz (Intermediat 13), 35 mg (0.093 mmol) *O*-(7-Azabenzotriazol-1-yl)-*N,N,N',N'*-tetra-methyluronium-hexafluorophosphat (HATU) sowie 45 µl (0.256 mmol) *N,N-*Diisopropylethylamin versetzt. Die Mischung wurde 2 h bei RT gerührt. Der Ansatz wurde dann im Vakuum eingeengt und der Rückstand direkt durch präparative HPLC gereinigt. Man erhielt so 10 mg (14% d. Th.) des Boc-geschützten Intermediats *N*-(*tert*.-Butoxycarbonyl)-*N*-methyl-L-valyl-*N*-[(3*R*,4*S*,5*S*)-1-{(2*S*)-2-[(1*R*,2*R*)-3-{[(2*S*)-3-(1*H*-indol-3-yl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid.

HPLC (Methode 6): Rₜ = 2.52 min;

LC-MS (Methode 1): Rₜ = 1.35 min; MS (ESIpos): m/z = 940 (M+H)⁺.

5 mg (0.005 mmol) dieses Intermediats wurden in 3 ml Dichlormethan gelöst. Nach Zugabe von 0.5 ml Trifluoressigsäure wurde der Ansatz 30 min im Ultraschallbad behandelt und anschließend weitere 30 min bei RT gerührt. Danach wurde im Vakuum eingeengt, der Rückstand mit Acetonitril versetzt und erneut eingeengt. Nach Lyophilisation aus Dioxan/Wasser wurden 5 mg (99% d. Th.) der Titelverbindung erhalten.

HPLC (Methode 6): Rₜ = 1.94 min;

LC-MS (Methode 2): Rₜ = 0.99 min; MS (ESIpos): m/z = 840 (M+H)⁺

¹H-NMR (400 MHz, DMSO-d₆): δ = 10.8 und 8.8 (2m, 3H), 8.35 und 8.05 (2d, 1H), 7.55 (m, 1H), 7.3 (d, 1H), 7.20-6.91 (m, 3H), 5.12 und 4.95 (2m, 1H), 4.7-4.5 (m, 2H), 4.1-3.9 (m, 2H), 3.85 (m, 2H), 3.75-3.4 (m, 5H), 3.21, 3.15, 3.14, 3.10, 2.95 und 2.85 (6s, 9H), 2.46 (m, 3H), 2.4-2.2 (m, 2H), 2.1-1.9 (m, 2H), 1.85-1.65 (m, 4H), 1.65-1.2 (m, 3H), 1.05 und 1.0 (2d, 3H), 0.95-0.8 (m, 15H), 0.75 (m, 3H) [weitere Signale unter H₂O-Peak verborgen].

### Beispiel 10

### N-Methyl-L-valyl-N-[(3R,4S,5S)-1-{(2S)-2-[(1R,2R)-3-{[(2S)-3-(4-hydroxy-3-nitrophenyl)-1-(1,2-oxazinan-2-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid-Hydrochlorid

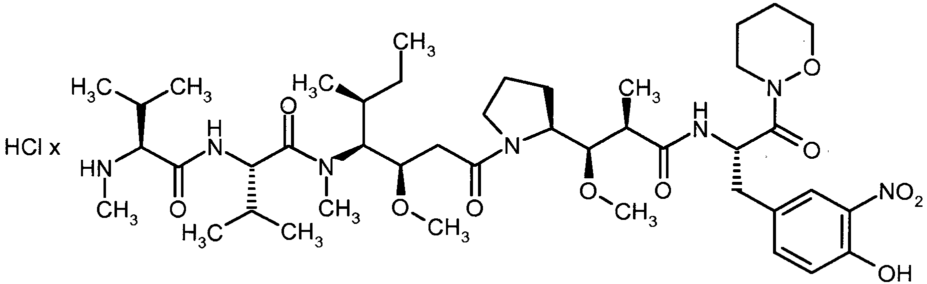

25 mg (0.026 mmol) *N*-Methyl-L-valyl-*N*-[(3*R*,4*S*,5*S*)-1-{(2*S*)-2-[(1*R*,2*R*)-3-{[(2*S*)-3-(4-hydroxy-3-nitrophenyl)-1-(1,2-oxazinan-2-yl)-1-oxopropan-2-yl]amino}-1-methoxy-2-methyl-3-oxopropyl]-pyrrolidin-1-yl}-3-methoxy-5-methyl-1-oxoheptan-4-yl]-*N*-methyl-L-valinamid Trifluoressigsäure-Salz (Beispiel 7) wurden in 2 ml 1 N Salzsäure gelöst und anschließend lyophilisiert. Es wurden so 16.4 mg (71% d. Th.) der Titelverbindung als leicht gelblicher Schaum erhalten.

HPLC (Methode 5): Rₜ = 1.63 min;

LC-MS (Methode 1): Rₜ = 0.87 min; MS (ESIpos): m/z = 862 (M+H)⁺.

### Beispiel 11

### N-Methyl-L-valyl-N-[(3R,4S,5S)-1-{(2S)-2-[(1R,2R)-3-({(2S)-1-[4-(benzyloxy)-1,2-oxazolidin-2-yl]-1-oxo-3-phenylpropan-2-yl}amino)-1-methoxy-2-methyl-3-oxopropyl]pyrrolidin-1-yl}-3-meth-oxy-5-methyl-1-oxoheptan-4-yl]-N-methyl-L-valinamid Trifluoressigsäure-Salz (Diastereomer 1)

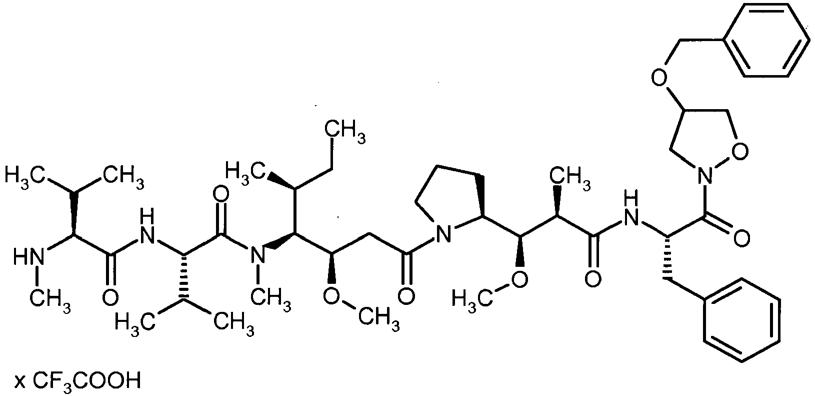

Die Titelverbindung wurde in Analogie zur Synthese von Beispiel 8 durch Kupplung von Ausgangsverbindung 1 mit Intermediat 28, anschließende Entschützung mit Trifluoressigsäure, dann Kupplung mit Intermediat 4 und schließlich Entschützung mit Piperidin hergestellt. Die Reinigung des Endprodukts erfolgte durch präparative HPLC (Eluent: Acetonitril / 0.1 % aq. TFA). Es wurden 16.4 mg der Titelverbindung erhalten.

HPLC (Methode 5): Rₜ = 2.08 min;

LC-MS (Methode 3): Rₜ = 1.90 min; MS (ESIpos): m/z = 893 (M+H)⁺

¹H-NMR (400 MHz, DMSO-d₆): δ = 8.8-8.6 (m, 3H), 8.45 und 8.4 (2d, 1H), 7.35-7.1 (m, 10H), 5.2 und 5.0 (2m, 1H), 4.7-4.5 (m, 5H), 4.15 (d, 1H), 4.0-3.6 (m, 5H), 3.6-3.4 (m, 2H), 3.75-3.4 (m, 5H), 3.22, 3.16, 3.15, 3.05 und 2.96 (5s, 9H), 3.1-2.9 und 2.8-2.6 (2m, 2H), 2.45 und 2.44 (2s, 3H), 2.4-2.2 (m, 2H), 2.1-2.0 (m, 2H), 1.85-1.6 (m, 2H), 1.5-1.2 (m, 3H), 1.1-0.8 (m, 18H), 0.75 (m, 3H).

### Beispiel 12

### N-Methyl-L-valyl-N-[(3R,4S,5S)-1-{(2S)-2-[(1R,2R)-3-({(2S)-1-[4-(benzyloxy)-1,2-oxazolidin-2-yl]-1-oxo-3-phenylpropan-2-yl}amino)-1-methoxy-2-methyl-3-oxopropyl]pyrrolidin-1-yl}-3-meth-oxy-5-methyl-1-oxoheptan-4-yl]-N-methyl-L-valinamid Trifluoressigsäure-Salz (Diastereomer 2)

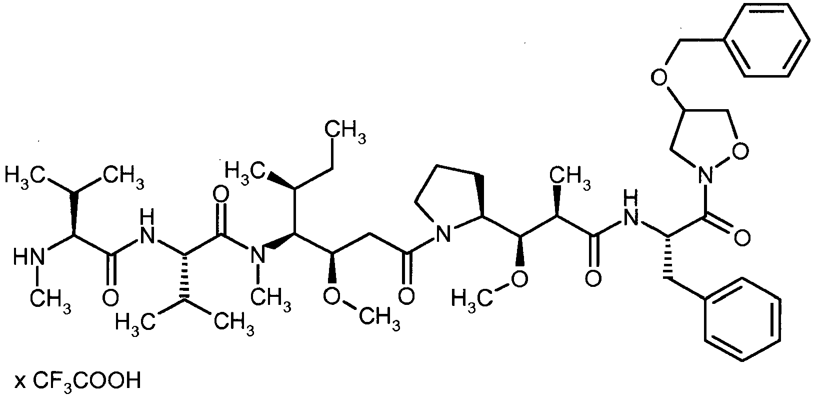

Die Titelverbindung wurde in Analogie zur Synthese von Beispiel 8 durch Kupplung von Ausgangsverbindung 1 mit Intermediat 29, anschließende Entschützung mit Trifluoressigsäure, dann Kupplung mit Intermediat 4 und schließlich Entschützung mit Piperidin hergestellt. Die Reinigung des Endprodukts erfolgte durch präparative HPLC (Eluent: Acetonitril / 0.1 % aq. TFA). Es wurden 28 mg der Titelverbindung erhalten.

HPLC (Methode 5): Rₜ = 2.11 min;

LC-MS (Methode 7): Rₜ = 1.96 min; MS (ESIpos): m/z = 893 (M+H)⁺

¹H-NMR (400 MHz, DMSO-d₆): δ = 8.9-8.6 (m, 3H), 8.45 und 8.3 (2d, 1H), 7.4-7.1 (m, 10H), 5.2 und 4.95 (2m, 1H), 4.7-4.5 (m, 5H), 4.25 (d, 1H), 4.0-3.8 (m, 3H), 3.7-3.6 (m, 2H), 3.6-3.4 (m, 2H), 3.23, 3.19, 3.18, 3.15, 3.05 und 2.96 (6s, 9H), 3.1-3.0 und 2.8-2.65 (2m, 2H), 2.5-2.4 (m, 3H), 2.35-2.15 (m, 2H), 2.1-1.95 (m, 2H), 1.8-1.6 (m, 2H), 1.5-1.2 (m, 3H), 1.1-0.8 (m, 18H), 0.75 (m, 3H).

### Beispiel 13

### N-Methyl-L-valyl-N-[(3R,4S,5S)-1-{(2S)-2-[(1R,2R)-3-{[(2S)-1-(4-hydroxy-1,2-oxazolidin-2-yl)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid (Diastereomer 2)

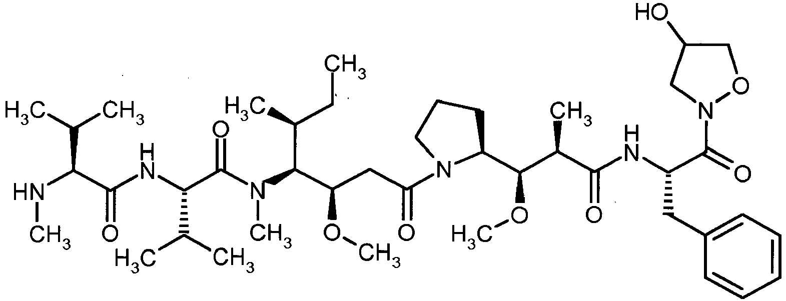

27 mg (0.03 mmol) der Verbindung aus Beispiel 12 wurden in einer Mischung aus 42 ml THF und 22 ml Wasser aufgenommen, mit 1.35 ml 2 M Salzsäure versetzt und 20 min bei RT und Normaldruck über 10% Palladium/Kohle hydriert. Der Katalysator wurde anschließend ab filtriert und die Reaktionsmischung mit wässriger Natriumhydrogencarbonat-Lösung neutralisiert. Dann wurde mit Ethylacetat extrahiert. Nach Trennung der Phasen wurde die Ethylacetat-Phase eingeengt und der Rückstand durch Flash-Chromatographie an Kieselgel mit Dichlormethan/Methanol/17% aq. Ammoniak (125:3:0.3) als Eluent gereinigt. Die entsprechenden Fraktionen wurden eingeengt und der Rückstand aus Dioxan lyophilisiert. Es wurden 25 mg der Titelverbindung erhalten.

HPLC (Methode 6): Rₜ = 1.74 min;

LC-MS (Methode 8): Rₜ = 2.06 min; MS (ESIpos): m/z = 803 (M+H)⁺

¹H-NMR (400 MHz, DMSO-d₆): δ = 8.45 und 8.2 (2d, 1H), 8.1 (m, 1H), 7.3-7.1 (m, 5H), 5.5 (m, 1H), 5.15 und 4.95 (2m, 1H), 4.7-4.4 (m, 3H), 4.1-3.9 (m, 3H), 3.5-3.4 (m, 3H), 3.23, 3.19, 3.18, 3.16, 3.05 und 2.96 (6s, 9H), 3.1-3.0 und 2.8-2.65 (2m, 2H), 2.4-2.15 (m, 5H), 2.1-1.95 (m, 2H), 1.8-1.6 (m, 3H), 1.5-1.2 (m, 2H), 1.05-0.8 (m, 18H), 0.75 (m, 3H).

### Beispiel 14

### N-Methyl-L-valyl-N-[(3R,4S,5S)-1-{(2S)-2-[(1R,2R)-3-{[(2S)-3,3-dimethyl-1-(1,2-oxazinan-2-yl)-1-oxobutan-2-yl]amino}-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

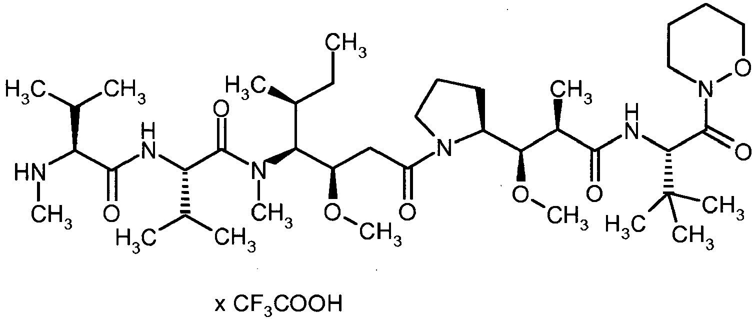

Die Titelverbindung wurde über drei Stufen ausgehend von Intermediat 21 zunächst durch Entschützung mit Trifluoressigsäure, anschließende Kupplung mit Intermediat 4 und schließlich Entschützung mit Piperidin hergestellt. Die Reinigung des Endprodukts erfolgte durch präparative HPLC (Eluent: Acetonitril + 0.01% TFA / Wasser + 0.01% TFA). Es wurden 2.5 mg der Titelverbindung erhalten.

HPLC (Methode 5): Rₜ = 1.67 min;

LC-MS (Methode 1): Rₜ = 0.90 min; MS (ESIpos): m/z = 767 (M+H)⁺.

### Beispiel 15

### N-Methyl-L-valyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3,3-diphenylpropan-2-yl]amino}-3-oxopropyl]pyrrolidin-1-yl}-5-methyl-1-oxoheptan-4-yl]-N-methyl-L-valinamid Trifluoressigsäure-Salz

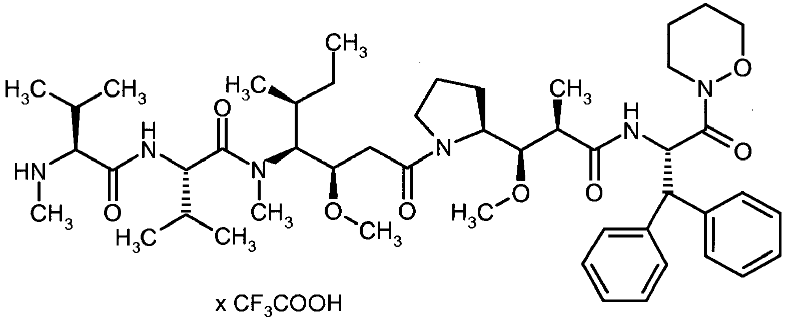

Die Titelverbindung wurde über drei Stufen ausgehend von Intermediat 22 zunächst durch Entschützung mit Trifluoressigsäure, anschließende Kupplung mit Intermediat 4 und schließlich Entschützung mit Piperidin hergestellt. Die Reinigung des Endprodukts erfolgte durch präparative HPLC (Eluent: Acetonitril + 0.01% TFA / Wasser + 0.01% TFA). Es wurden 20 mg der Titelverbindung erhalten.

HPLC (Methode 5): Rₜ = 1.78 min;

LC-MS (Methode 1): Rₜ = 0.95 min; MS (ESIpos): m/z = 877 (M+H)⁺.

### Beispiel 16

### N-Methyl-L-valyl-N-[(3R,4S,5S)-3-methoxy-1-{(2S)-2-[(1R,2R)-1-methoxy-2-methyl-3-{[(1S,2S)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid Trifluoressigsäure-Salz

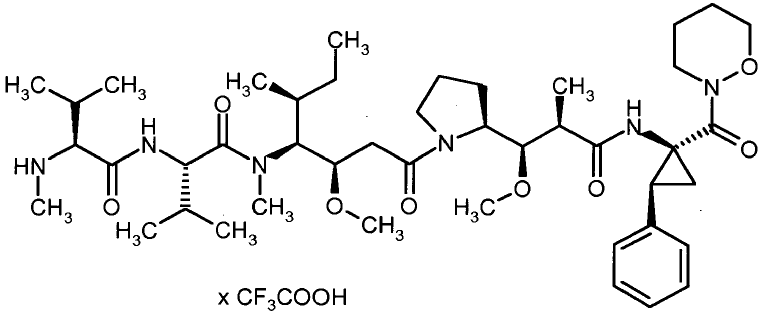

Die Titelverbindung wurde über drei Stufen ausgehend von Intermediat 23 zunächst durch Entschützung mit Trifluoressigsäure, anschließende Kupplung mit Intermediat 4 und schließlich Entschützung mit Piperidin hergestellt. Die Reinigung des Endprodukts erfolgte durch präparative HPLC (Eluent: Acetonitril / 0.1% aq. TFA). Es wurden 9 mg der Titelverbindung erhalten.

HPLC (Methode 6): Rₜ = 1.95 min;

LC-MS (Methode 1): Rₜ = 0.90 min; MS (ESIpos): m/z = 813 (M+H)⁺.

¹H-NMR (500 MHz, DMSO-d₆): δ = 8.85 (m, 2H), 8.7 (m, 1H), 8.2 und 7.8 (2s, 1H), 7.3-7.1 (m, 5H), 4.75 und 4.65 (2m, 1H), 4.7 und 4.6 (2t, 1H), 4.0-3.9 (m, 2H), 3.85 (m, 1H), 3.75-3.6 (m, 4H), 3.55 (m, 1H), 3.22, 3.20, 3.18, 3.17, 3.03 und 2.98 (6s, 9H), 2.92 und 2.82 (2t, 1H), 2.5-2.45 (m, 3H), 2.4-1.3 (m, 15H), 1.0-0.7 (m, 18H), 0.65 und 0.6 (2d, 3H) [weitere Signale unter H₂O-Peak verborgen].

### Beispiel 17

### N-Methyl-L-valyl-N-[(3R,4S,5S)-3-methoxy-1-{(2S)-2-[(1R,2R)-1-methoxy-2-methy1-3-{[(1S,2R)-1-(1,2-oxazinan-2-ylcarbonyl)-2-phenylcyclopropyl]amino}-3-oxopropyl]pyrrolidin-1-yl}-5-methyl-1-oxoheptan-4-yl]-N-methyl-L-valinamid Trifluoressigsäure-Salz

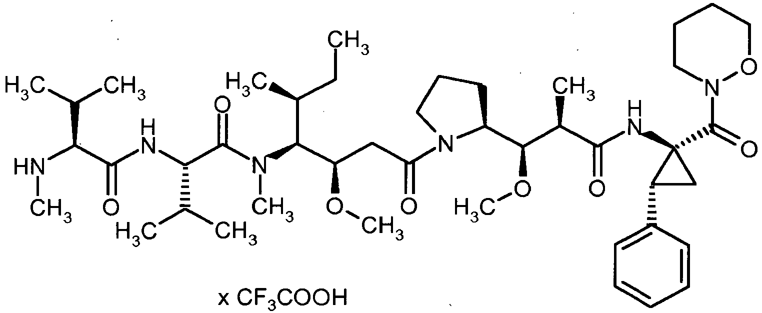

617 mg (1.2 mmol) *tert*.-Butyl-(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]pyrrolidin-1-carboxylat (Intermediat 24) wurden in 44 ml Dichlormethan aufgenommen, mit 4.4 ml Trifluoressigsäure versetzt und 30 min bei RT gerührt. Anschließend wurde im Vakuum eingeengt und der verbliebene Rückstand aus Dioxan/Wasser lyophilisiert. Es wurden 702 mg (quant.) der entschützten Verbindung (2*R*,3*R*)-3-Methoxy-2-methyl-*N*-[(1*S*,2*R*)-1-(1,2-oxazinan-2-ylcarbonyl)-2-phenylcyclopropyl]-3-[(2*S*)-pyrrolidin-2-yl]propanamid Trifluoressigsäure-Salz als Rohprodukt erhalten, das ohne weitere Reinigung in der folgenden Stufe eingesetzt wurde.

470 mg (0.74 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N*-methyl-L-valinamid (Intermediat 4) wurden in 57 ml DMF aufgenommen und nacheinander mit 390 mg (ca. 0.74 mmol) des oben erhaltenen (2*R*,3*R*)-3-Methoxy-2-methyl-*N*-[(1*S*,2*R*)-1-(1,2-oxazinan-2-ylcarbonyl)-2-phenylcyclopropyl]-3-[(2*S*)-pyrrolidin-2-yl]propanamid Trifluoressigsäure-Salzes, 336 mg (0.88 mmol) *O*-(7-Azabenzotriazol-1-yl)-*N,N,N',N'*-tetramethyluronium-hexafluorophosphat (HATU) sowie 423 µl (2.4 mmol) *N,N*-Diisopropylethylamin versetzt. Die Mischung wurde 2 h bei RT gerührt. Der Ansatz wurde dann in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Natriumsulfat getrocknet, filtriert und eingeengt. Der Rückstand wurde durch präparative HPLC gereinigt. Man erhielt so 453 mg (59% d. Th.) des Fmoc-geschützten Intermediats *N*-[(9*H*-Fluoren-9-ylmethoxy)carbonyl]-*N*-methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]pyrrolidin-1-yl}-5-methyl-1-oxoheptan-4-yl]-*N*-methyl-L-valinamid.

HPLC (Methode 5): Rₜ = 2.58 min;

LC-MS (Methode 1): Rₜ = 3.10 min; MS (ESIpos): m/z = 1035 (M+H)⁺.

453 mg (0.438 mmol) dieses Intermediats wurden in 24 ml DMF gelöst. Nach Zugabe von 2.4 ml Piperidin wurde der Ansatz 30 min bei RT gerührt. Anschließend wurde im Vakuum eingeengt und der Rückstand durch präparative HPLC gereinigt (Eluent: Acetonitril / 0.1 % aq. TFA). Es wurden 260 mg (64% d. Th.) der Titelverbindung als farbloser Schaum erhalten.

HPLC (Methode 5): Rₜ = 1.64 min;

LC-MS (Methode 1): Rₜ = 0.86 min; MS (ESIpos): m/z = 813 (M+H)⁺

¹H-NMR (400 MHz, DMSO-d₆): δ = 8.8 (m, 2H), 8.65 (m, 2H), 7.3-7.1 (m, 5H), 4.8-4.05 (m, 2H), 4.0 und 3.82 (2m, 2H), 3.8-3.5 (m, 8H), 3.32, 3.29, 3.20, 3.19, 3.12 und 3.00 (6s, 9H), 2.65 (t, 1H), 2.5-2.45 (m, 3H), 2.4-1.3 (m, 15H), 1.15-0.85 (m, 18H), 0.8 und 0.75 (2d, 3H) [weitere Signale unter H₂O-Peak verborgen].

### Beispiel 18

### N-Methyl-L-valyl-N-[(3R,4S,5S)-1-{(2S)-2-[(1R,2R)-3-{[(1S,2R)-1-(1,4-dihydro-3H-2,3-benzoxazin-3-ylcarbonyl)-2-phenylcyclopropyl]amino}-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

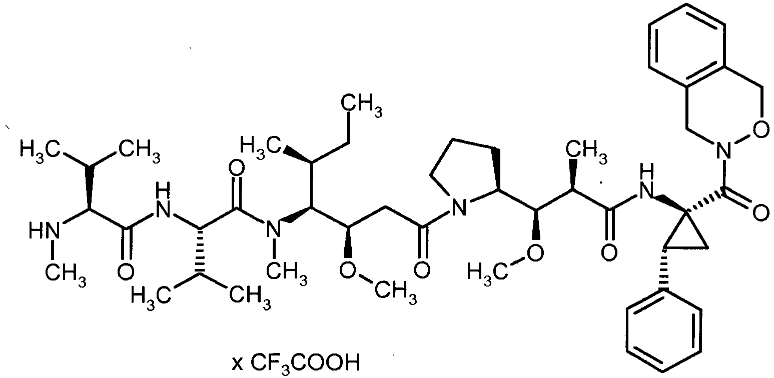

166 mg (0.196 mmol) *N*-[(9*H*-Fluoren-9-ylmethoxy)carbonyl]-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 30) wurden in 40 ml DMF aufgenommen und nacheinander mit 80 mg (0.196 mmol) [(1*S*,2*R*)-1-Amino-2-phenylcyclopropyl](1,4-dihydro-3*H*-2,3-benzoxazin-3-yl)methanon Trifluoressigsäure-Salz (Intermediat 31), 112 mg (0.294 mmol) *O*-(7-Azabenzotriazol-1-yl)-*N,N,N',N'*-tetramethyluronium-hexafluorophosphat (HATU) sowie 682 µl (3.9 mmol) *N*,*N-*Diisopropylethylamin versetzt. Die Mischung wurde anschließend über Nacht bei RT gerührt. Der Ansatz wurde danach im Vakuum eingeengt, der Rückstand in Ethylacetat aufgenommen und die Lösung mit gesättigter wässriger Natriumchlorid-Lösung gewaschen. Die organische Phase wurde über Magnesiumsulfat getrocknet, filtriert und eingeengt. Der Rückstand wurde schließlich durch präparative HPLC gereinigt. Man erhielt auf diese Weise 19 mg (9% d. Th.) des Fmoc-geschützten Intermediats *N*-[(9*H*-Fluoren-9-ylmethoxy)carbonyl]-*N*-methyl-L-valyl-*N*-[(3*R*,4*S*,5*S*)-1-{(2*S*)-2-[(1*R*,2*R*)-3-{[(1*S*,2*R*)-1-(1,4-dihydro-3*H*-2,3-benzoxazin-3-ylcarbonyl)-2-phenylcyclopropyl]-amino}-1-methoxy-2-methyl-3-oxopropyl]pyrrolidin-1-yl}-3-methoxy-5-Methyl-1-oxoheptan-4-yl]-*N*-methyl-L-valinamid.

HPLC (Methode 5): Rₜ = 1.68 min;

LC-MS (Methode 1): Rₜ = 1.51 min; MS (ESIpos): m/z = 1083 (M+H)⁺.

19 mg (0.015 mmol) dieses Intermediats wurden in 4 ml DMF gelöst. Nach Zugabe von 817 µl Piperidin wurde der Ansatz 5 min bei RT gerührt. Anschließend wurde im Vakuum eingeengt und der Rückstand zunächst mit Diethylether digeriert und anschließend mittels präparativer HPLC gereinigt (Eluent: Acetonitril + 0.1% TFA / 0.1 % aq. TFA). Die entsprechenden Fraktionen wurden vereinigt, das Lösungsmittel im Vakuum entfernt und der Rückstand dann aus Dioxan/Wasser lyophilisiert. Es wurden 12 mg (92% d. Th.) der Titelverbindung als farbloser Schaum erhalten.

HPLC (Methode 6): Rₜ = 2.0 min;

LC-MS (Methode 1): Rₜ = 0.94 min; MS (ESIpos): m/z = 861 (M+H)⁺.

### Beispiel 19

### N-Methyl-L-threonyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid

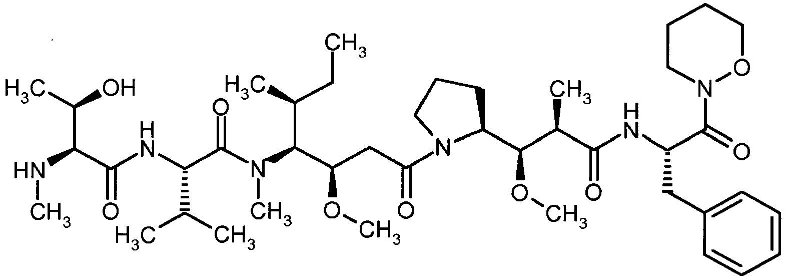

100 mg (0.181 mmol) *N*-[(Benzyloxy)carbonyl]-*N*-methyl-L-threonyl-*N*-[(2*R*,3*S*,4*S*)-1-carboxy-2-methoxy-4-methylhexan-3-yl]-*N*-methyl-L-valinamid (Intermediat 32) wurden in 5 ml DMF aufgenommen und nacheinander mit 94 mg (0.181 mmol) (2*R*,3*R*)-3-Methoxy-2-methyl-*N*-[(2*S*)-1-(1,2-oxazinan-2-yl)-1-oxo-3-phenylpropan-2-yl]-3-[(2*S*)-pyrrolidin-2-yl]propanamid Trifluoressigsäure-Salz (Intermediat 7), 42 mg (0.218 mmol) 1-(3-Dimethylaminopropyl)-3-ethylcarbodiimid-Hydrochlorid, 33 mg (0.218 mmol) 1-Hydroxy-1*H*-benzotriazol-Hydrat sowie 63 µl (0.36 mmol) *N,N*-Diisopropylethylamin versetzt. Die Mischung wurde anschließend 2 h bei RT gerührt. Das Lösungsmittel wurde danach im Vakuum entfernt und der Rückstand durch präparative HPLC gereinigt. Es wurden so 128 mg (75% d. Th.) des Z-geschützten Intermediats *N*-[(Benzyloxy)carbonyl]-*N*-methyl-L-threonyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxo-heptan-4-yl]-*N*-methyl-L-valinamid erhalten.

LC-MS (Methode 2): Rₜ = 1.32 min; MS (ESIpos): m/z = 937 (M+H)⁺.

100 mg (0.107 mmol) dieses Intermediats wurden in 20 ml Methanol gelöst und bei RT und Normaldruck 1 h lang über 10% Palladium/Kohle hydriert. Der Katalysator wurde anschließend abfiltriert und das Lösungsmittel abgedampft. Der verbliebene Rückstand wurde aus Dioxan/Wasser (1:1) lyophilisiert. Es wurden 88 mg (97% d. Th.) der Titelverbindung erhalten.

LC-MS (Methode 1): Rₜ = 0.83 min; MS (ESIpos): m/z = 803 (M+H)⁺.

### Beispiel 20

### N,N-Dimethyl-L-threonyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid

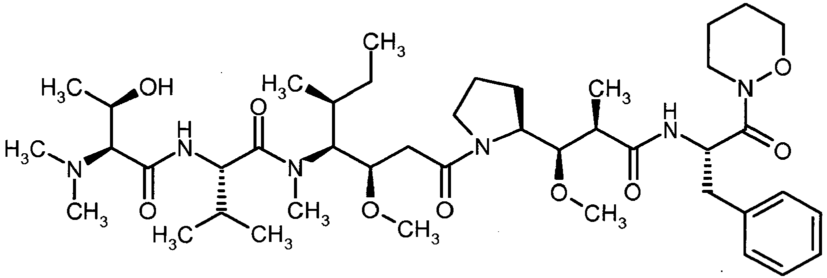

84 mg (0.105 mmol) *N*-Methyl-L-threonyl-*N*-[(3*R*,4*S*,5*S*)-3-methoxy-1-{(2*S*)-2-[(1*R*,2*R*)-1-meth-oxy-2-methyl-3- {[(2*S*)-1-(1,2-oxazinan-2-yl)-1-oxo-3-phenylpropan-2-yl]amino}-3-oxopropyl]-pyrrolidin-1-yl}-5-methyl-1-oxoheptan-4-yl]-*N*-methyl-L-valinamid (Beispiel 19) wurden in 5 ml Dioxan/Wasser (1:1) aufgenommen und mit 31 µl (0.418 mmol) einer 37%-igen wässrigen Formaldehyd-Lösung sowie 8 mg (0.126 mmol) Natriumcyanoborhydrid versetzt. Die Mischung wurde anschließend mit 1 ml 0.1 N Salzsäure auf pH 6-7 eingestellt und 1 h bei 100°C gerührt. Der Ansatz wurde danach in eine Mischung aus halbgesättigter wässriger Natriumhydrogencarbonat-Lösung und Ethylacetat gegossen. Die organische Phase wurde abgetrennt, mit gesättigter Natriumchlorid-Lösung gewaschen, über Magnesiumsulfat getrocknet, filtriert und eingeengt. Der Rückstand wurde schließlich aus Acetonitril/Wasser lyophilisiert. Es wurden so 72 mg (84% d. Th.) der Titelverbindung in Form eines farblosen Schaums erhalten.

HPLC (Methode 5): Rₜ = 1.6 min;

LC-MS (Methode 2): Rₜ = 0.96 min; MS (ESIpos): m/z = 817 (M+H)⁺.

### Beispiel 21

### N-Methyl-L-valyl-N-[(3R,4S,5S)-3-methoxy-1-{(2S)-2-[(1R,2R)-1-methoxy-2-methyl-3-{[(2R)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid Trifluoressigsäure-Salz

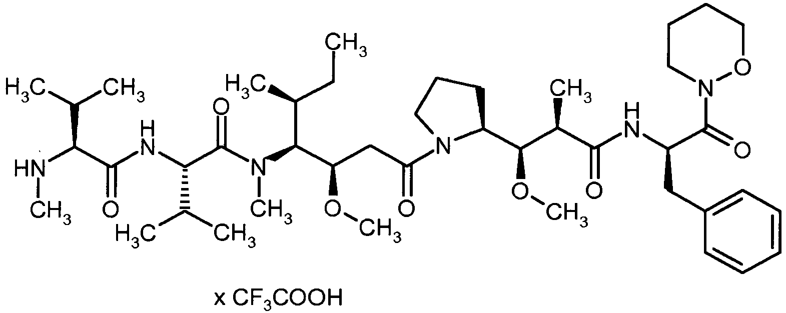

Die Titelverbindung wurde in Analogie zur Synthese von Beispiel 18 durch Kupplung von Intermediat 30 mit (2R)-2-Amino-1-(1,2-oxazinan-2-yl)-3-phenylpropan-1-on Trifluoressigsäure-Salz (Intermediat 33) und nachfolgende Entschützung mit Piperidin hergestellt. Die Reinigung des Endprodukts erfolgte durch präparative HPLC (Eluent: Acetonitril + 0.1% TFA / 0.1% aq. TFA).

HPLC (Methode 6): Rₜ = 1.9 min;

LC-MS (Methode 1): Rₜ = 0.91 min; MS (ESIpos): m/z = 801 (M+H)⁺

¹H-NMR (500 MHz, DMSO-d₆): δ = 8.8 (m, 2H), 8.65 (m, 1H), 8.32 und 8.1 (2d, 1H), 7.3-7.1 (m, 5H), 5.05 und 4.95 (2m, 1H), 4.65 (m, 1H), 4.62 und 4.56 (2t, 1H), 4.1-3.75 (m, 5H), 3.7-3.45 (m, 4H), 3.28, 3.22, 3.18, 3.17, 3.04 und 2.99 (6s, 9H), 2.9 und 2.75 (2m, 2H), 2.46 (m, 3H), 2.45-2.2 (m, 3H), 2.1-1.5 (m, 12H), 1.0-0.8 (m, 18H), 0.8 und 0.75 (2d, 3H) [weitere Signale unter H₂O-Peak verborgen].

### Beispiel 22

### N-Methyl-L-valyl-N-[(3R,4S,5S)-3-methoxy-1-{(2S)-2-[(1R,2R)-1-methoxy-2-methyl-3-{[(2S)-2-methyl-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid Trifluoressigsäure-Salz

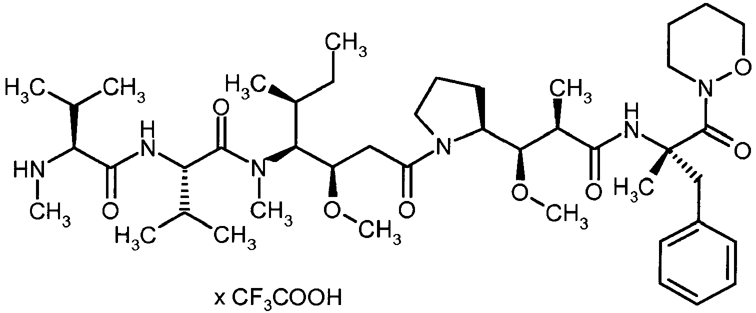

Die Titelverbindung wurde in Analogie zur Synthese von Beispiel 18 durch Kupplung von Intermediat 30 mit (2*S*)-2-Amino-2-methyl-1-(1,2-oxazinan-2-yl)-3-phenylpropan-1-on Trifluoressigsäure-Salz (Intermediat 34) und nachfolgende Entschützung mit Piperidin hergestellt. Die Reinigung des Endprodukts erfolgte durch präparative HPLC (Eluent: Acetonitril + 0.1% TFA / 0.1 % aq. TFA).

LC-MS (Methode 1): Rₜ = 0.95 min; MS (ESIpos): m/z = 815 (M+H)⁺.

### Beispiel 23

### N-(3-Carboxypropyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]-pyrrolidin-1-yl}-5-methyl-1-oxoheptan-4-yl]-N-methyl-L-valinamid

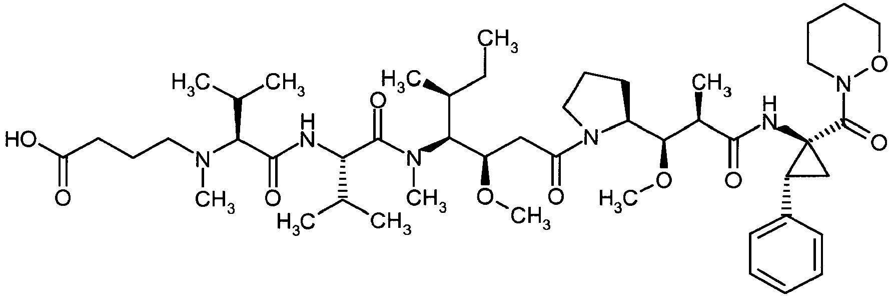

50 mg (0.054 mmol) *N*-Methyl-L-valyl-*N*-[(3*R*,4*R*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]-pyrrolidin-1-yl}-5-methyl-1-oxoheptan-4-yl]-*N*-methyl-L-valinamid Trifluoressigsäure-Salz (Beispiel 17) wurden in 8 ml Dioxan/Wasser (1:1) gelöst und mit 70 ml (0.108 mmol) einer 15%-igen Lösung von 4-Oxobutansäure in Wasser versetzt. Der Ansatz wurde anschließend 1 h bei 100°C gerührt. Nach Abkühlen auf RT wurden 3.7 mg (0.059 mmol) Natriumcyanoborhydrid zugegeben und die Mischung durch Zugabe von etwa 300 µl 0.1 N Salzsäure auf einen pH-Wert von 3 eingestellt. Der Ansatz wurde danach weitere 2 h bei 100°C gerührt. Nach Abkühlen wurden erneut 70 ml (0.108 mmol) der 15%-igen 4-Oxobutansäure-Lösung zugesetzt und der Ansatz wiederum 1 h bei 100°C gerührt. Dann wurden weitere 3.7 mg (0.059 mmol) Natriumcyanoborhydrid zugegeben und anschließend mit etwa 300 µl 0.1 N Salzsäure der pH-Wert wieder auf 3 eingestellt. Der Ansatz wurde danach erneut 2 h bei 100°C gerührt. Bei immer noch nicht vollständiger Umsetzung wurde diese Prozedur noch ein drittes Mal wiederholt. Der Ansatz wurde schließlich eingeengt und der Rückstand mittels präparativer HPLC gereinigt. Es wurden auf diese Weise 32 mg (65% d. Th.) der Titelverbindung in Form eines farblosen Schaums erhalten.

HPLC (Methode 6): Rₜ = 1.64 min;

LC-MS (Methode 9): Rₜ = 4.76 min; MS (ESIpos): m/z = 899 (M+H)⁺

¹H-NMR (500 MHz, DMSO-d₆): δ = 8.95 und 8.8 (2m, 1H), 8.88 und 8.65 (2s, 1H), 7.4-7.1 (m, 5H), 5.0, 4.78, 4.65 und 4.55 (4m, 2H), 4.1-3.7 (m, 5H), 3.32, 3.29, 3.20, 3.12, 3.1 und 3.0 (6s, 9H), 2.75 (m, 2H), 2.63 (t, 1H), 2.4-2.2 (m, 4H), 2.1-1.2 (m, 12H), 1.2-0.8 (m, 16H), 0.75 (m, 3H) [weitere Signale unter H₂O- und DMSO-Peaks verborgen].

### Beispiel 24

### N-(3-Carboxypropyl)-N-methyl-L-valyl-N-[(3R,4S,5S)-3-methoxy-1-(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid

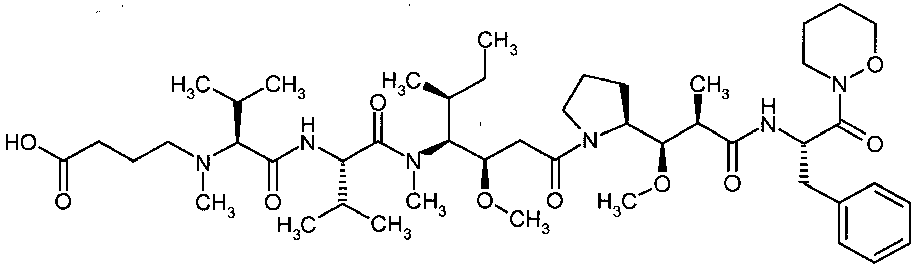

Die Titelverbindung wurde in Analogie zur Synthese von Beispiel 23 durch Umsetzung von 50 mg *N*-Methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid Trifluoressigsäure-Salz (Beispiel 1) mit 4-Oxobutansäure hergestellt.

Ausbeute: 34 mg (70% d. Th.)

HPLC (Methode 5): Rₜ = 1.64 min;

LC-MS (Methode 9): Rₜ = 4.77 min; MS (ESIpos): m/z = 887 (M+H)⁺.

### Beispiel 25

### N-(4-Carboxybenzyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]-pyrrolidin-1-yl}-5-methyl-1-oxoheptan-4-yl]-N-methyl-L-valinamid

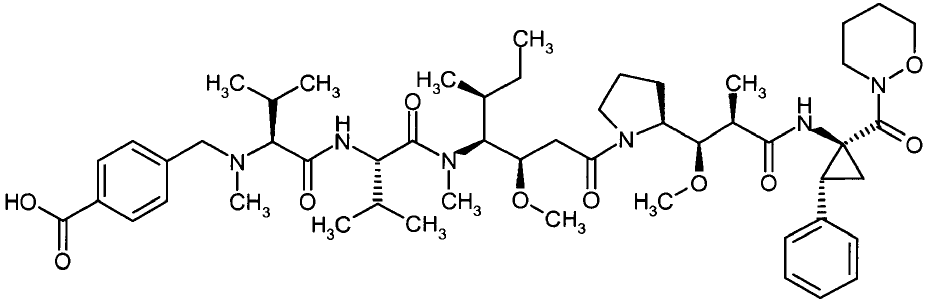

Die Titelverbindung wurde in Analogie zur Synthese von Beispiel 23 durch Umsetzung von 15 mg *N*-Methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]pyrrolidin-1-yl}-5-methyl-1-oxoheptan-4-yl]-*N*-methyl-L-valinamid Trifluoressigsäure-Salz (Beispiel 17) mit 4-Formylbenzoesäure hergestellt.

Ausbeute: 7.5 mg (48% d. Th.)

HPLC (Methode 5): Rₜ = 1.75 min;

LC-MS (Methode 1): Rₜ = 0.97 min; MS (ESIpos): m/z = 947 (M+H)⁺.

### Beispiel 26

### N-(5-Carboxypentyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]-pyrrolidin-1-yl}-5-methyl-1-oxoheptan-4-yl]-N-methyl-L-valinamid

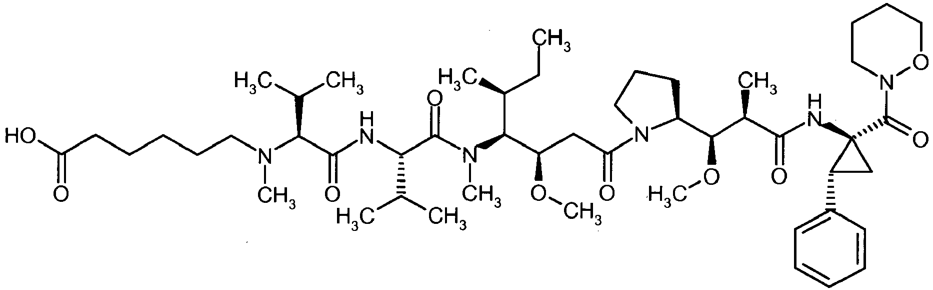

10 mg (0.011 mmol) *N*-Methyl-L-valyl-*N*-[(3*R*,4*S*,5*S*)-3-methoxy-1-{(2*S*)-2-[(1*R*,2*S*)-1-methoxy-2-methyl-3-{[(1*S*,2*R*)-1-(1,2-oxazinan-2-ylcarbonyl)-2-phenylcyclopropyl]amino}-3-oxopropyl]pyrrolidin-1-yl}-5-methyl-1-oxoheptan-4-yl]-*N*-methyl-L-valinamid Trifluoressigsäure-Salz (Beispiel 17) wurden in 2 ml Dioxan/Wasser (1:1) gelöst und mit 2.8 mg (0.022 mmol) 6-Oxohexansäure versetzt. Der Ansatz wurde anschließend 1 h bei 100°C gerührt. Nach Abkühlen auf RT wurden 0.75 mg (0.012 mmol) Natriumcyanoborhydrid zugegeben und die Mischung durch Zugabe von 0.1 N Salzsäure auf einen pH-Wert von 3 eingestellt. Der Ansatz wurde danach eine weitere Stunde bei 100°C gerührt. Nach Abkühlen wurden erneut 2.8 mg (0.022 mmol) 6-Oxohexansäure zugesetzt und der Ansatz wiederum 1 h bei 100°C gerührt. Es wurden weitere 0.75 mg (0.012 mmol) Natriumcyanoborhydrid zugegeben und anschließend mit 0.1 N Salzsäure der pH-Wert wieder auf 3 eingestellt. Der Ansatz wurde danach erneut 1 h bei 100°C gerührt. Diese Prozedur wurde dann noch ein drittes Mal wiederholt. Der Ansatz wurde schließlich eingeengt und das Rohprodukt mittels präparativer HPLC gereinigt. Es wurden so 6.4 mg (64% d. Th.) der Titelverbindung in Form eines farblosen Schaums erhalten.

HPLC (Methode 5): Rₜ = 1.68 min;

LC-MS (Methode 9): Rₜ = 4.86 min; MS (ESIpos): m/z = 927 (M+H)⁺.

### Beispiel 27

### N-(2-Aminoethyl)-N-methyl-L-valyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid Bis(trifluoressigsäure)-Salz

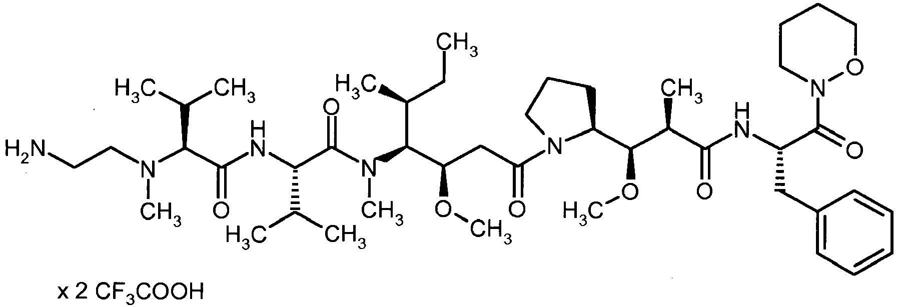

Die Titelverbindung wurde in Analogie zur Synthese von Beispiel 2 durch Umsetzung von 68 mg *N-*Methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxazinan-2-yl)-1-oxo-3-phenylpropan-2-yl]anmino}-3-oxopropyl]pyrrolidin-1-yl}-5-methyl-1-oxo-heptan-4-yl]-*N*-methyl-L-valinamid Trifluoressigsäure-Salz (Beispiel 1) mit *tert*.-Butyl-(2-oxo-ethyl)carbamat und nachfolgende Abspaltung der Boc-Schutzgruppe mit Trifluoressigsäure hergestellt.

Ausbeute: 49 mg (62% d. Th. über zwei Stufen)

HPLC (Methode 5): Rₜ = 1.58 min;

LC-MS (Methode 2): Rₜ = 1.05 min; MS (ESIpos): m/z = 844 (M+H)⁺

¹H-NMR (600 MHz, DMSO-d₆): δ = 8.25 (m, 1H), 8.45 und 8.15 (2d, 1H), 7.65-7.55 (m, 3H), 7.23-7.1 (m, 5H), 5.12 und 4.95 (2m, 1H), 4.72 und 4.62 (2m, 1H), 4.6 und 4.52 (2t, 1H), 4.2-3.8 (m, 4H), 3.7 (d, 1H), 3.23, 3.20, 3.19, 3.18, 3.03 und 2.98 (6s, 9H), 3.0-2.7 (m, 6H), 2.4-1.2 (m, 15H), 1.05, 1.0, 0.88 und 0.82 (4d, 6H), 0.92 (m, 6H), 0.73 (m, 6H) [weitere Signale unter H₂O-Peak verborgen].

### Beispiel 28

### N-(3-Aminopropyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]-pyrrolidin-1-yl}-5-methyl-1-oxoheptan-4-yl]-N-methyl-L-valinamid

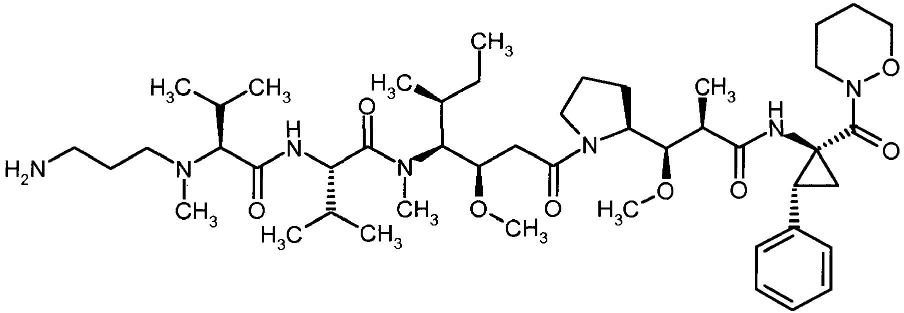

Die Titelverbindung wurde in Analogie zur Synthese von Beispiel 27 durch Umsetzung von 25 mg (0.027 mmol) *N*-Methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]pyrrolidin-1-yl}-5-methyl-1-oxoheptan-4-yl]-*N*-methyl-L-valinamid Trifluoressigsäure-Salz (Beispiel 17) mit Benzyl-(3-oxopropyl)carbamat und nachfolgende hydrogenolytische Abspaltung der Z-Schutzgruppe (mit 10% Palladium auf Kohle als Katalysator, in Ethanol als Lösungsmittel) hergestellt.

Ausbeute: 11 mg (41% d. Th. über zwei Stufen)

HPLC (Methode 5): Rₜ = 1.53 min;

LC-MS (Methode 1): Rₜ = 0.72 min; MS (ESIpos): m/z = 870 (M+H)⁺.

### Beispiel 29

### N-(4-Methoxy-4-oxobutyl)-N-methyl-L-valyl-N-[(3R,4S,5S)-3-methoxy-1-{(2S)-2-[(1R,2R)-1-meth-oxy-2-methyl-3-{[(2S)-1-(1,2-oxazinan-2-yl)-1-oxo-3-phenylpropan-2-yl]amino}-3-oxopropyl]-pyrrolidin-1-yl}-5-methyl-1-oxoheptan-4-yl]-N-methyl-L-valinamid

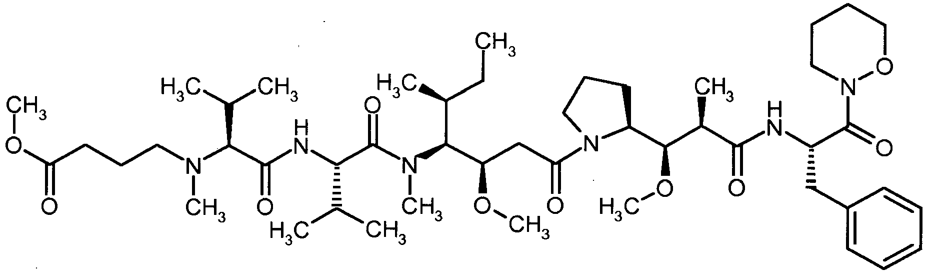

Die Titelverbindung wurde in Analogie zur Synthese von Beispiel 23 durch Umsetzung von 9.5 mg *N*-Methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid Trifluoressigsäure-Salz (Beispiel 1) mit Methyl-4-oxo-butanoat hergestellt.

Ausbeute: 4 mg (43% d. Th.)

HPLC (Methode 5): Rₜ = 1.73 min;

LC-MS (Methode 9): Rₜ = 4.91 min; MS (ESIpos): m/z = 901 (M+H)⁺.

### Beispiel 30

### N-(6-Aminohexyl)-N-methyl-L-valyl-N-[(3R,4S,5S)-3-methoxy-1-{(2S)-2-[(1R,2R)-1-methoxy-2-methyl-3-{[(2S)-1-(1,2-oxazinan-2-yl)-1-oxo-3-phenylpropan-2-yl]amino}-3-oxopropyl]pyrrolidin-1-yl}-5-methyl-1-oxoheptan-4-yl]-N-methyl-L-valinamid

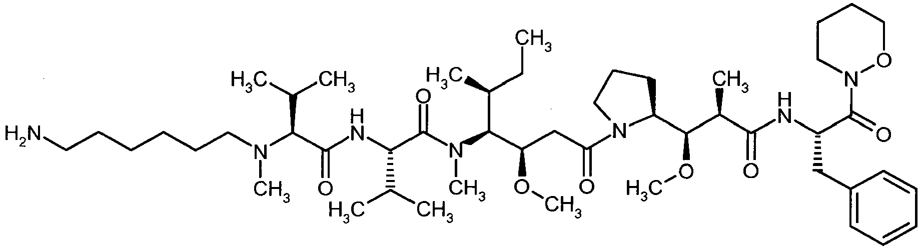

Die Titelverbindung wurde in Analogie zur Synthese von Beispiel 28 durch Umsetzung von 20 mg (16 µmol) *N*-Methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(1,2-oxazinan-2-yl)-1-oxo-3-phenylpropan-2-yl]amino}-3-oxopropyl]pyrrolidin-1-yl}-5-methyl-1-oxoheptan-4-yl]-*N*-methyl-L-valinamid Trifluoressigsäure-Salz (Beispiel 1) mit Benzyl-(6-oxohexyl)carbamat und nachfolgende hydrogenolytische Abspaltung der Z-Schutzgruppe (mit 10% Palladium auf Kohle als Katalysator, in Methanol als Lösungsmittel) hergestellt.

Ausbeute: 7.6 mg (55% d. Th. über zwei Stufen)

HPLC (Methode 6): Rₜ = 1.8 min;

LC-MS (Methode 1): Rₜ = 0.70 min; MS (ESIpos): m/z = 901 (M+H)⁺.

### Beispiel 31

### N-(6-Aminohexyl)-N-methyl-L-valyl-N-[(3R,4S,5S)-3-methoxy-1-{(2S)-2-[(1R,2R)-1-methoxy-2-methyl-3-{[(1S,2R)-1-(1,2-oxazinan-2-ylcarbonyl)-2-phenylcyclopropyl]amino}-3-oxopropyl]-pyrrolidin-1-yl}-5-methyl-1-oxoheptan-4-yl]-N-methyl-L-valinamid

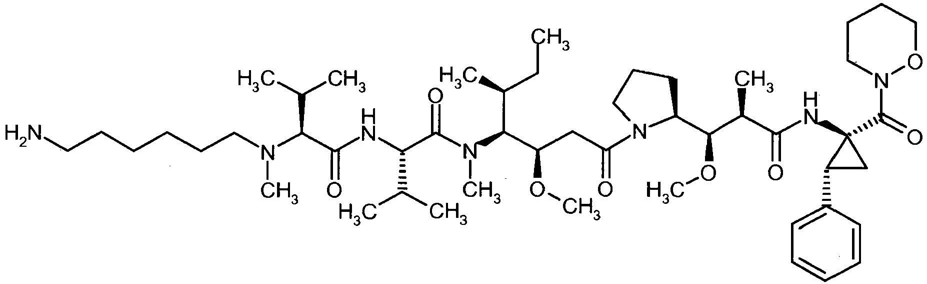

Die Titelverbindung wurde in Analogie zur Synthese von Beispiel 28 durch Umsetzung von 200 mg (0.108 mmol) *N*-Methyl-L-valyl-*N*-[(3*R*,4*S*,5*S*)-3-methoxy-1-{(2*S*)-2-[(1*R*,2*R*)-1-methoxy-2-methyl-3-{[(1*S*,2*R*)-1-(1,2-oxazinan-2-ylcarbonyl)-2-phenylcyclopropyl]amino}-3-oxopropyl]pyrrolidin-1-yl}-5-methyl-1-oxoheptan-4-yl]-*N*-methyl-L-valinamid Trifluoressigsäure-Salz (Beispiel 17) mit Benzyl-(6-oxohexyl)carbamat und nachfolgende hydrogenolytische Abspaltung der Z-Schutzgruppe (mit 5% Palladium auf Kohle als Katalysator, in Methanol als Lösungsmittel) hergestellt.

Ausbeute: 69 mg (65% d. Th. über zwei Stufen)

HPLC (Methode 5): Rₜ = 1.7 min;

LC-MS (Methode 1): Rₜ = 0.76 min; MS (ESIpos): m/z = 912 (M+H)⁺.

### B. Bewertung der biologischen Wirksamkeit

Die biologische Aktivität der erfindungsgemäßen Verbindungen kann durch *in vitro*- und *in vivo-*Untersuchungen, wie sie dem Fachmann bekannt sind, nachgewiesen werden. Beispielsweise können die pharmakologischen und pharmakokinetischen Eigenschaften der erfindungsgemäßen Verbindungen mit Hilfe der nachstehend beschriebenen Assays bestimmt werden:

### B-1. Bestimmung der antiproliferativen Wirkung an der 786-O RCC-Zelllinie:

Eine definierte Zellzahl der humanen renalen Krebszelllinie 786-O wurde in einer 96-well-Mikrotiterplatte im Vollmedium ausgesät (2 500 oder 7 000 Zellen/well) und über Nacht bei 37°C / 5% CO₂ inkubiert. Nach 18 h wurde das Aussaatmedium durch serumfreies Medium oder Medium mit 2% FCS ersetzt. Die Behandlung startete mit Zugabe der jeweiligen Testsubstanz in variierender Konzentration (10⁻⁵ M bis 10⁻¹⁴ M). Es wurden Inkubationszeiten von 48 h bis 96 h gewählt. Die Proliferation wurde mit Hilfe des MTT-Assays (ATCC, Manassas, Virginia, USA, Katalog-Nr. 30-1010K) bestimmt. Nach Ablauf der Inkubationszeit wurde das MTT-Reagens für 4 h mit den Zellen inkubiert, bevor durch Zugabe des Detergens die Lyse der Zellen über Nacht erfolgte. Die Detektion des gebildeten Farbstoffs erfolgte bei 570 nm. Die Proliferation nicht mit Testsubstanz, aber ansonsten identisch behandelter Zellen wurde als 100%-Wert definiert. Die aus diesem Test gewonnenen Daten repräsentieren Dreifachbestimmungen, und es wurden mindestens zwei unabhängige Experimente durchgeführt.

In der folgenden Tabelle 1 sind die IC₅₀-Werte repräsentativer Ausführungsbeispiele aus diesem Assay aufgeführt:

**Tabelle 1**

| **Ausführungsbeispiel** | **IC₅₀ [nM]** |
|---|---|
| 1 | 4 |
| 4 | 14 |
| 6 | 0.5 |
| 8 | 2.4 |
| 9 | 3 |
| 11 | 1.2 |
| 12 | 0.2 |
| 13 | 4 |
| 16 | 8 |
| 17 | 4 |
| 23 | 11 |
| 24 | 5 |
| 26 | 18 |

Im Vergleich hierzu weist Monomethylauristatin F (MMAF) in diesem Test einen IC₅₀-Wert von 260 nM auf.

### B-2. Bestimmung der antiproliferativen Wirkung an der HT29wt-Zelllinie:

Eine definierte Zellzahl der humanen Colonkarzinom-Zelllinie HT29wt (Wildtyp) wurde in einer 96-well-Mikrotiterplatte im Vollmedium (10% FCS-RPMI) ausgesät (2 500 Zellen/well) und über Nacht bei 37°C / 5% CO₂ inkubiert. Nach 18 h wurde das Aussaatmedium durch frisches Medium mit 10% FCS ersetzt. Die Behandlung startete mit Zugabe der jeweiligen Testsubstanz. Von den zu untersuchenden Substanzen wurden Dosis-Wirkungskurven in einem Konzentrationsbereich von 10⁻⁵ M bis 10⁻¹⁴M (1:10-Verdünnungsreihe) bestimmt. Es wurden Inkubationszeiten von 48 h bis 96 h gewählt. Die Detektion der Proliferation erfolgte mit Hilfe des MTT-Assays (ATCC, Manassas, Virginia, USA, Katalog-Nr. 30-1010K). Nach Ablauf der gewählten Inkubationszeit wurde das MTT-Reagens für 4 h mit den Zellen inkubiert, bevor durch Zugabe des Detergens die Lyse der Zellen über Nacht erfolgte. Die Detektion des gebildeten Farbstoffs erfolgte bei 570 nm. Die Proliferation nicht mit Testsubstanz, aber ansonsten identisch behandelter Zellen wurde als 100%-Wert definiert. Die aus diesem Test gewonnenen Daten repräsentieren Dreifachbestimmungen, und es wurden mindestens zwei unabhängige Experimente durchgeführt.

In der folgenden Tabelle 2 sind die IC₅₀-Werte repräsentativer Ausführungsbeispiele aus diesem Assay aufgeführt:

**Tabelle 2**

| **Ausführungsbeispiel** | **IC₅₀ [nM]** |
|---|---|
| 1 | 0.1 |
| 2 | 0.5 |
| 4 | 0.2 |
| 9 | 1.4 |
| 17 | 0.5 |
| 18 | 1.2 |
| 23 | 0.7 |
| 24 | 1.5 |
| 29 | 0.1 |

Im Vergleich hierzu weist Monomethylauristatin F (MMAF) in diesem Test einen IC₅₀-Wert von 10 nM auf.

### B-3. Bestimmung des Einflusses auf die Tubulinpolymerisation:

Krebszellen sind entartete Zellen, die häufig auch durch eine gesteigerte Zellteilung zu einer Tumorbildung führen. Microtubuli bilden die Spindelfasern des Spindelapparates und sind ein essentieller Bestandteil des Zellzyklus. Der geregelte Auf- und Abbau von Microtubuli ermöglicht die genaue Aufteilung der Chromosomen auf die Tochterzellen und stellt einen kontinuierlich dynamischen Prozess dar. Eine Störung dieser Dynamik führt zu einer fehlerhaften Zellteilung und letztlich zum Zelltod. Die gesteigerte Zellteilung von Krebszellen macht diese jedoch auch besonders empfänglich gegenüber Spindelfasergiften, die einen festen Bestandteil der Chemotherapie darstellen. Spindelfasergifte wie Paclitaxel oder Epothilon führen zu einer stark erhöhten Polymerisationsgeschwindigkeit der Microtubuli, während Vinca-Alkaloide oder auch Monomethylauristatin E (MMAE) zu einer stark reduzierten Polymerisationsgeschwindigkeit der Microtubuli führen. In beiden Fällen ist die notwendige Dynamik des Zellzyklus empfindlich gestört. Die im Rahmen der vorliegenden Erfindung untersuchten Verbindungen führen zu einer reduzierten Polymerisationsgeschwindigkeit der Microtubuli.

Zur Untersuchung der Tubulinpolymerisation wurde das "Fluorescence-based Microtubule Polymerisation Assay Kit" der Firma Cytoskeleton (Denver, Colorado, USA; Bestellnummer: BK011) verwendet. Bei diesem Assay wird unpolymerisiertem Tubulin GTP zugesetzt, womit die Polymerisation spontan stattfinden kann. Der Assay beruht auf der Bindung des Fluorophors 4',6-Diamidino-2-phenylindol (DAPI) an Tubulin. Freies und gebundenes DAPI können aufgrund unterschiedlicher Emissionsspektra differenziert werden. Da DAPI eine deutlich höhere Affinität gegenüber polymerisiertem im Vergleich zu nicht-polymerisiertem Tubulin zeigt, läßt sich die Tubulinpolymerisation über die Zunahme der Fluoreszenz gebundener DAPI-Fluorophore verfolgen.

Zur Durchführung dieses Assays wurden die in DMSO gelösten Testsubstanzen von ihrer Ausgangskonzentration von 10 mM auf 1 µM in Wasser verdünnt. Neben der Puffer-Kontrolle wurde als Assay-Kontrolle auch polymerisationssteigernd Paclitaxel und andererseits polymerisationshemmend Vinblastin mitgeführt. Für die Messung wurden 96-well-Lochplatten mit halber Bodenfläche verwendet. Die Kinetik der Tubulinpolymerisation wurde 1 h lang bei 37°C in einem Fluorimeter verfolgt. Die Anregungswellenlänge betrug 355 nm, die Emission wurde bei 460 nm verfolgt. Für den Bereich des linearen Anstiegs innerhalb der ersten 10 Minuten wurde die Fluoreszenzänderung pro Minute (ΔF/min) berechnet, welche die Polymerisationsgeschwindigkeit der Microtubuli darstellt. Die Potenz der Testsubstanzen wurde anhand der jeweiligen Reduktion der Polymerisationsgeschwindigkeit quantifiziert.

### B-4. Bestimmung der Plasma-Stabilität in vitro:

### Methode A:

1 mg der jeweiligen Testsubstanz wurde in 0.5 ml Acetonitril/DMSO (9:1) gelöst. Von dieser Lösung wurden 20 µl abgenommen und zu 1 ml 37°C warmen Ratten- bzw. Humanplasma gegeben (Plasma von männlichen Wistar-Ratten mit Li-Heparin, Fa. Harlan & Winkelmann bzw. humanes Leukozyten-depletiertes Frischplasma aus Vollblutentnahme). Aus dieser kräftig geschüttelten Plasmalösung wurden sofort nach Zugabe der Probe (Anfangswert als Bezugsgröße) und dann nach 5, 10, 30, 60, 120, 180 und 240 Minuten sowie gegebenenfalls nach 24 Stunden jeweils 100 µl-Aliquote entnommen und in 300 µl Acetonitril gegeben. Die ausgefällten Plasmaproteine wurden für 10 Minuten bei 5000 U/min abzentrifugiert und 30 µl des Überstandes per HPLC auf seinen Gehalt an unveränderter Testsubstanz analysiert. Quantifiziert wurde über die Flächenprozente der entsprechenden Peaks.

### HPLC-Methode bei Rattenplasma:

Instrument: Agilent 1200 mit DAD, binärer Pumpe, Autosampler, Säulenofen und Thermostat; Säule: Kromasil 100 C18, 250 mm x 4 mm, 5 µm; Säulentemperatur: 45°C; Eluent A: 5 ml Perchlorsäure / L Wasser, Eluent B: Acetonitril; Gradient: 0-8 min 98% A, 2% B; 8-15 min 56% A, 44% B; 15-20 min 10% A, 90% B; 20-21 min 10% A, 90% B; 21-23 min 98% A, 2% B; 23-25 min 98% A, 2% B; Flussrate: 2 ml/min; UV-Detektion: 220 nm.

### HPLC-Methode bei Humanplasma:

Instrument: Agilent 1100 mit DAD, binärer Pumpe, Autosampler, Säulenofen und Thermostat; Säule: Kromasil 100 C18, 250 mm x 4 mm, 5 um; Säulentemperatur: 45°C; Eluent A: 5 ml Perchlorsäure / L Wasser, Eluent B: Acetonitril; Gradient: 0-3 min 98% A, 2% B; 3-10 min 65% A, 35% B; 10-15 min 40% A, 60% B; 15-21 min 10% A, 90% B; 21-22 min 10% A, 90% B; 22-24 min 98% A, 2% B; 24-26 min 98% A, 2% B; Flussrate: 2 ml/min; UV-Detektion: 220 nm.

### Methode B:

Die jeweilige Testsubstanz wurde in Ratten- bzw. Humanplasma bei 37°C über einen Zeitraum von 5 h unter leichtem Rühren inkubiert. Zu verschiedenen Zeitpunkten (0, 2, 5, 10, 20, 30, 60, 120, 180 und 300 Minuten) wurde jeweils ein 100 µl-Aliquot entnommen. Nach Zugabe von internem Standard (10 µl) wurden die Proteine durch Zugabe von 200 µl Acetonitril gefällt und das Gemisch in einer Eppendorf-Zentrifuge für 5 Minuten zentrifugiert. Nach Zugabe von 150 µl Ammoniumacetat-Puffer pH 3 zu 150 µl des Überstandes wurde der Gehalt an unveränderter Testsubstanz mittels LC/MSMS analysiert.

In der folgenden Tabelle 3 sind die aus diesen Daten ermittelten Halbwertszeiten (t_{1/2}) repräsentativer Ausführungsbeispiele in Rattenplasma aufgeführt:

**Tabelle 3**

| **Ausführungsbeispiel** | **t_{1/2} [h]** | **Methode** |
|---|---|---|
| 4 | 3 | B |
| 16 | 0.75 | A |
| 17 | >24 | A |
| 18 | >24 | A |
| 22 | 2 | A |
| 23 | >24 | A |

Im Vergleich hierzu weist der Methylester von Monomethylauristatin F (MMAF-OMe) in Rattenplasma einen t_{1/2}-Wert (nach Methode A) von < 1 min auf.

In Humanplasma wurde exemplarisch für die erfindungsgemäßen Verbindungen bei den Ausführungsbeispielen 1, 17 und 21 keinerlei Abbau nach 24 h festgestellt, während der Methylester von Monomethylauristatin F (MMAF-OMe) in diesem Zeitraum zu etwa 20% abgebaut wurde.

### B-5. Bestimmung der Zell-Permeabilität:

Die Zell-Permeabilität einer Substanz kann mittels *in vitro*-Testung in einem Flux-Assay unter Verwendung von Caco-2-Zellen untersucht werden [M.D. Troutman und D.R. Thakker, Pharm. Res. 20 (8), 1210-1224 (2003)]. Hierzu wurden die Zellen auf 24-Loch-Filterplatten für 15-16 Tage kultiviert. Zur Bestimmung der Permeation wurde die jeweilige Testsubstanz in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC (Agilent 1200, Böblingen, Deutschland) unter Verwendung von reverse phase-Säulen aufgetrennt. Das HPLC-System war über ein Turbo Ion Spray-Interface an ein Triple Quadropol-Massenspektrometer API 4000 (Applied Biosystems Applera, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)]. Eine Substanz wurde als aktiv transportiert klassifiziert, wenn das Verhältnis von Pₐₚₚ (B-A) zu Pₐₚₚ (A-B) >2 oder <0.5 war.

### B-6. Bestimmung der Substrateigenschaften für P-Glycoprotein (P-gp):

Viele Tumorzellen exprimieren Transportproteine für Wirkstoffe, was häufig mit einer Resistenzentwicklung gegenüber Cytostatika einhergeht. Substanzen, die keine Substrate von solchen Transportproteinen wie beispielsweise P-Glycoprotein (P-gp) oder BCRP sind, könnten somit ein verbessertes Wirkprofil aufzeigen.

Die Substrateigenschaften einer Substanz für P-gp (ABCB1) wurden mittels eines Flux-Assays unter Verwendung von LLC-PK1-Zellen, die P-gp überexprimieren (L-MDR1-Zellen), bestimmt [A.H. Schinkel et al., J. Clin. Invest. 96, 1698-1705 (1995)]. Hierzu wurden die LLC-PK1- oder L-MDR1-Zellen auf 96-Loch-Filterplatten für 3-4 Tage kultiviert. Zur Bestimmung der Permeation wurde die jeweilige Testsubstanz allein oder in Gegenwart eines Inhibitors (wie z.B. Ivermectin oder Verapamil) in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC unter Verwendung von reverse phase-Säulen aufgetrennt. Das HPLC-System war über ein Turbo Ion Spray-Interface an ein Triple Quadropol-Massenspektrometer API 3000 (Applied Biosystems Applera, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)]. Eine Substanz wurde als P-gp-Substrat klassifiziert, wenn das Efflux-Verhältnis Pₐₚₚ (B-A) zu Pₐₚₚ (A-B) > 2 war.

Als weitere Kriterien zur Bewertung der P-gp-Substrateigenschaften können die Efflux-Verhältnisse in L-MDR1- und LLC-PK1-Zellen oder das Efflux-Verhältnis in An- oder Abwesenheit eines Inhibitors miteinander verglichen werden. Wenn sich diese Werte um mehr als einen Faktor 2 unterscheiden, so handelt es sich bei der betreffenden Substanz um ein P-gp-Substrat.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfmdungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 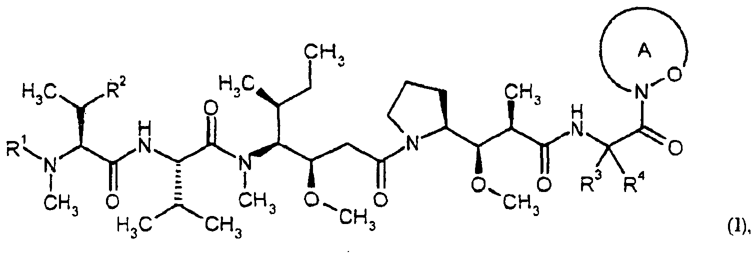 in welcher
R¹ für Wasserstoff, (C₁-C₆)-Alkyl oder eine Gruppe der Formel Q¹-L¹-* oder Q²-L²-* steht, worin
* die Verknüpfungsstelle mit dem Stickstoffatom kennzeichnet,
Q¹ Hydroxycarbonyl, (C₁-C₄)-Alkoxycarbonyl oder Benzyloxycarbonyl bedeutet,
L¹ geradkettiges (C₁-C₁₂-Alkandiyl bedeutet, das bis zu vierfach mit Methyl substituiert sein kann und in dem (*a*) zwei Kohlenstoffatome in 1,2-, 1.3-oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können oder *(b)* bis zu drei zueinander nicht benachbarte CH₂-Gruppen gegen -O- ausgetauscht sein können,
Q² Hydroxy, Amino oder Mono-(C₁-C₄)-alkylaznino bedeutet,
und
L² geradkettiges (C₂-C₁₂-Alkandiyl bedeutet, das bis zu vierfach mit Methyl substituiert sein kann und in dem (*a*) zwei Kohlenstoffatome in 1,2-, 1,3-oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können oder (*b*) bis zu drei zueinander nicht benachbarte CH₂-Gruppen gegen -O- ausgetauscht sein können,
R² für Methyl oder Hydroxy steht,
R³ für Wasserstoff oder Methyl steht,
R⁴ für Isopropyl, Isobutyl, *sec*.-Butyl, *tert*.-Butyl, Phenyl, Benzyl, 4-Hydroxybenzyl, 4-Hydroxy-3-nitrobenzyl, 1-Phenylethyl, Diphenylmethyl, 1*H*-Imidazol-4-ylmethyl oder 1*H*-Indol-3-ylmethyl steht,
oder
R³ und R⁴ zusammen mit dem Kohlenstoffatom, an das sie beide gebunden sind, eine 2-Phenylcyclopropan-1,1-diyl-Gruppe der Formel 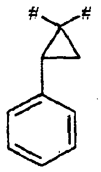 binden, worin
# die Verknüpfungsstellen mit den übrigen Teilen des Moleküls kennzeichnet,
und
der Ring A mit der darin enthaltenen N-O-Gruppierung für einen mono- oder bicyclischen, gegebenenfalls substituierten Heterocyclus der Formel 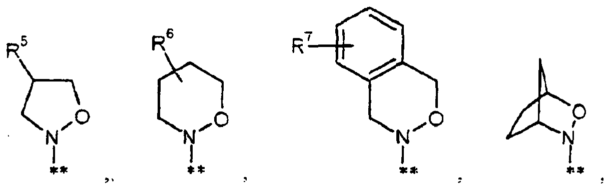 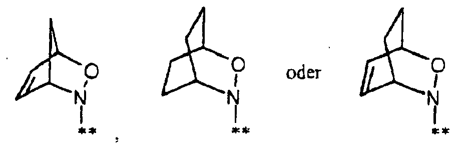 steht, worin
** die Verknüpfungsstelle mit der Carbonyl-Gruppe kennzeichnet,
R⁵ und R⁶ jeweils Wasserstoff, Hydroxy, (C₁-C₄)-Alkoxy oder Benzyloxy bedeuten,
und
R⁷ Wasserstoff, Fluor, Chlor, Cyano, Methyl oder Trifluormethyl bedeutet.
sowie ihre Salze, Solvate und Solvate der Salze.

2. Verbindung der Formel (I) nach Anspruch 1, in welcher
R¹ für Wasserstoff, (C₁-C₄)-Alkyl oder eine Gruppe der Formel Q¹-L¹-* oder Q²-L²-* steht, worin
* die Verknüpfungsstelle mit dem Stickstoffatom kennzeichnet,
Q¹ Hydroxycarbonyl oder (C₁-C₄)-Alkoxycarbonyl bedeutet,
L¹ geradkettiges (C₁-C₁₂)-Alkandiyl bedeutet, in dem (*a*) zwei Kohlenstoffatome in 1,3- oder 1,4-Relation zueinander unter Einbezug des einen beziehungsweise der beiden zwischen ihnen liegenden Kohlenstoffatome zu einem Phenyl-Ring verbrückt sein können oder (*b*) bis zu drei zueinander nicht benachbarte CH₂-Gruppen gegen -O- ausgetauscht sein können,
Q² Hydroxy, Amino oder Methylamino bedeutet,
und
L² geradkettiges (C₂-C₁₂)-Alkandiyl bedeutet, das ein- oder zweifach mit Methyl substituiert sein kann und in dem bis zu drei zueinander nicht benachbarte CH₂-Gruppen gegen -O- ausgetauscht sein können,
R² für Methyl oder Hydroxy steht,
R³ für Wasserstoff steht,
R⁴ für Benzyl, 4-Hydroxybenzyl, 1-Phenylethyl oder 1*H*-Indol-3-ylmetnyl steht,
oder R³ und R⁴ zusammen mit dem Kohlenstoffatom, an das sie beide gebunden sind, eine 2-Phenylcyclopropan-1,1-diyl-Gruppe der Formel 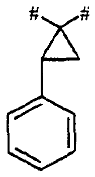 bilden, worin
# die Verknüpfungsstellen mit den übrigen Teilen des Moleküls kennzeichnet,
und
der Ring A mit der darin enthaltenen N-O-Gruppierung für einen mono- oder bicyclischen, gegebenenfalls substituierten Heterocyclus der Formel 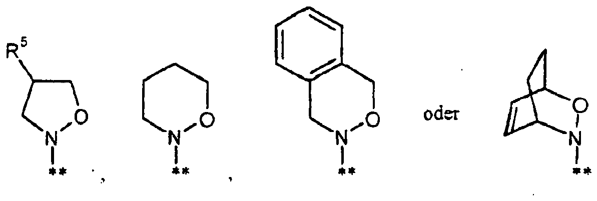 steht, worin
** die Verknüpfungsstelle mit der Carbonyl-Gruppe kennzeichnet,
und
R⁵ Wasserstoff, Hydroxy oder Benzyloxy bedeutet,
sowie ihre Salze, Solvate und Solvate der Salze.

3. Verbindung der Formel (I) nach Anspruch 1 oder 2, in welcher
R¹ für Wasserstoff, Methyl oder eine Gruppe der Formel Q¹-L¹-* oder Q²-L²-* steht, worin
* die Verknüpfungsstelle mit dem Stickstoffatom kennzeichnet,
Q¹ Hydroxycarbonyl, Methoxycarbonyl oder Ethoxycarbonyl bedeutet,
L¹ geradkettiges (C₁-C₆)-Alkandiyl bedeutet, in dem zwei Kohlenstoffatome in 1,4-relation zueinander unter Einbezug der beiden zwischen ihnen liegenden Kohlenstoffatome zu einem Phenyl-Ring verbrückt sein können,
Q² Hydroxy oder Amino bedeutet,
und
L³ geradkettiges (C₂-C₆)-Alkandiyl bedeutet,
R² für Methyl steht,
R³ für Wasserstoff steht,
R⁴ für Benzyl, 1-Phenylethyl oder 1*H*-Indol-3-ylmethyl steht,
oder
R³ und R⁴ zusammen mit dem Kohlenstoffatom, an das sie beide gebunden sind, eine (1*S*,2*R*)-2-Phenylcyclopropan-1,1-diyl-Gruppe der Formel 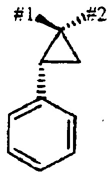 bilden, worin
#1 die Verknüpfungsstelle mit dem angrenzenden Stickstoffatom
und
#2 die Verknüpfunsstelle mit der Carbonyl-Gruppe kennzeichnen,
und
der Ring A mit der darin enthaltenen N-O-Gruppierung für einen mono- oder bicyclischen, gegebenenfalls substituierten Heterocyclus der Formel 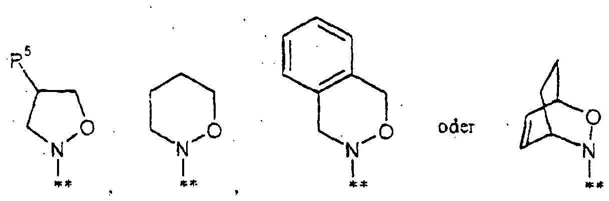 steht, worin
** die Verknüpfungsstelle mit der Carbonyl-Gruppe kennzeichnet,
und
R⁵ Wasserstoff, Hydroxy oder Benzyloxy bedeutet,
sowie thre Salze, Solvate und Solvate der Salze.

4. Verbindung gemäß Anspruch 1, 2 oder 3 mit der Formel (1-A) 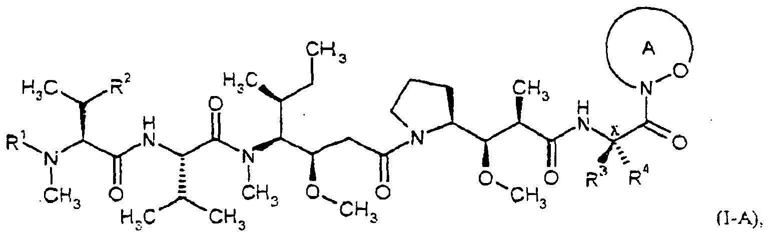 in weicher R¹, R², R³, R⁴ und der Ring A die in Anspruch 1, 2 oder 3 definierten Bedeutungen haben und das die Reste R³ und R⁴ tragende C^{x}-Kohlenstoffatom die abgebildete *S*-Konfiguration aufweist,
sowie ihre Salze, Solvate und Solvate der Salze.

5. The compound of claim 1, wherein said compound is selected from the group consisting of: 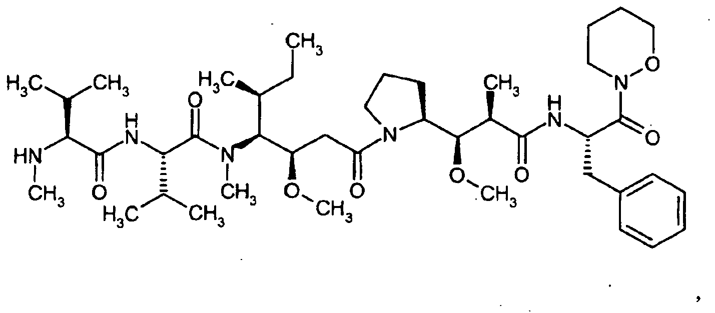 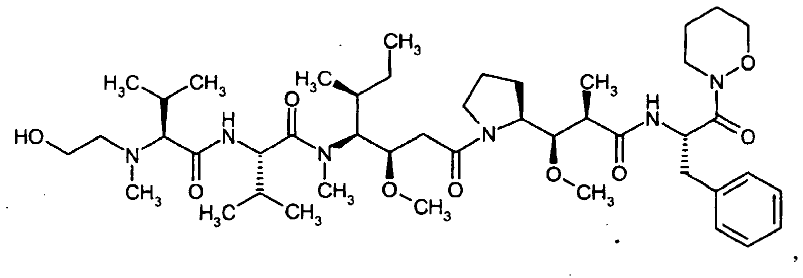 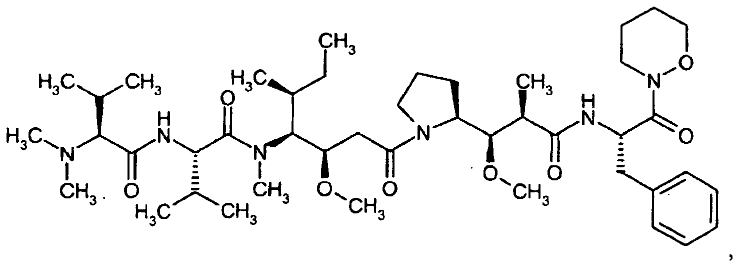 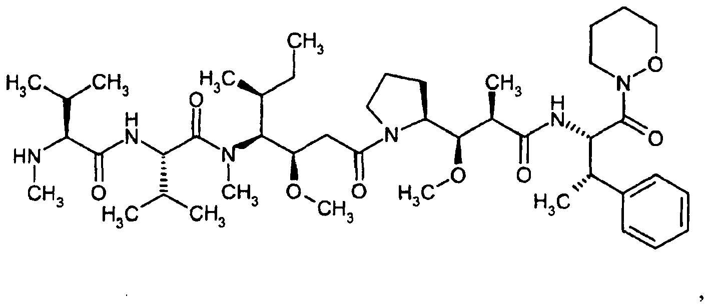 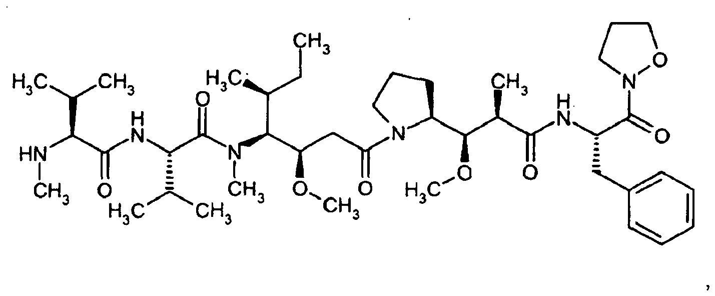 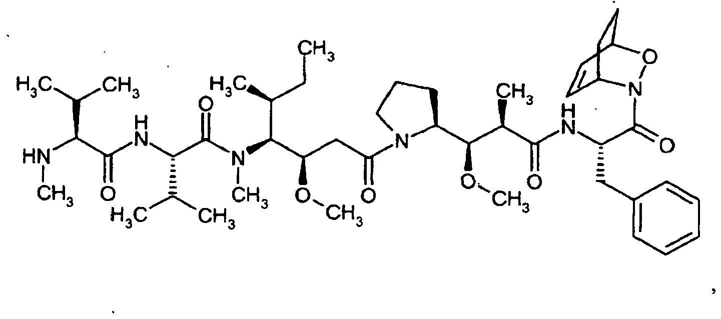 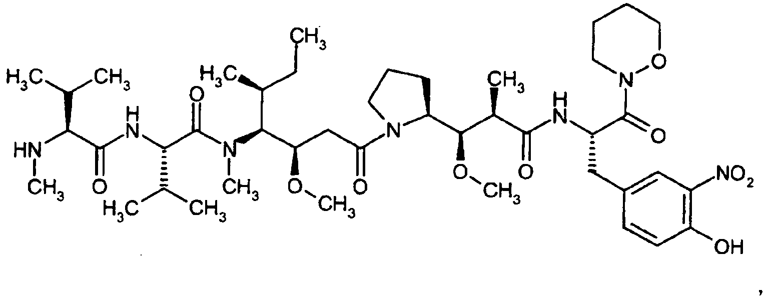 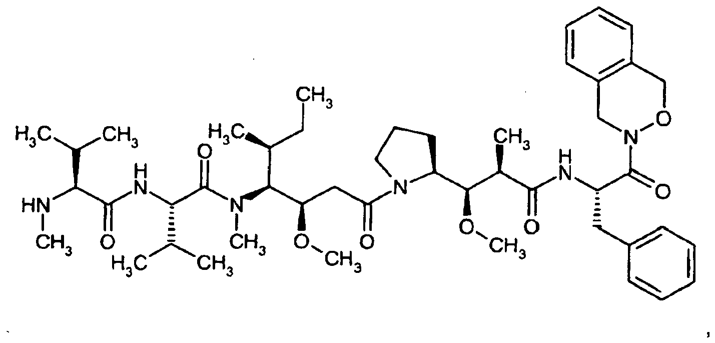 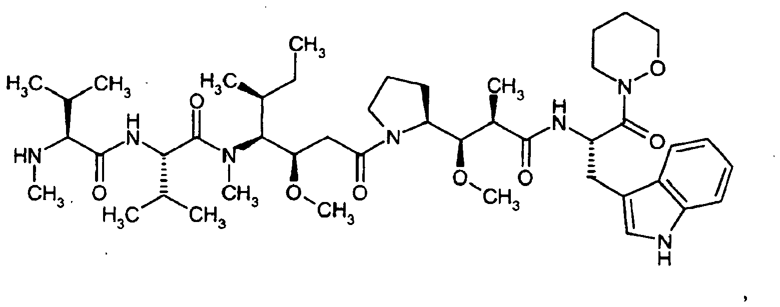 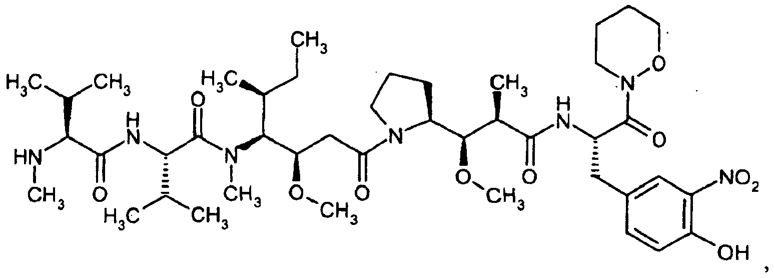 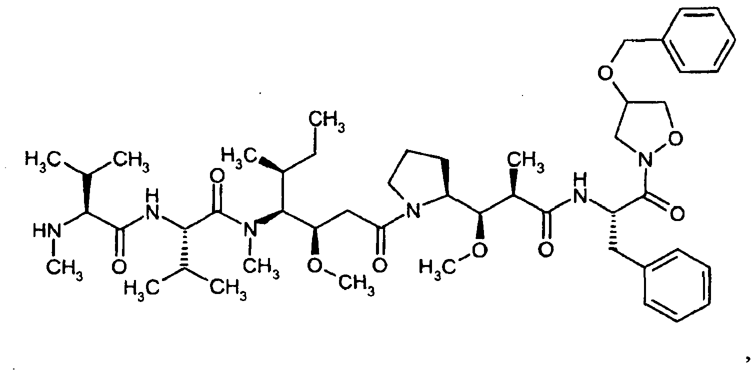 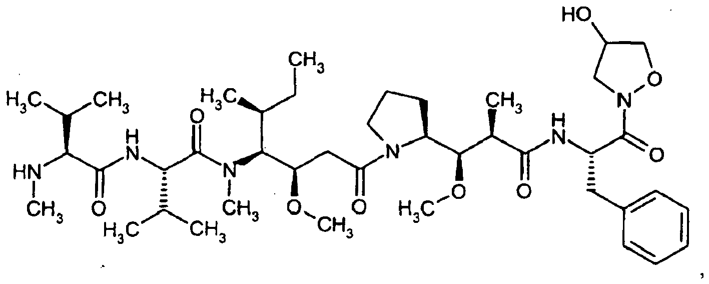 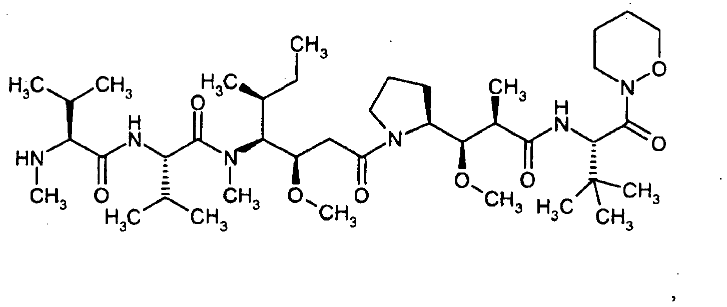 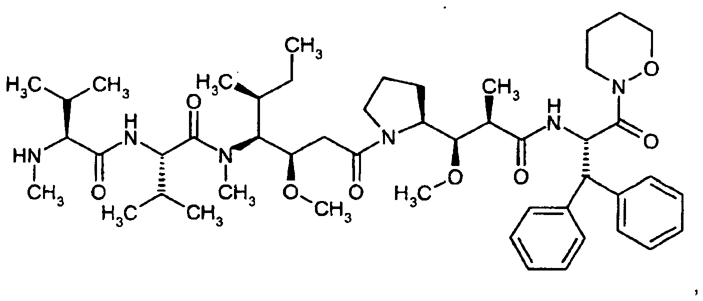 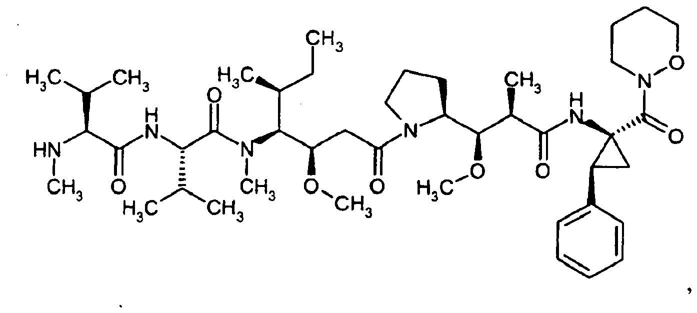 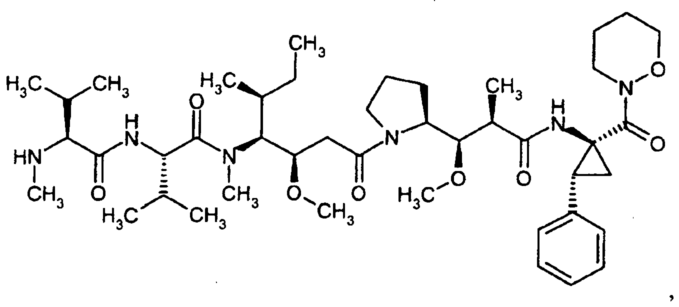 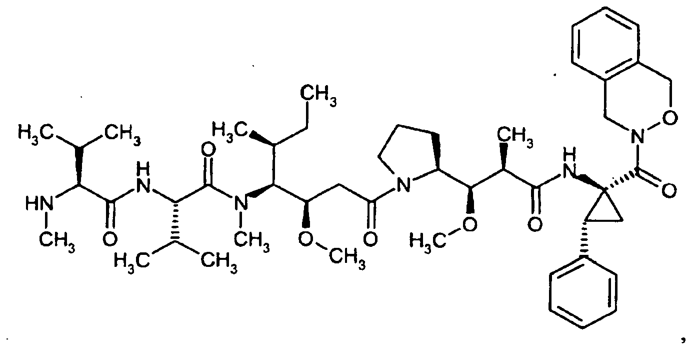 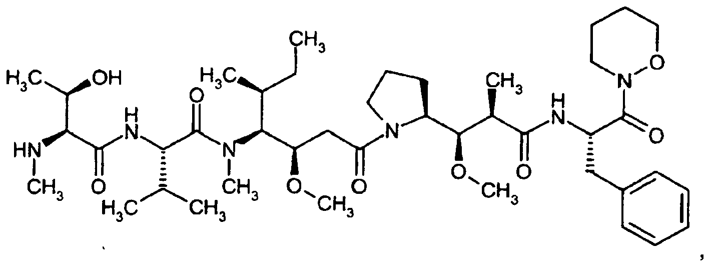 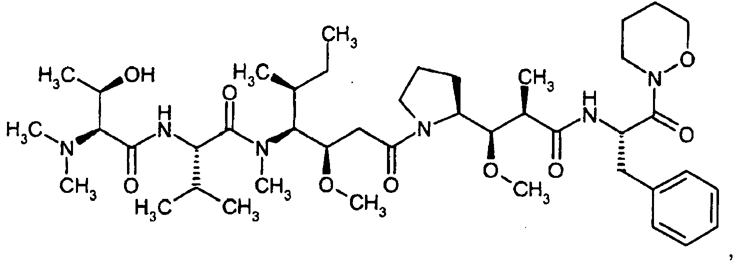 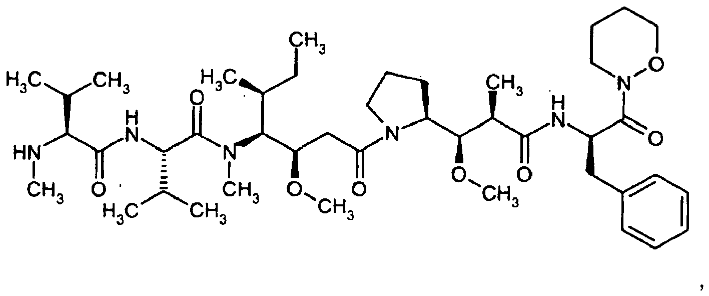 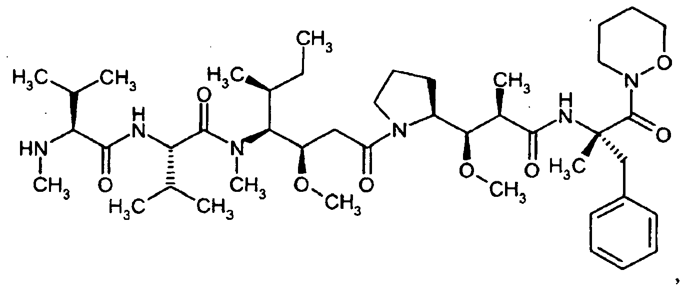 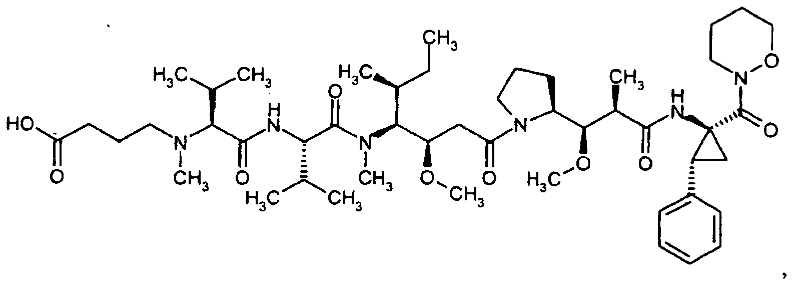 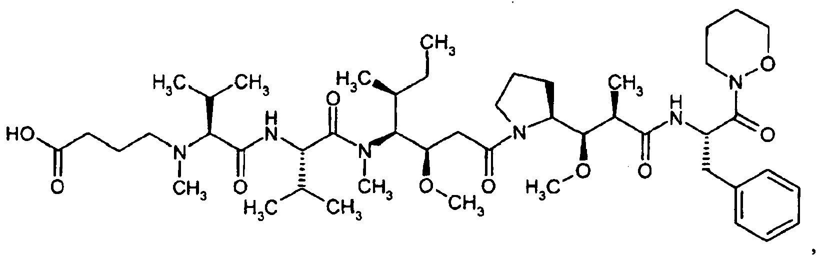 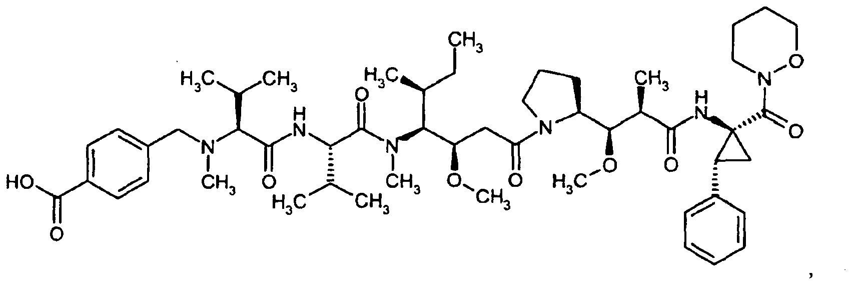 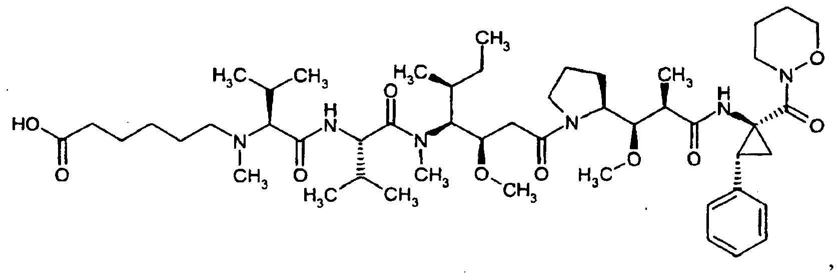 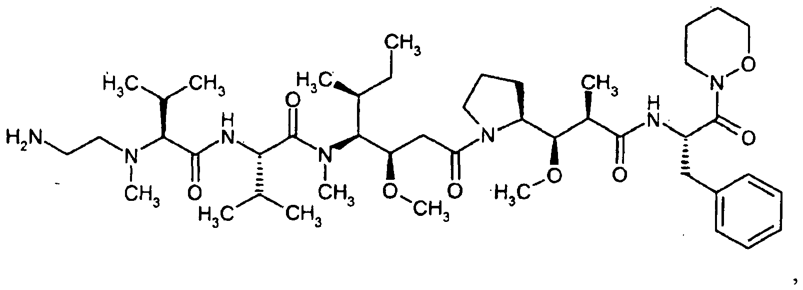 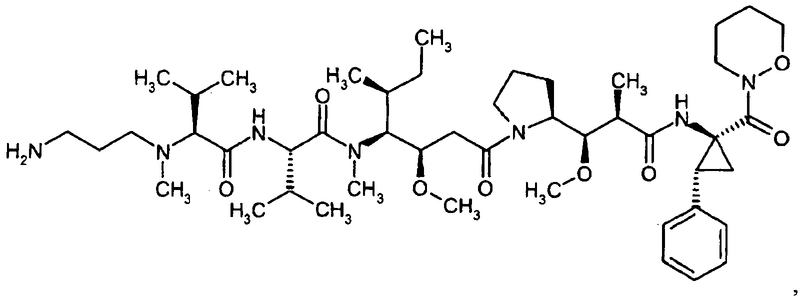 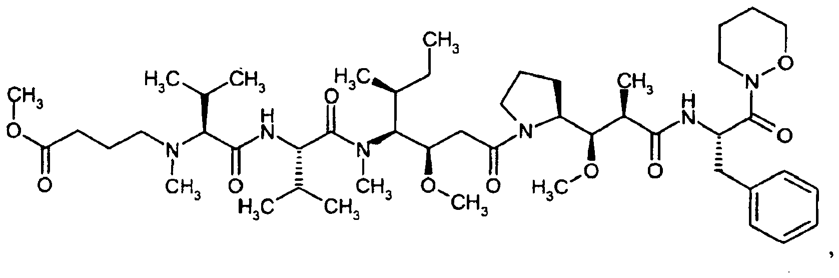 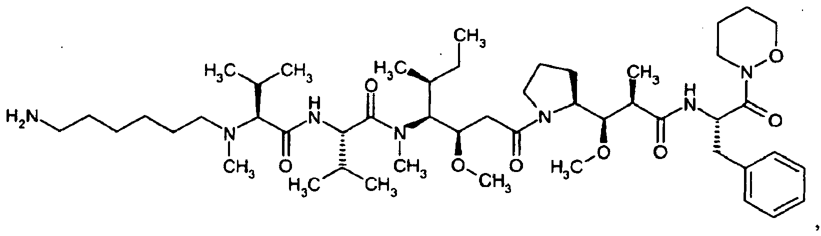 and

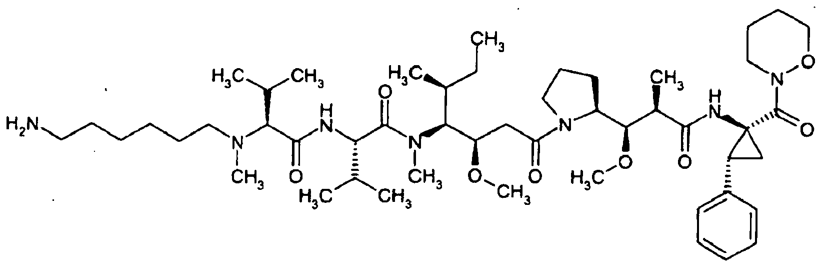

6. Verfahren zur Herstellung einer Verbindung der Formel (I), wie in den Ansprüchen 1 bis 4 definiert, **dadurch gekennzeichnet, dass** man eine Verbindung der Formel (II) 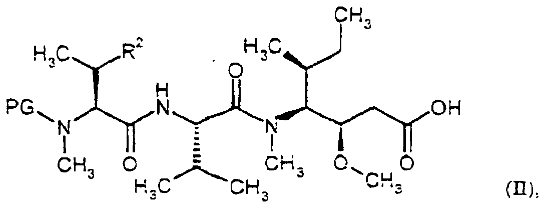 in welcher R² die in den Ansprüchen 1 bis 4 angegebene Bedeutung hat
und
PG für eine Amino-Schutzgruppe wie beispielsweise (9*H*-Fluoren-9-ylmethoxy)carbonyl, *tert*.-Butoxycarbonyl oder Benzyloxycarbonyl steht,
in einem inerten Lösungsmittel unter Aktivierung der Carboxyl-Funkrion in (II) entweder
[A] zunächst mit der Verbindung der Formel (III) 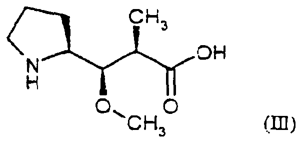 oder einem Salz hiervon zu einer Verbindung der Formel (IV) 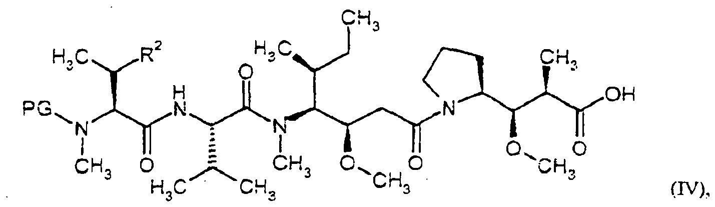 in welcher R² und PG die oben angegebenen Bedeutungen haben
kuppelt und diese anschließend in einem inerten Lösungsmittel unter Aktivierung der Carboxyl-Funktion mit einer Verbindung der Formel (V) 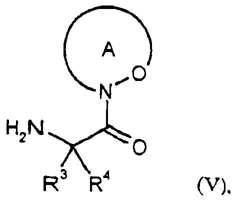 in welcher R³, R⁴ und der Ring A die in den Ansprüchen 1 bis 4 angegebenen Bedeutungen haben,
oder einem Salz dieser Verbindung zu einer Verbindung der Formel (VI) 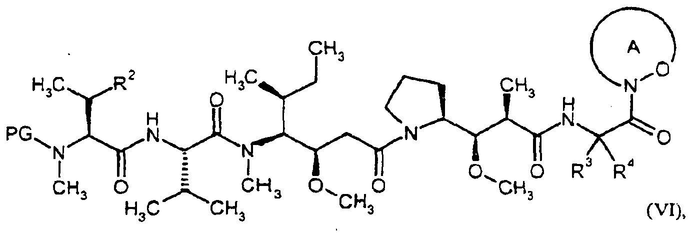 in welcher R², R\ R⁴, der Ring A und PG die oben angegebenen Bedeutungen haben,
kuppelt
oder
[B] mit einer Verbindung der Formel (VII) 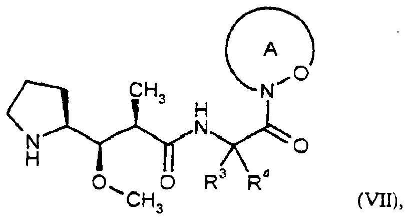 in welcher R³, R⁴ und der Ring A die in den Ansprüchen 1 bis 4 angegebenen Bedeutungen haben,
oder einem Salz dieser Verbindung gleichfalls zu der Verbindung der Formel (VI) 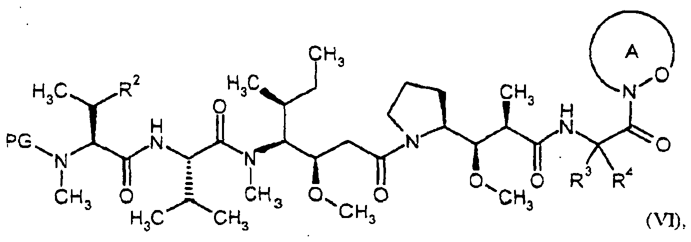 in welcher R², R³, R⁴, der Ring A und PG die oben angegebenen Bedeutungen haben,
kuppelt
und die jeweils resultierende Verbindung der Formel (VI) dann nach üblichen Methoden der Peptidchemie zu einer erfindungsgemäßen Verbindung der Formel (1-B) 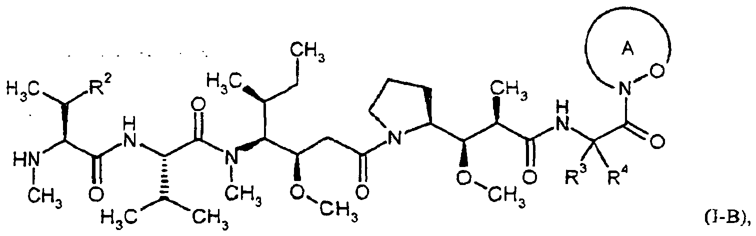 in welcher R², R³, R⁴ und der Ring A die oben angegebenen Bedeutungen haben,
entschützt und diese nachfolgend, falls gewünscht, entweder (*i*) durch baseninduzierte Alkylierung mit einer Verbindung der Formel (VIII)
R^{1A}-X (VIII),
in welcher
R^{1A} die in den Ansprüchen 1 bis 4 angegebene Bedeutung von R¹ hat, jedoch nicht für Wasserstoff steht,
und
X für eine Fluchtgruppe wie beispielsweise Chlorid, Bromid, Iodid, Mesylat, Triflat oder Tosylat steht,
in eine erfindungsgemäße Verbindung der Formel (I-C) 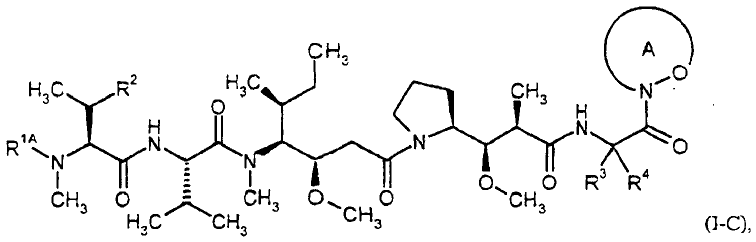 in welcher R^{1A}, R², R³, R⁴ und der Ring A die oben angegebene Bedeutungen haben,
überführt oder (*ii*) durch Umsetzung mit einer Verbindung der Formel (IX) 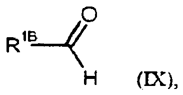 in welcher
R¹⁸ die oben angegebene, jedoch in der Alkyl-Kettenlänge um eine CH₂-Einheit verkürzte Bedeutung von R^{1A} hat,
in Gegenwart eines geeigneten Reduktionsmittels in eine erfindungsgemäße Verbindung der Formel (I-D) 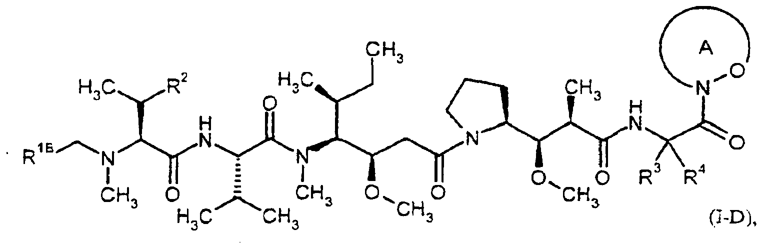 in welcher R^{1B}, R², R³, R⁴ und der Ring A die oben angegebenen Bedeutungen haben, überführt
und die so erhaltenen Verbindungen der Formeln (I-B), (I-C) bzw. (I-D) gegebenenfalls in ihre Enantiomere und/oder Diastereomere trennt und/oder mit den entsprechenden (i) Lösungsmitteln und/oder (*ii*) Basen oder Säuren zu ihren Solvaten, Salzen und/oder Solvaten der Salze umsetzt.

7. Verbindung, wie in einem der Ansprüche 1 bis 5 definiert, zur Verwendung in einem Verfahren zur Behandlung und/oder Prävention von Krankheiten.

8. Verbindung, wie in einem der Ansprüche 1 bis 5 definiert, zur Verwendung in einem Verfahren zur Behandlung und/oder Prävention von Krebs- und Tumorerkrankungen.

9. Verwendung einer Verbindung, wie in einem der Ansprüche 1 bis 5 definiert, zur Herstellung eines Arzneimittels zur Behandlung und/oder Prävention von Krebs- und Tumorerkrankungen.

10. Arzneimittel enthaltend eine Verbindung, wie in einem der Ansprüche 1 bis 5 definiert, in Kombination mit einem oder mehreren inerten, nicht-toxischen, pharmazeutisch geeigneten Hilfsstoffen.

11. Arzneimittel enthaltend eine Verbindung, wie in einem der Ansprüche 1 bis 5 definiert, in Kombination mit einem oder mehreren weiteren Wirkstoffen.

12. Arzneimittel nach Anspruch 10 oder 11 zur Verwendung in einem Verfahren zur Behandlung und/oder Prävention von Krebs- und Tumorerkrankungen.

13. Antiproliferativ wirkendes Konjugat, bei dem die Verbindung nach einem oder mehreren der Ansprüche 1-5 mit einem Protein verknüpft ist.

14. Antiproliferativ wirkendes Konjugat nach Anspruch 13, wobei das Protein ein Antikörper ist.

## Claims

1. Compound of the formula (I) 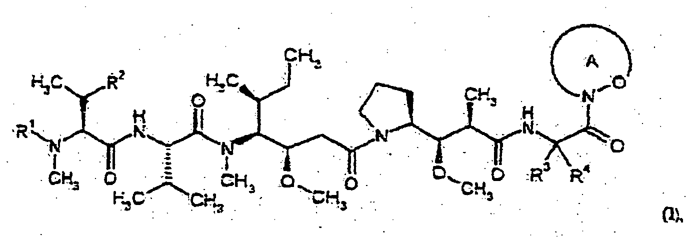 in which
R¹ represents hydrogen, (C₁-C₆ alkyl) or a group of the formula Q¹-L¹-* or Q²-L²- *, wherein
* indicates the point of attachment with the nitrogen atom,
Q¹ denotes hydroxycarbonyl, (C₁-C₄) alkoxycarbonyl or benzyloxycarbonyl,
L¹ denotes straight-chain (C₁-C₁₂) alkanediyl which may be substituted up to four times with methyl and in which (*a*) two carbon atoms in 1,2 relation, 1,3 relation or 1,4 relation to one another including the carbon atoms optionally lying between them may be bridged to form a (C₃-C₆) cycloalkyl ring or a phenyl ring or (*b*) up to three CH₂ groups not adjacent to one another may be exchanged for -O-,
Q² denotes hydroxy, amino or mono-(C₁-C₄) alkylamino,
and
L² denotes straight-chain (C₂-C₁₂) alkanediyl which may be substituted up to four times with methyl and in which (*a*) two carbon atoms in 1,2 relation, 1,3 relation or 1,4 relation to one another including the carbon atoms optionally lying between them may be bridged to form a (C₃-C₆) cycloalkyl ring or a phenyl ring or (*b*) up to three CH₂ groups not adjacent to one another may be exchanged for -O-,
R² represents methyl or hydroxy,
R³ represents hydrogen or methyl,
R⁴ represents isopropyl, isobutyl, *sec*.-butyl, *tert*.-butyl, phenyl, benzyl, 4-hydroxybenzyl, 4-hydroxy-3-nitrobenzyl, 1-phenylethyl, diphenylmethyl, 1*H*-imidazol-4-ylmethyl or 1*H*-indol-3-ylmethyl,
or
R³ and R⁴ together with the carbon atom, to which they are both bound, form a 2-phenylcyclopropane-1,1-diyl group of the formula 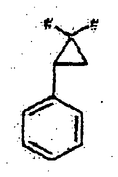 wherein
# indicates the points of attachment with the remaining moieties of the molecule,
and
the ring A with the N-O group present therein represents a monocyclic or bicyclic, optionally substituted heterocycle of the formula 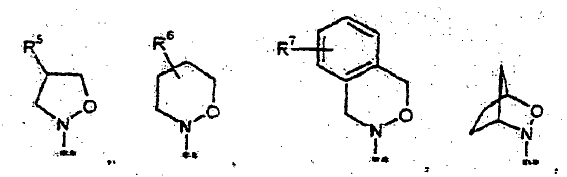 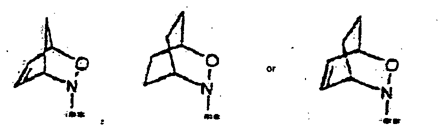 wherein
** indicates the point of attachment with the carbonyl group,
R⁵ and R⁶ denote in each case hydrogen, hydroxy, (C₁-C₄) alkoxy or benzyloxy,
and
R⁷ denotes hydrogen, fluorine, chlorine, cyano, methyl or trifluoromethyl, and its salts, solvates and solvates of the salts.

2. Compound of the formula (I) according to claim 1, in which
R¹ represents hydrogen, (C₁-C₄ alkyl) or a group of the formula Q¹-L¹-* or Q²-L²- *, wherein
* indicates the point of attachment with the nitrogen atom,
Q¹ denotes hydroxycarbonyl, (C₁-C₄) alkoxycarbonyl,
L¹ denotes straight-chain (C₁-C₁₂) alkanediyl in which (*a*) two carbon atoms in 1,3 relation or 1,4 relation to one another including the one or the two carbon atoms lying between them may be bridged to form a phenyl ring or (*b*) up to three CH₂ groups not adjacent to one another may be exchanged for -O-,
Q² d enotes hydroxy, amino or methylamino,
and
L² denotes straight-chain (C₂-C₁₂) alkanediyl which may be substituted once or twice with methyl and in which up to three CH₂ groups not adjacent to one another may be exchanged for -O-,
R² represents methyl or hydroxy,
R³ represents hydrogen,
R⁴ represents benzyl, 4-hydroxybenzyl, 1-phenylethyl or 1*H*-indol-3-ylmethyl,
or
R³ and R⁴ together with the carbon atom, to which they are both bound, form a 2-phenylcyclopropane-1,1-diyl group of the formula 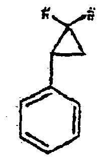 wherein
# indicates the points of attachment with the remaining moieties of the molecule,
and
the ring A with the N-O group present therein represents a monocyclic or bicyclic, optionally substituted heterocycle of the formula 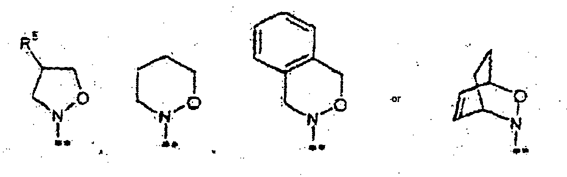 wherein
** indicates the point of attachment with the carbonyl group,
and
R⁵ denotes hydrogen, hydroxy or benzyloxy,
and its salts, solvates and solvates of the salts.

3. Compound of the formula (I) according to claim 1 or 2, in which
R¹ represents hydrogen, methyl or a group of the formula Q¹-L¹-* or Q²-L²- *, wherein
* indicates the point of attachment with the nitrogen atom,
Q¹ denotes hydroxycarbonyl, methoxycarbonyl or ethoxycarbonyl,
L¹ denotes straight-chain (C₁-C₆) alkanediyl, in which two carbon atoms in 1,4 relation to one another including the two carbon atoms lying between them may be bridged to form a phenyl ring
Q² denotes hydroxy or amino,
and
L² denotes straight-chain (C₂-C₆) alkanediyl,
R² represents methyl,
R³ represents hydrogen,
R⁴ represents benzyl, 1-phenylethyl or 1*H*-indol-3-ylmethyl,
or
R³ and R⁴ together with the carbon atom, to which they are both bound, form a (1*S*,2*R*)-2-phenylcyclopropane-1,1-diyl group of the formula 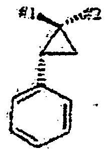 wherein
#1 indicates the point of attachment with the adjoining nitrogen atom,
and
#2 indicate the point of attachment with the carbonyl group,
and
the ring A with the N-O group present therein represents a monocyclic or bicyclic, optionally substituted heterocycle of the formula 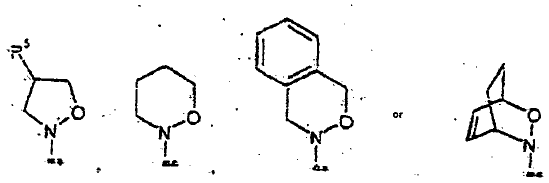 wherein
** indicates the point of attachment with the carbonyl group,
and
R⁵ denotes hydrogen, hydroxy or benzyloxy,
and its salts, solvates and solvates of the salts.

4. Compound according to claim 1, 2 or 3 with the formula (1-A) 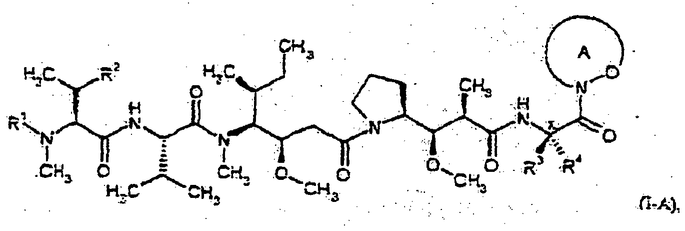 in which R¹, R², R³, R⁴ and the ring A have the meanings defined in claim 1,2 or 3 and the carbon atom carrying the radicals R³ and R⁴ has the S configuration shown,
and its salts, solvates and solvates of the salts.

5. Compound of claim 1, wherein said compound is selected from the group consisting of: 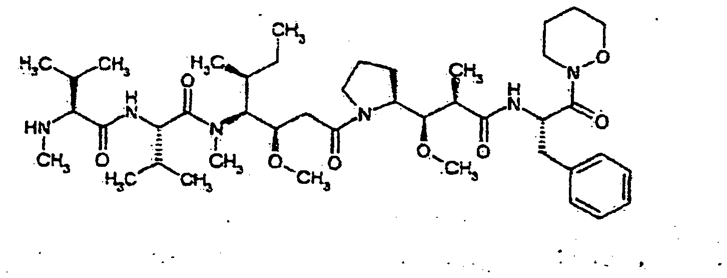 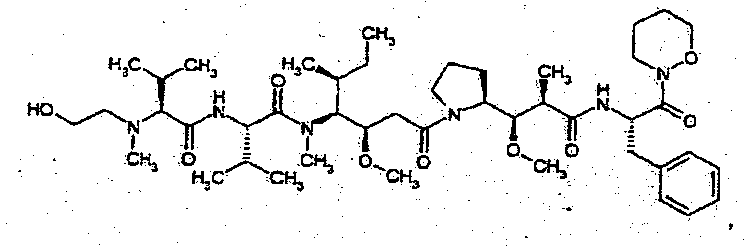 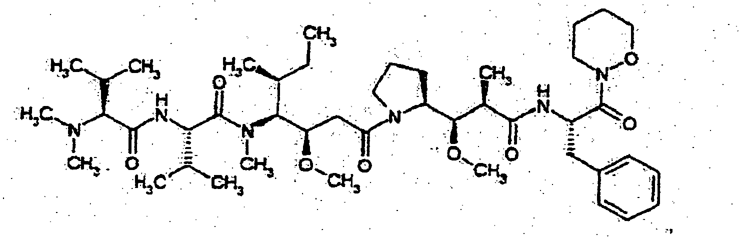 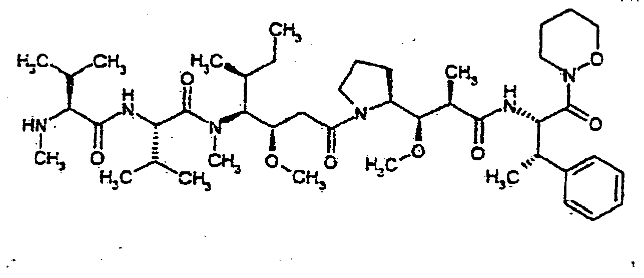 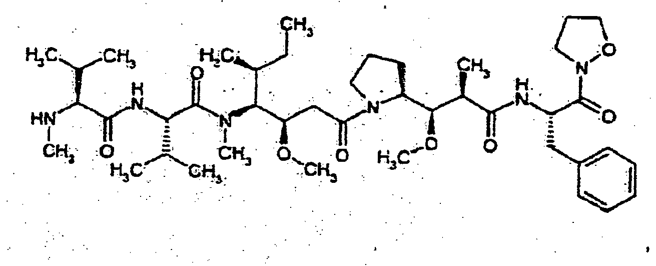 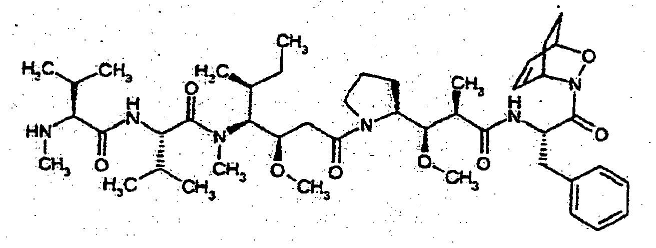 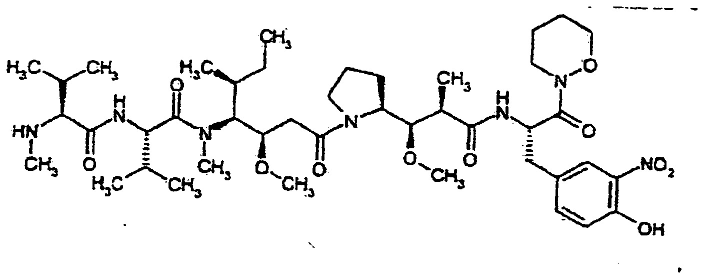 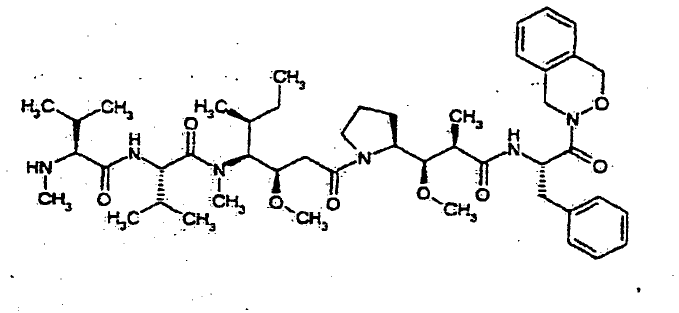 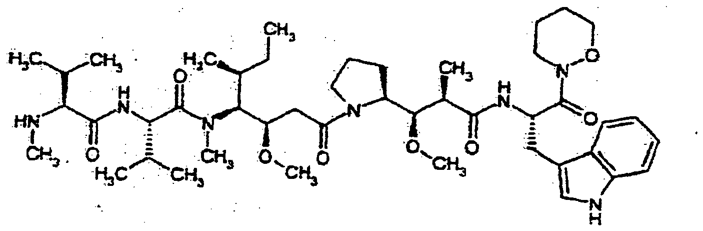 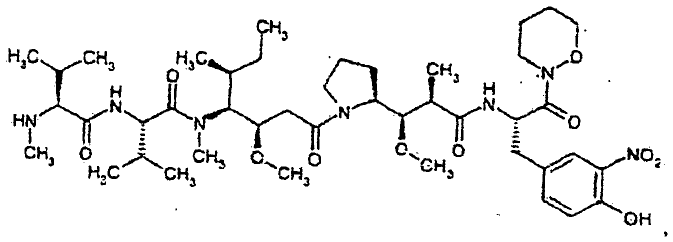 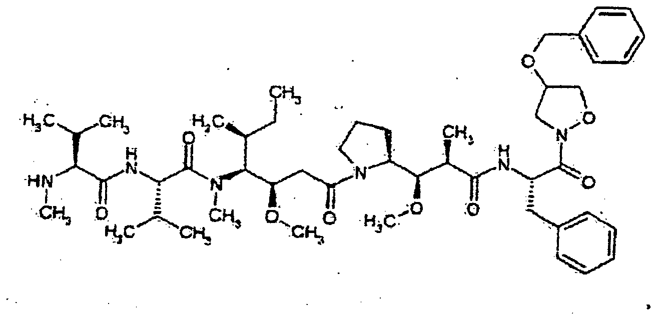 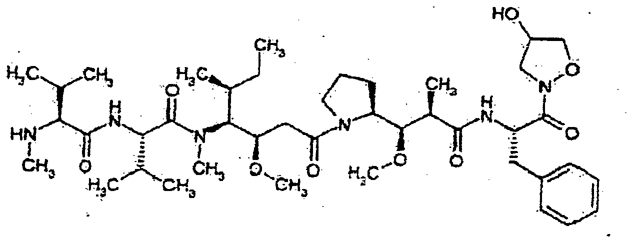 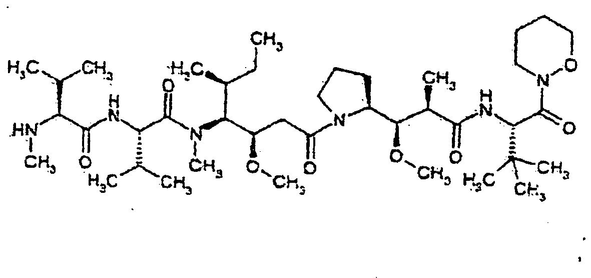 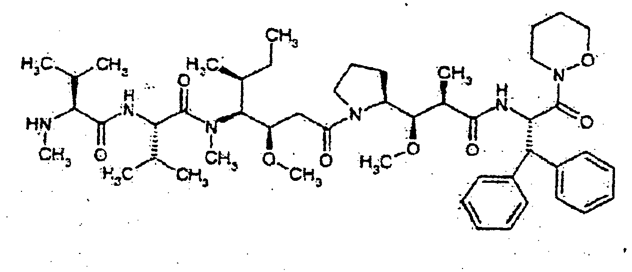 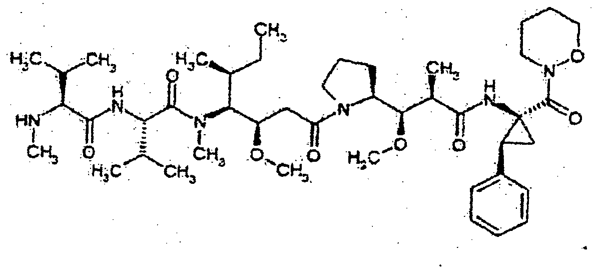 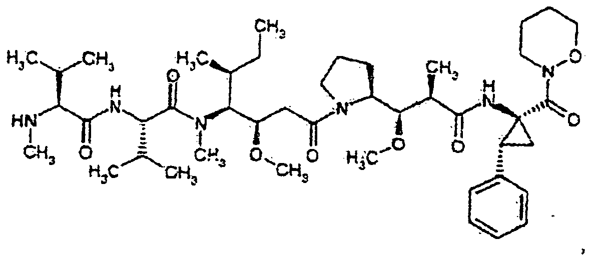 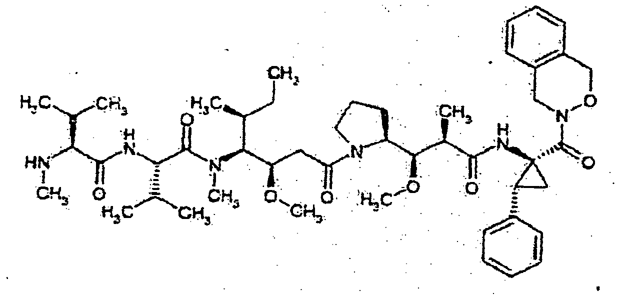 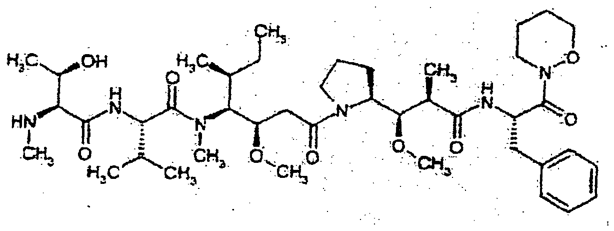 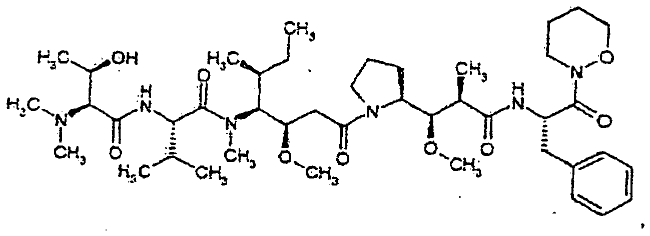 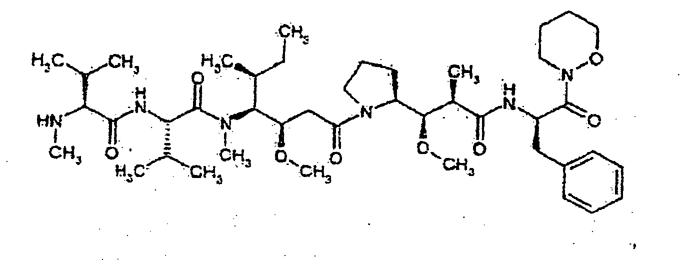 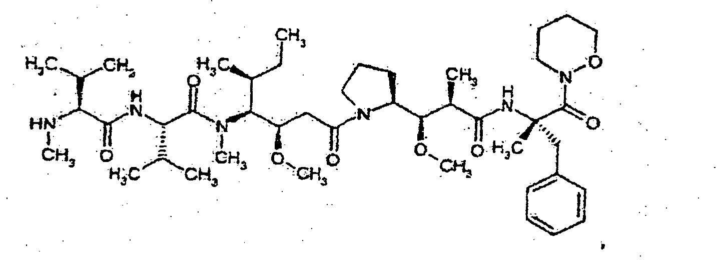 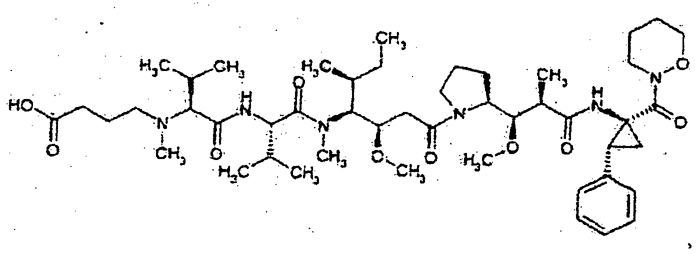 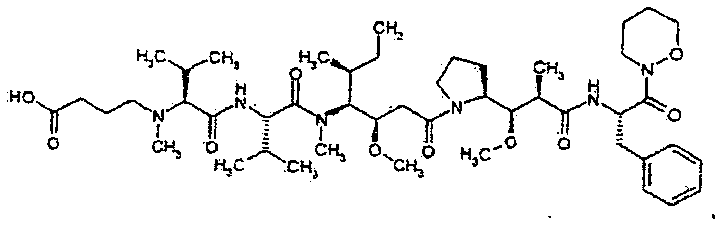 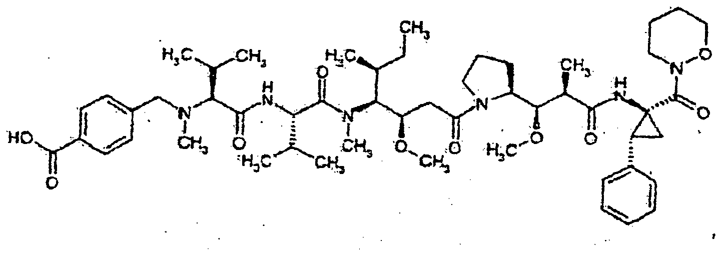 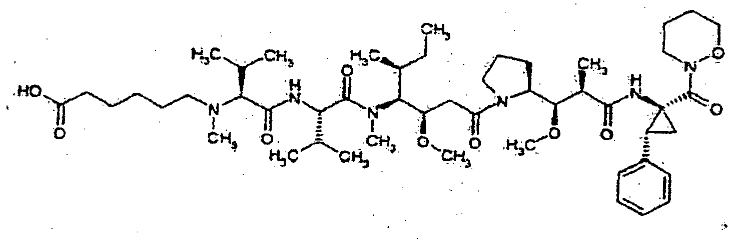 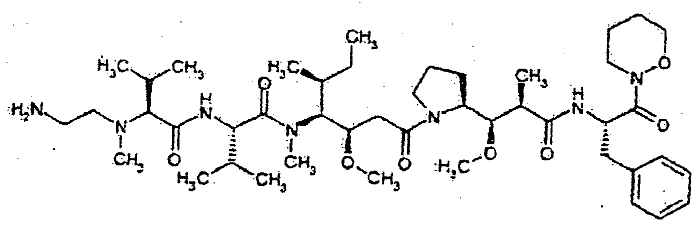 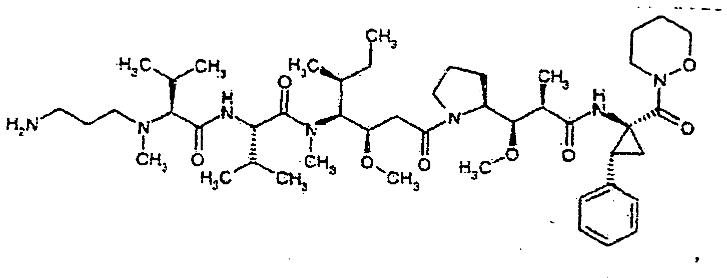 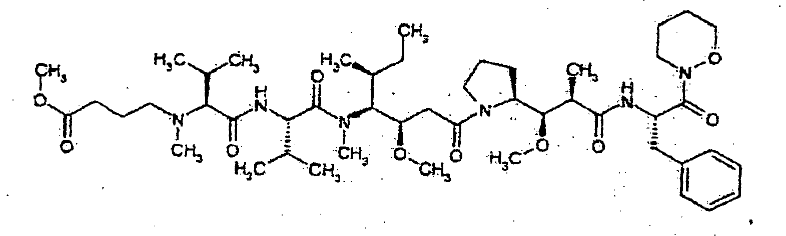 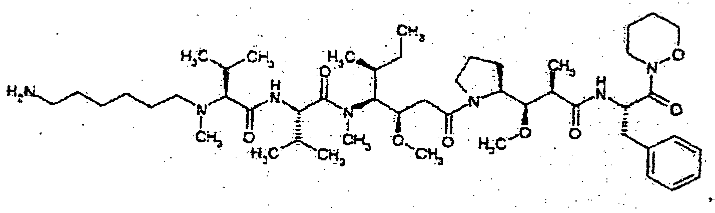 and

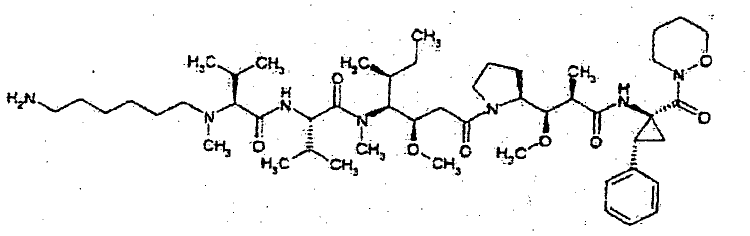

6. Method for producing a compound of the formula (I), as defined in claims 1 to 4, **characterised in that** a compound of the formula (II) 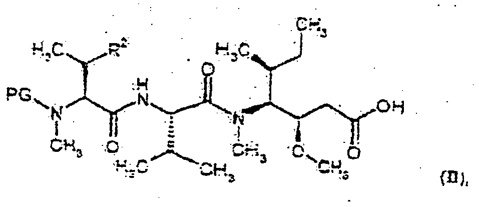 in which R² has the meaning indicated in claims 1 to 4
and
PG represents an amino protective group, such as for example (9*H*-fluoren-9-ylmethoxy)carbonyl, *tert*.-butoxycarbonyl or benzyloxycarbonyl,
is coupled in an inert solvent with activation of the carboxyl function in (II) either
[A] initially with the compound of the formula (III) 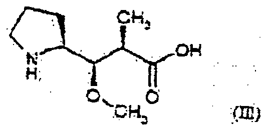 or a salt thereof to form a compound of the formulae (IV) 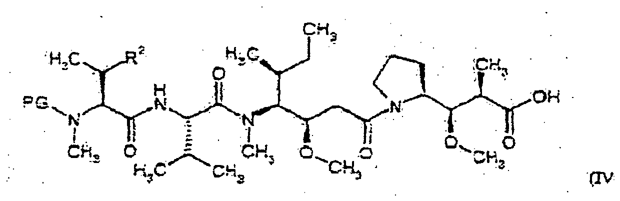 in which R² and PG have the meanings indicated above,
and then the latter is coupled in an inert solvent with activation of the carboxyl function with a compound of the formula (V) 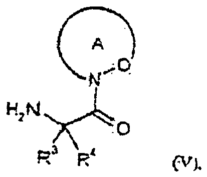 in which R³, R⁴ and the ring A have the meanings indicated in claims 1 to 4,
or a salt of this compound to form a compound of the formula (VI) 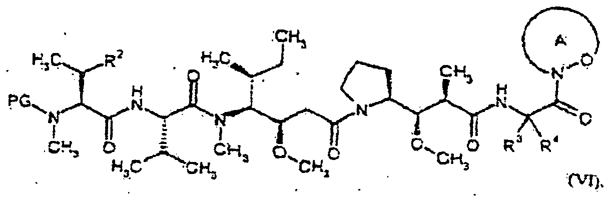 in which R², R³, R⁴, the ring A and PG have the meanings indicated above,
or
[B] is coupled with a compound of the formula (VII) 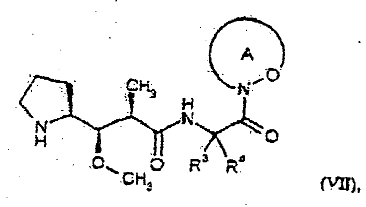 in which R³, R⁴ and the ring A have the meanings indicated in claims 1 to 4,
or a salt of this compound likewise to form the compound of the formula (VI) 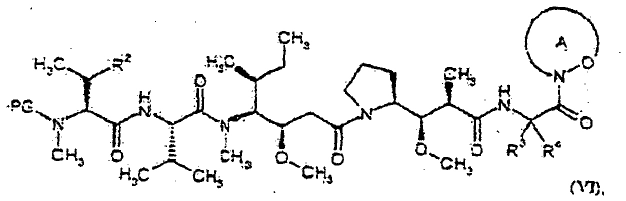 in which R², R³, R⁴, the ring A and PG have the meanings indicated above, and the particular resulting compound of the formula (VI) is then de-protected by conventional methods of peptide chemistry to form a compound of the invention of the formula (1-B) 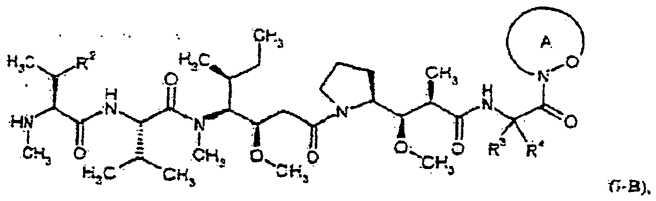 in which R², R³, R⁴ and the ring A have the meanings indicated above,
and the latter is then converted, if required, either (*i*) by base-induced alkylation with a compound of the formula (VIII)
R^{1A}-X (VIII),
in which
R^{1A} has the meaning of R¹ indicated in claims 1 to 4, but does not represent hydrogen,
and
X represents a volatile group, such as for example chloride, bromide, iodide, mesylate, triflate or tosylate,
to a compound of the invention of the formula (I-C) 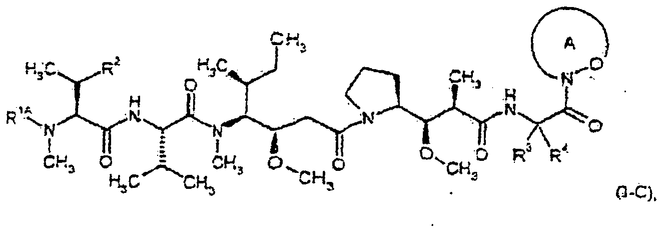 in which R^{1A}, R², R³, R⁴ and the ring A have the meanings indicated above, or (*ii*) is converted by reacting with a compound of the formula (IX) 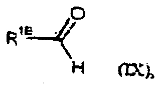 in which
R^{1B} has the meaning of R^{1A} indicated above, but shortened in the alkyl chain length by one CH₂ unit,
in the presence of a suitable reducing agent to a compound of the invention of the formula (I-D) 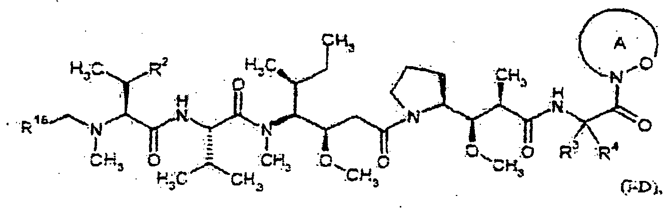
in which R^{1B}, R², R³, R⁴ and the ring A have the meanings indicated above,
and the compounds thus obtained of the formulae (1-B), (1-C) or (1-D) are separated optionally into their enantiomers and/or diastereomers and/or reacted with the corresponding (i) solvents and/or (*ii*) bases or acids to form their solvates, salts and/or solvates of the salts.

7. Compound as defined in one of claims 1 to 5 for use in a method for treating and/or preventing diseases.

8. Compound as defined in one of claims 1 to 5 for use in a method for treating and/or preventing carcinosis and oncosis.

9. Use of a compound as defined in one of claims 1 to 5 for producing a medicament for treating and/or preventing carcinosis and oncosis.

10. Medicament containing a compound as defined in one of claims 1 to 5 in combination with one or more inert, non-toxic, pharmaceutically suitable adjuvants.

11. Medicament containing a compound as defined in one of claims 1 to 5 in combination with one or more further active ingredients.

12. Medicament according to claim 10 or 11 for use in a method for treating and/or preventing carcinosis and oncosis.

13. Antiproliferative-acting conjugate in which the compound according to one or more of claims 1-5 is linked to a protein.

14. Antiproliferative-acting conjugate according to claim 13, wherein the protein is an antibody.

## Revendications

1. Composé de formule (I) 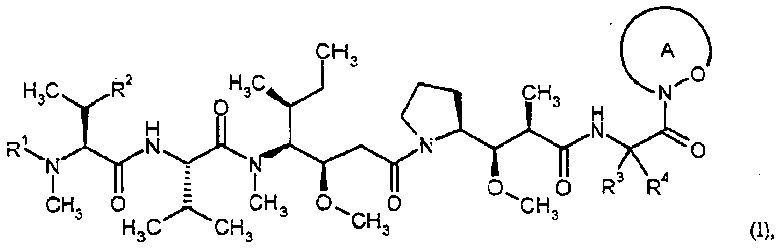 dans laquelle
R¹ représente un atome d'hydrogène, un groupe alkyle (en C₁-C₆) ou un groupe de formule Q¹-L¹-* ou Q²-L²-*, dans laquelle
* caractérise le site de liaison avec l'atome d'azote,
Q¹ représente un groupe hydroxycarbonyle, alcoxy(en C₁-C₄)carbonyle ou benzyloxycarbonyle,
L¹ représente un groupe alcane(en C₁-C₁₂)diyle linéaire qui peut être substitué jusqu'à quatre fois par un groupe méthyle et dans lequel *(a)* deux atomes de carbone peuvent être reliés en position 1,2, 1,3 ou 1,4 l'un avec l'autre en intégrant les atomes de carbone se trouvant le cas échéant entre eux pour former un radical cycloalkyle (en C₃-C₆) ou un radical phényle ou *(b)* jusqu'à trois groupes CH₂ non adjacents peuvent être remplacés par un atome de -O-,
Q² représente un groupe hydroxy, amino ou monoalkyl(en C₁-C₄) amino,
et
L² représente un groupe alcane(en C₂-C₁₂) diyle linéaire qui peut être substitué jusqu'à quatre fois par un groupe méthyle et dans lequel *(a)* deux atomes de carbone peuvent être reliés en position 1,2, 1,3 ou 1,4 l'un avec l'autre en intégrant les atomes de carbone se trouvant le cas échéant entre eux pour former un radical cycloalkyle (en C₃-C₆) ou un radical phényle ou *(b)* jusqu'à trois groupes CH₂ non adjacents peuvent être remplacés par un atome de -O-,
R² représente un groupe méthyle ou hydroxy,
R³ représente un atome d'hydrogène ou un groupe méthyle,
R⁴ représente un groupe isopropyle, isobutyle, *sec.-*butyle, *tert*.-butyle, phényle, benzyle, 4-hydroxybenzyle, 4-hydroxy-3-nitrobenzyle, 1-phényléthyle, diphénylméthyle, 1*H-*imidazol-4-ylméthyle, ou 1*H*-indol-3-ylméthyle,
ou
R³ et R⁴ forment conjointement avec l'atome de carbone auquel ils sont reliés un groupe 2-phénylcyclopropane-1,1-diyle de formule 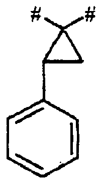 dans laquelle
# caractérise les sites de liaison avec les parties restantes de la molécule,
et
le radical A avec le groupement N-O qu'il contient représente un hétérocycle mono- ou bicyclique, le cas échéant substitué, de formules 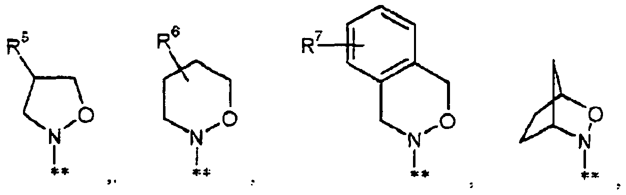 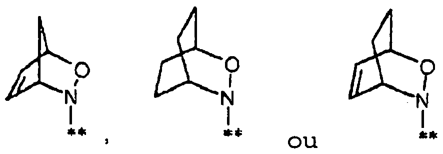 dans lesquelles
** caractérise le site de liaison avec le groupe carbonyle,
R⁵ et R⁶ représentent respectivement un atome d'hydrogène, un groupe hydroxy, alcoxy (en C₁-C₄) ou benzyloxy,
et
R⁷ représente un atome d'hydrogène, de fluor, de chlore, un groupe cyano, méthyle ou trifluorométhyle,
ainsi que leurs sels, solvates et solvates de leurs sels.

2. Composé de formule (I) selon la revendication 1, dans lequel
R¹ représente un atome d'hydrogène, un groupe alkyle (en C₁-C₄) ou un groupe de formule Q¹-L¹-* ou Q²-L²-*, dans laquelle
* caractérise le site de liaison avec l'atome d'azote,
Q¹ représente un groupe hydroxycarbonyle ou alcoxy(en C₁-C₄) carbonyle,
L¹ représente un groupe alcane(en C₁-C₁₂) diyle linéaire dans lequel *(a)* deux atomes de carbone peuvent être reliés en position 1,3 ou 1,4 l'un avec l'autre en intégrant l'un ou bien les deux atomes de carbone se trouvant entre eux pour former un radical phényle ou *(b)* jusqu'à trois groupes CH₂ non adjacents peuvent être remplacés par un atome de -O-,
Q² représente un groupe hydroxy, amino ou méthylamino,
et
L² représente un groupe alcane(en C₂-C₁₂) diyle linéaire qui peut être substitué une ou deux fois par un groupe méthyle et dans lequel jusqu'à trois groupes CH₂ non adjacents peuvent être remplacés par un atome de -O-,
R² représente un groupe méthyle ou hydroxy,
R³ représente un atome d'hydrogène,
R⁴ représente un groupe benzyle, 4-hydroxybenzyle, 1-phényléthyle, ou 1*H*-indol-3-ylméthyle,
ou
R³ et R⁴ forment conjointement avec l'atome de carbone auquel ils sont reliés un groupe 2-phénylcyclopropane-1,1-diyle de formule 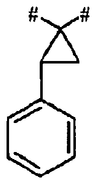 dans laquelle
# caractérise les sites de liaison avec les parties restantes de la molécule,
et
le radical A avec le groupement N-O qu'il contient représente un hétérocycle mono- ou bicyclique, le cas échéant substitué, de formules 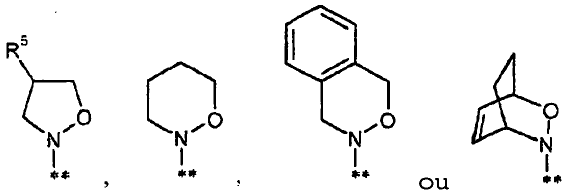 dans lesquelles
** caractérise le site de liaison avec le groupe carbonyle,
et
R⁵ représente un atome d'hydrogène, un groupe hydroxy ou benzyloxy,
ainsi que leurs sels, solvates et solvates de leurs sels.

3. Composé de formule (I) selon la revendication 1 ou 2, dans lequel
R¹ représente un atome d'hydrogène, un groupe méthyle ou un groupe de formule Q¹-L¹-* ou Q²-L²-* , dans laquelle
* caractérise le site de liaison avec l'atome d'azote,
Q¹ représente un groupe hydroxycarbonyle, méthoxycarbonyle ou éthoxycarbonyle,
L¹ représente un groupe alcane(en C₁-C₆) diyle linéaire dans lequel deux atomes de carbone peuvent être reliés en position 1,4 l'un avec l'autre en intégrant les deux atomes de carbone se trouvant entre eux pour former un radical phényle,
Q² représente un groupe hydroxy ou amino,
et
L² représente un groupe alcane (en C₂-C₆) diyle linéaire,
R² représente un groupe méthyle,
R³ représente un atome d'hydrogène,
R⁴ représente un groupe benzyle, 1-phényléthyle, ou 1H-indol-3-ylméthyle,
ou
R³ et R⁴ forment conjointement avec l'atome de carbone auquel ils sont reliés un groupe (1*S*,2*R*)-2-phénylcyclopropane-1,1-diyle de formule 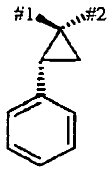 dans laquelle
#1 caractérise le site de liaison avec l'atome d'azote adjacent,
et
#2 caractérise le site de liaison avec le groupe carbonyle,
et
le radical A avec le groupement N-O qu'il contient représente un hétérocycle mono- ou bicyclique, le cas échéant substitué, de formules 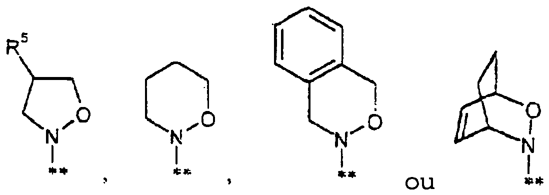 dans lesquelles
** caractérise le site de liaison avec le groupe carbonyle,
et
R⁵ représente un atome d'hydrogène, un groupe hydroxy ou benzyloxy,
ainsi que leurs sels, solvates et solvates de leurs sels.

4. Composé selon la revendication 1, 2 ou 3 de formule (I-A) 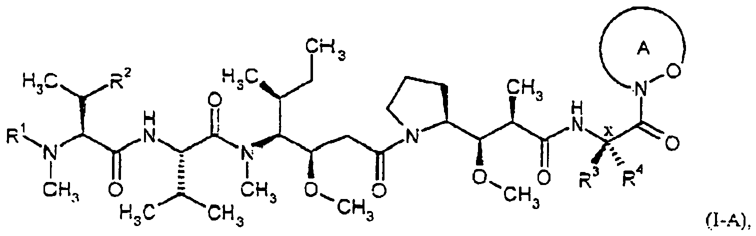 dans laquelle R¹, R², R³, R⁴ et le radical A ont les significations définies dans la revendication 1, 2 ou 3, et l'atome de carbone en C^{x} portant les radicaux R³ et R⁴ présente la configuration *S* reproduite,
ainsi que leurs sels, solvates et solvates de leurs sels.

5. Composé selon la revendication 1, dans lequel ledit composé est choisi dans le groupe constitué par : 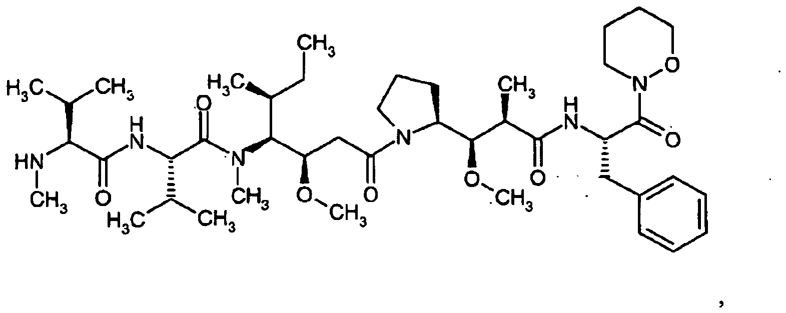 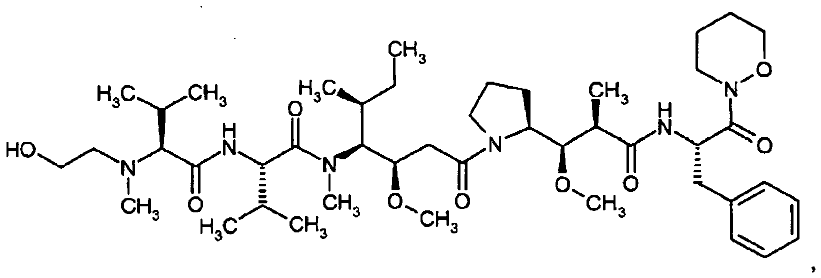 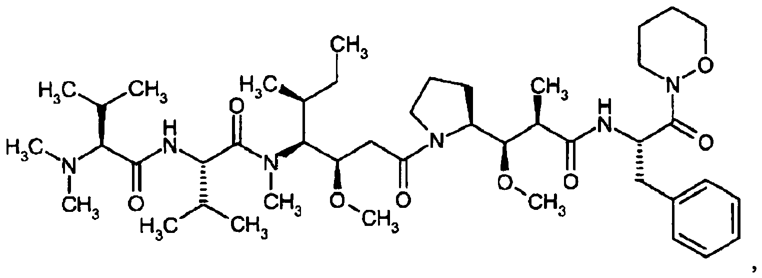 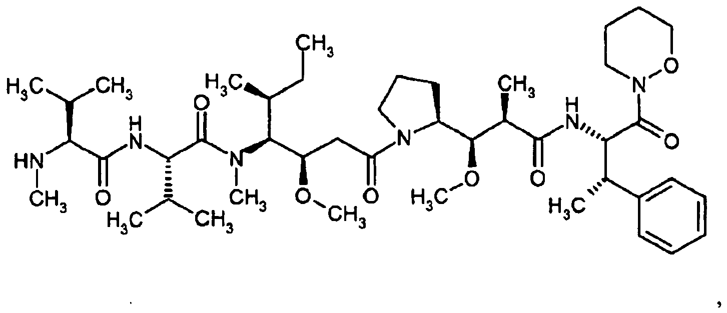 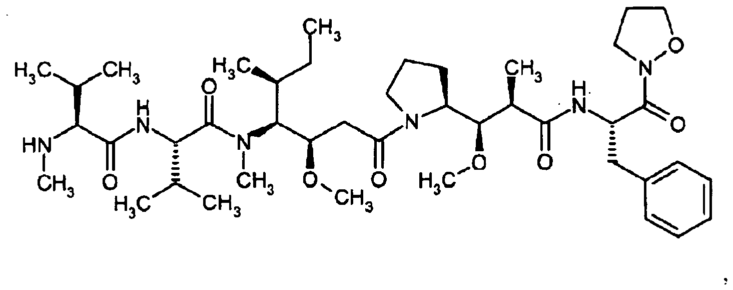 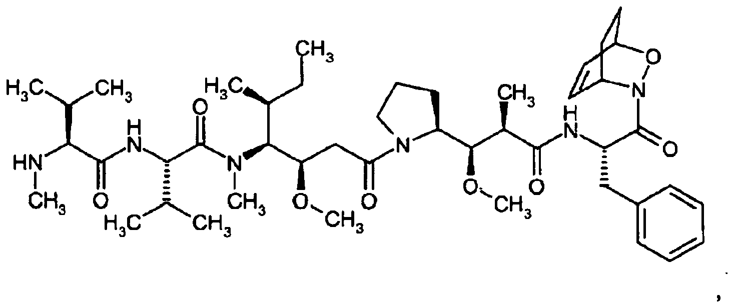 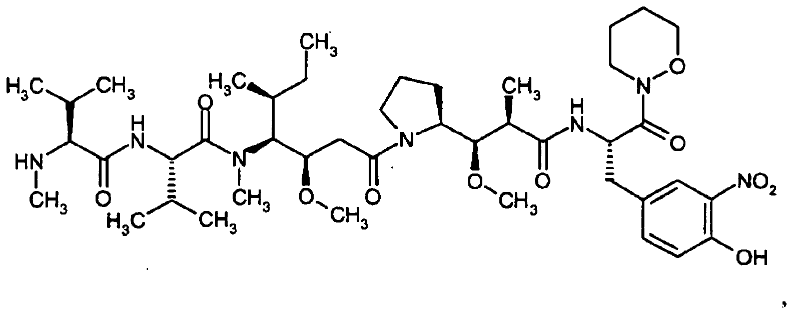 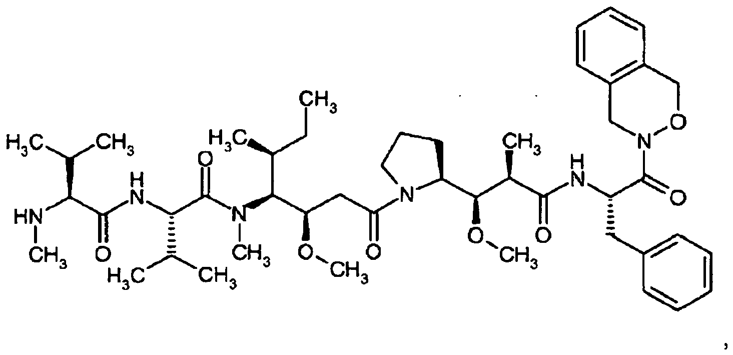 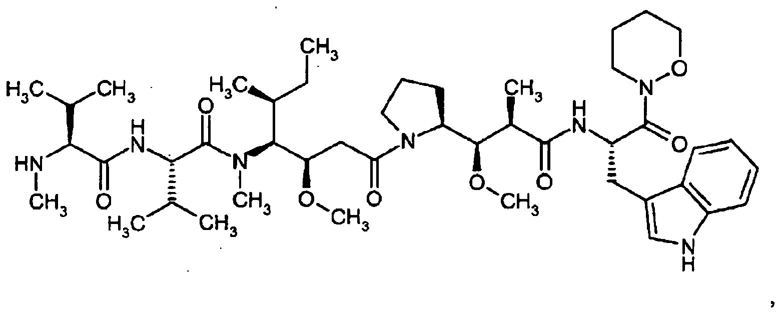 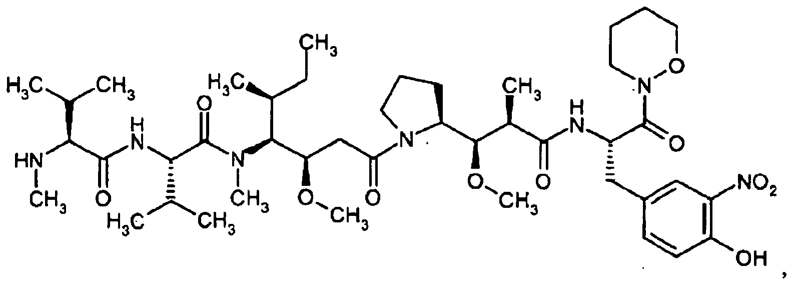 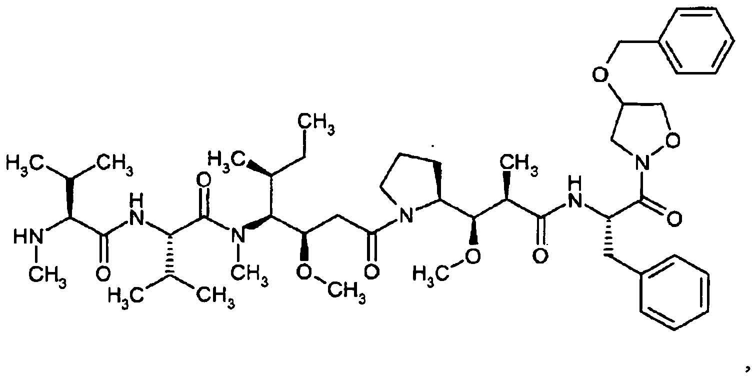 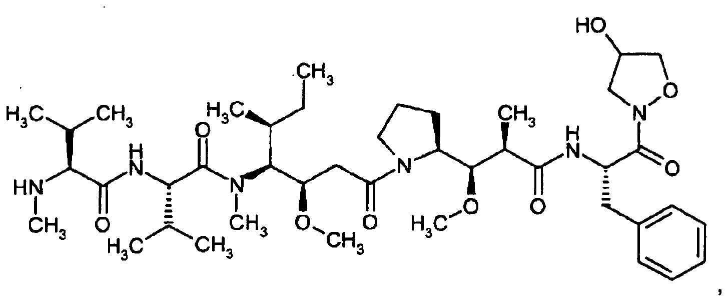 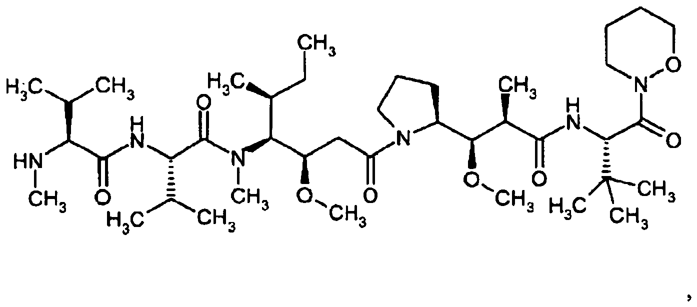 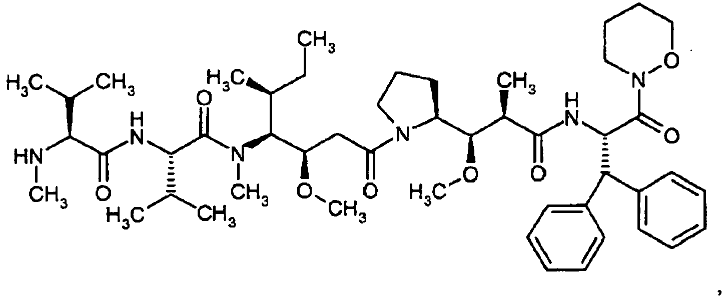 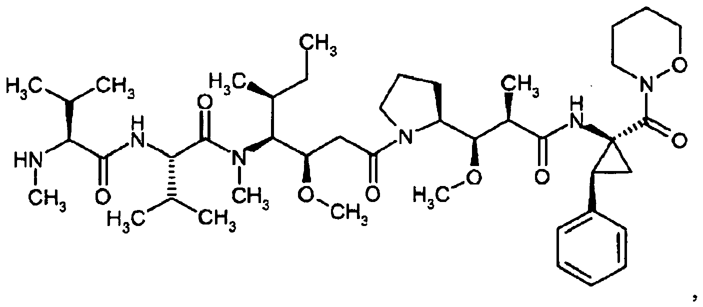 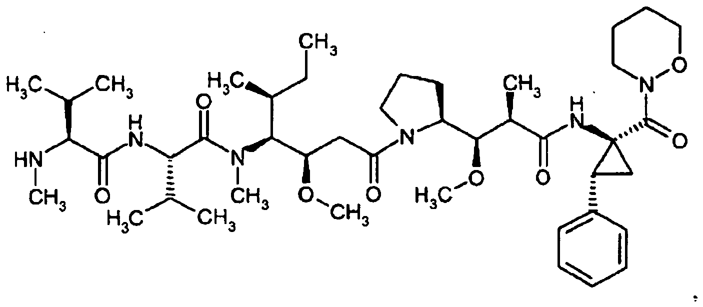 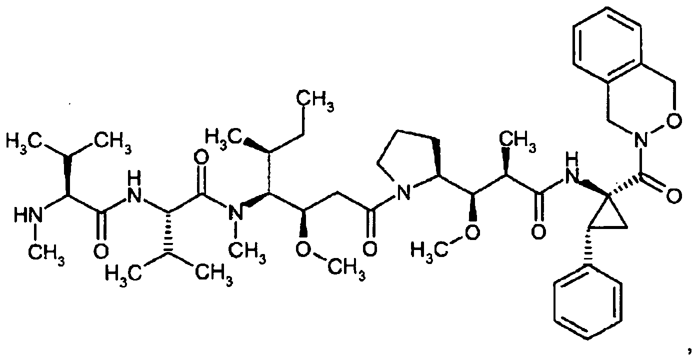 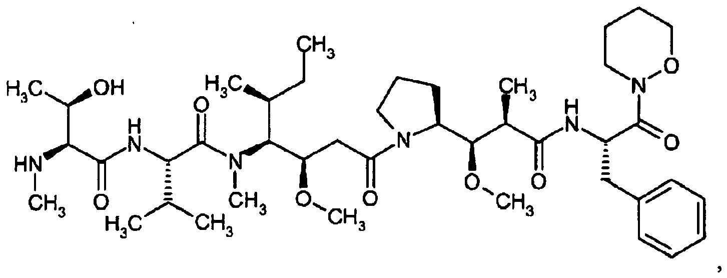 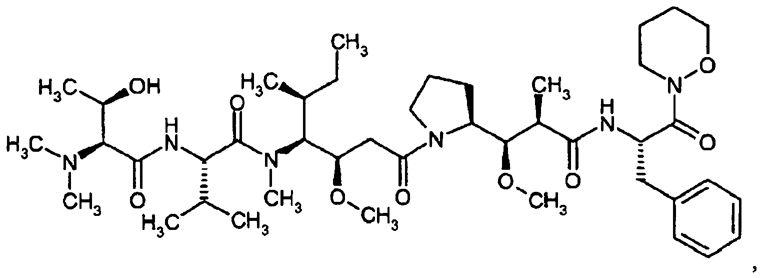 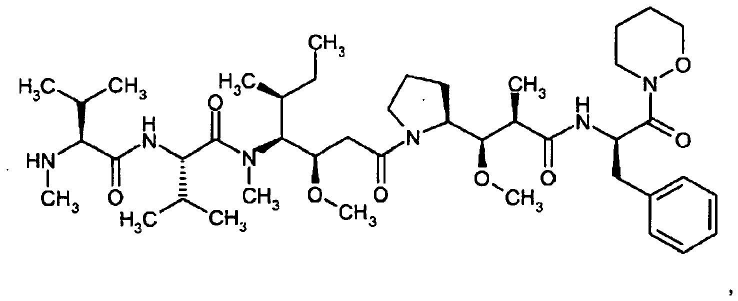 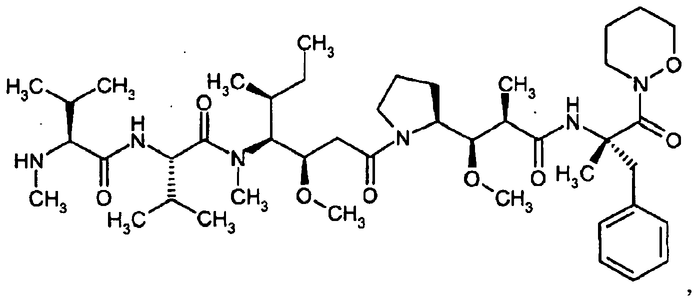 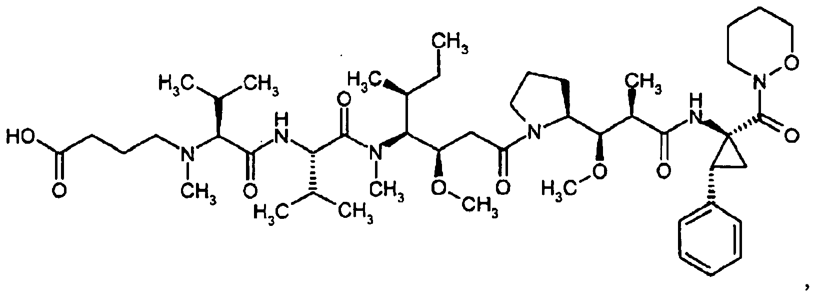 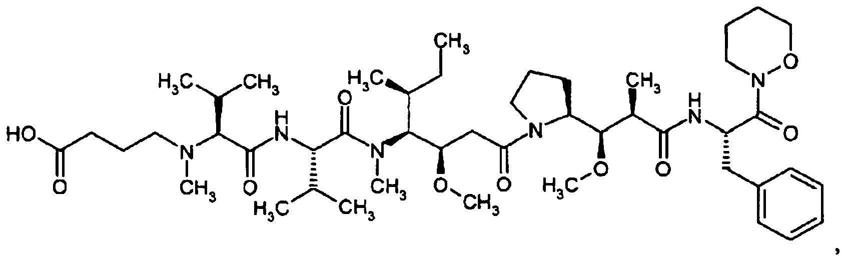 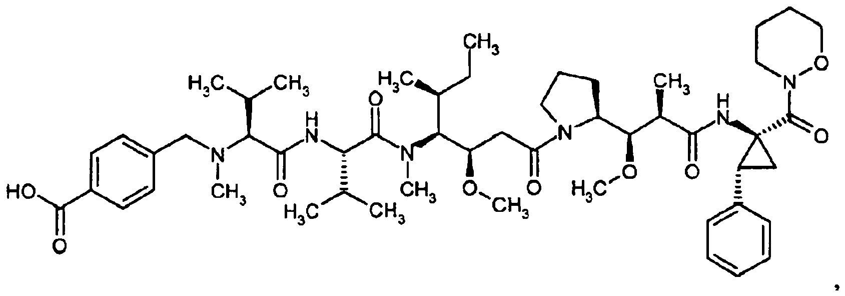 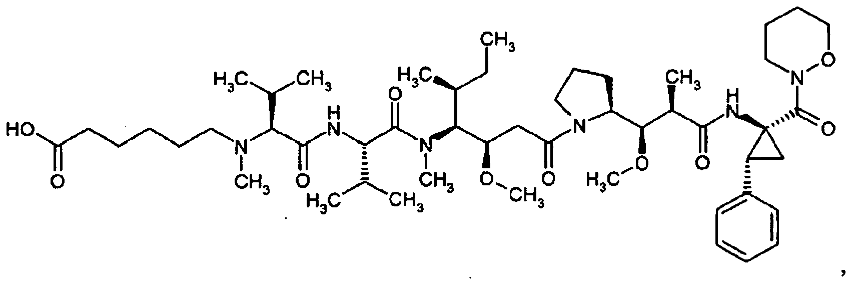 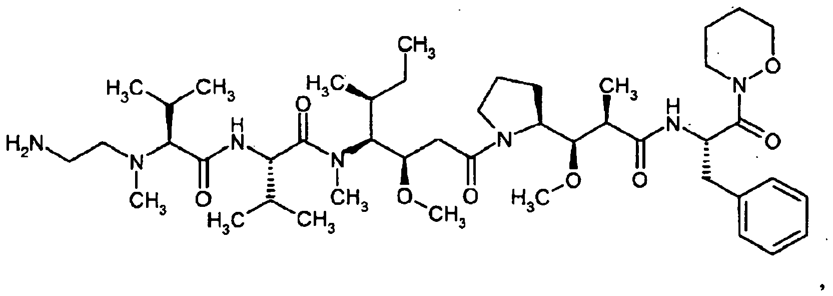 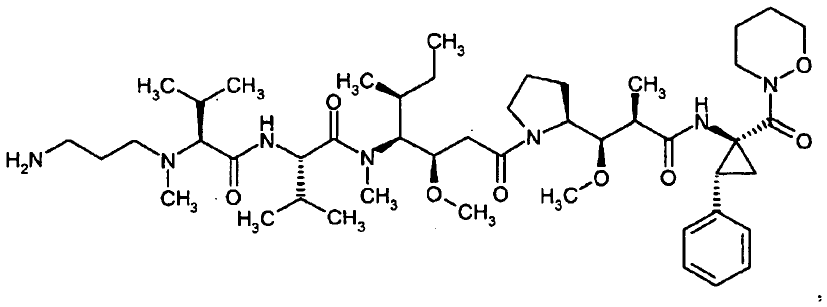 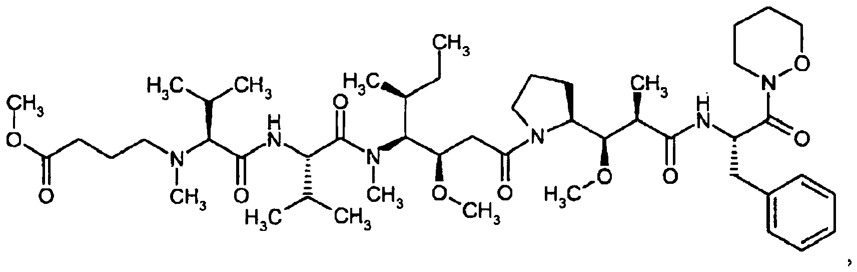 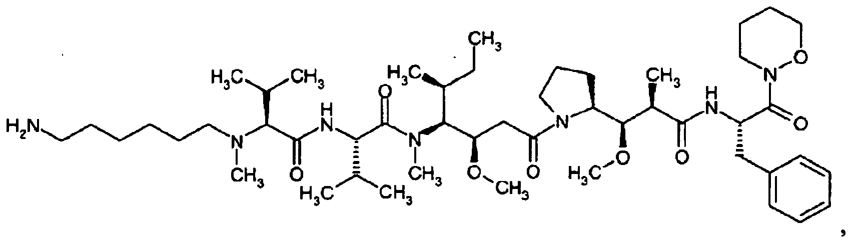 et

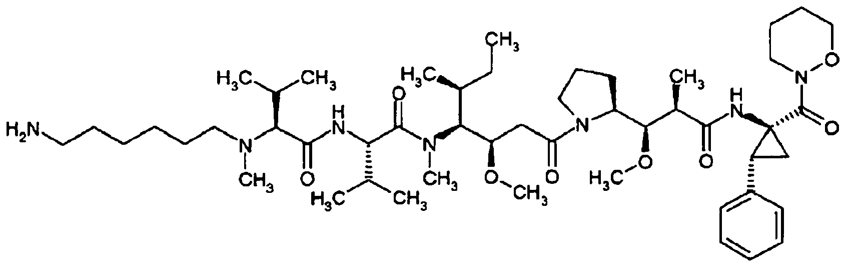

6. Procédé de préparation d'un composé de formule (I), tel que défini dans les revendications 1 à 4, **caractérisé en ce que** l'on couple un composé de formule (II) 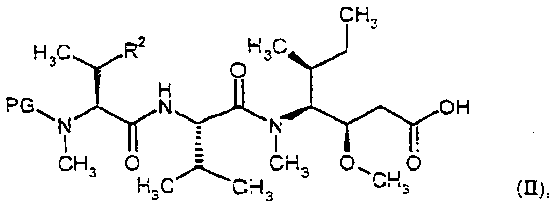 dans laquelle R² a la signification indiquée dans les revendications 1 à 4,
et
PG représente un groupe protecteur amino tel que, par exemple, le (9*H*-fluorèn-9-ylméthoxy)carbonyle, le *tert.-*butoxycarbonyle ou le benzyloxycarbonyle,
dans un solvant inerte en activant la fonction carboxyle dans (II) soit
[A] d'abord avec le composé de formule (III) 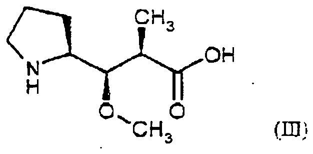 ou un sel de celui-ci pour donner un composé de formule (IV) 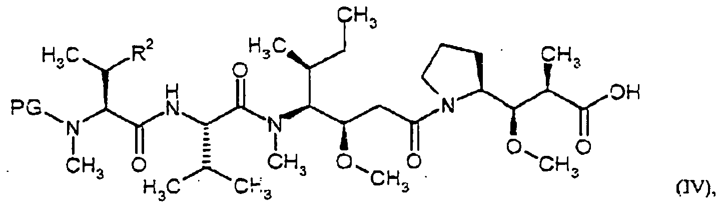 dans laquelle R² et PG ont les significations indiquées ci-dessus,
et l'on couple celui-ci par la suite dans un solvant inerte en activant la fonction carboxyle avec un composé de formule (V) 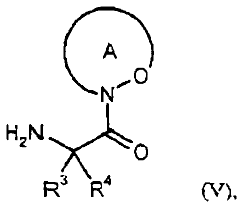 dans laquelle R³, R⁴ et le cycle A ont les significations indiquées dans les revendications 1 à 4,
ou un sel de ce composé pour donner un composé de formule (VI) 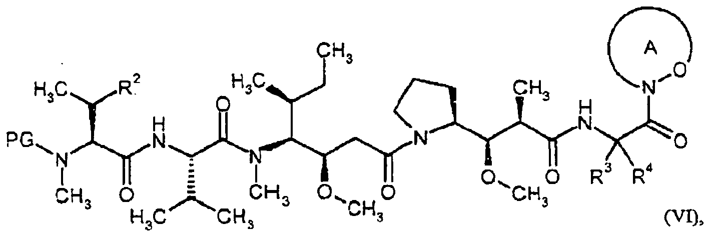 dans laquelle R², R³, R⁴, le cycle A et PG ont les significations indiquées ci-dessus,
ou
[B] avec un composé de formule (VII) 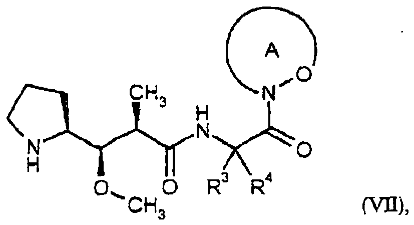
dans laquelle R³, R⁴ et le cycle A ont les significations indiquées dans les revendications 1 à 4,
ou un sel de ce composé pour donner également un composé de formule (VI) 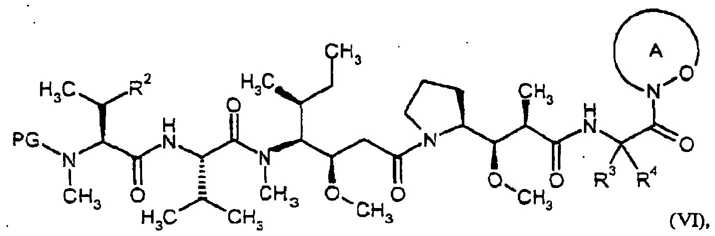 dans laquelle R², R³, R⁴, le cycle A et PG ont les significations indiquées ci-dessus,
et l'on déprotège ensuite le composé de formule (VI) résultant à chaque fois selon les méthodes usuelles de la chimie des peptides pour donner un composé selon l'invention de formule (I-B) 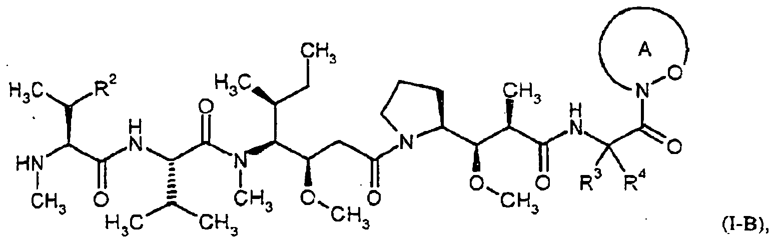 dans laquelle R², R³, R⁴ et le cycle A ont les significations indiquées ci-dessus,
et l'on convertit celui-ci par la suite, si désiré, soit (i) par alkylation induite par des bases avec un composé de formule (VIII)
R^{1A}-X (VIII),
dans laquelle
R^{1A} a la signification de R¹ indiquée dans les revendications 1 à 4, mais ne représente cependant pas un atome d'hydrogène,
et
X représente un groupe sortant, comme par exemple le chlorure, le bromure, l'iodure, le mésylate, le triflate ou le tosylate,
en un composé selon l'invention de formule (I-C) 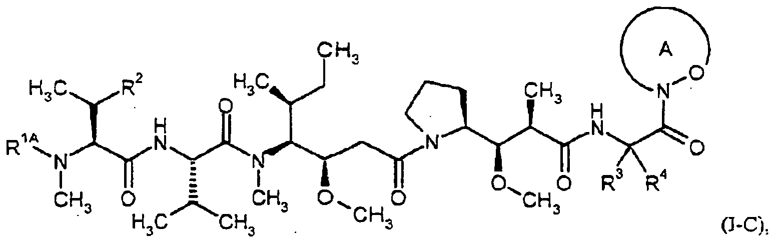 dans laquelle R^{1A}, R², R³, R⁴ et le cycle A ont les significations indiquées ci-dessus,
ou *(ii)* par réaction avec un composé de formule (IX) 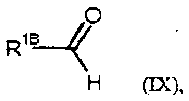 dans laquelle
R^{1B} a la signification de R^{1A} indiquée ci-dessus, mais
cependant raccourcie d'une unité CH₂ dans la longueur de chaîne alkyle,
en présence d'un agent réducteur approprié en un composé selon l'invention de formule (I-D) 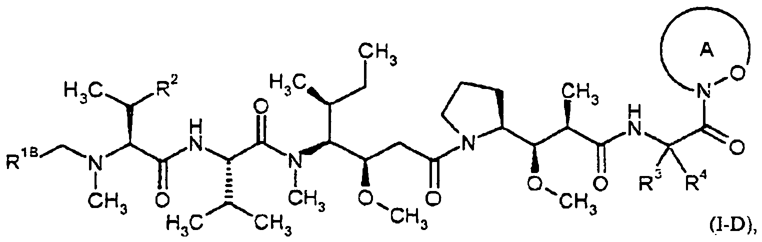 dans laquelle R^{1B}, R², R³, R⁴ et le cycle A ont les significations indiquées ci-dessus,
et l'on sépare les composés de formules (I-B), (I-C) ou (I-D) ainsi obtenus le cas échéant en leurs énantiomères et/ou diastéréomères et/ou l'on fait réagir avec les (i) solvants et/ou (ii) bases ou acides correspondants en leurs solvates, sels et/ou solvates de leurs sels.

7. Composé, tel que défini dans l'une des revendications 1 à 5, pour l'utilisation dans un procédé destiné au traitement et/ou à la prévention des maladies.

8. Composé, tel que défini dans l'une des revendications 1 à 5, pour l'utilisation dans un procédé destiné au traitement et/ou à la prévention des affections cancéreuses et tumorales.

9. Utilisation d'un composé, tel que défini dans l'une des revendications 1 à 5, pour la préparation d'un médicament destiné au traitement et/ou à la prévention des affections cancéreuses et tumorales.

10. Médicament contenant un composé, tel que défini dans l'une des revendications 1 à 5, combiné à un ou plusieurs auxiliaires pharmaceutiques, inertes et non toxiques.

11. Médicament contenant un composé, tel que défini dans l'une des revendications 1 à 5, combiné à un ou plusieurs autres principes actifs.

12. Médicament selon la revendication 10 ou 11 pour l'utilisation dans un procédé destiné au traitement et/ou à la prévention des affections cancéreuses et tumorales.

13. Conjugué ayant une action antiproliférative, dans lequel le composé selon une ou plusieurs des revendications 1 à 5 est lié à une protéine.

14. Conjugué ayant une action antiproliférative selon la revendication 13, dans lequel la protéine est un anticorps.
